(19) Europäisches Patentamt
European Patent Office
Office européen des brevets

(11) Numéro de publication: **0 033 259 B1**

# (12) FASCICULE DE BREVET EUROPEEN

(45) Date de publication du fascicule du brevet: **24.10.84**

(21) Numéro de dépôt: **81400051.9**

(22) Date de dépôt: **16.01.81**

(51) Int. Cl.³: **C 07 C 61/40**, C 07 C 62/32, C 07 C 62/36, C 07 C 69/63, C 07 C 69/743, C 07 C 103/00, C 07 C 121/48, C 07 C 153/067, C 07 C 51/353 // C07F9/54, C07C51/00, C07C67/327

(54) **Dérivés 3-(buta-1',3'-diényl) substitués de l'acide cyclopropane 1-carboxylique, les compositions contre les parasites des végétaux et des animaux à sang chaud, ainsi que les compositions parfumantes les renfermant, leur préparation.**

(30) Priorité: **25.01.80 FR 8001640**

(43) Date de publication de la demande: **05.08.81 Bulletin 81/31**

(45) Mention de la délivrance du brevet: **24.10.84 Bulletin 84/43**

(84) Etats contractants désignés: **AT BE CH DE FR GB IT LI LU NL SE**

(56) Documents cités:
**DE - A - 2 638 356**
**FR - A - 2 144 336**
**FR - A - 2 364 884**

(73) Titulaire: **ROUSSEL-UCLAF, 35, boulevard des Invalides, F-75007 Paris (FR)**

(72) Inventeur: **Martel, Jacques, 15, rue Douvillez, F-93140-Bondy (FR)**
Inventeur: **Tessier, Jean, 30, rue Jean Moulin, F-94300-Vincennes (FR)**
Inventeur: **Girault, Pierre, 4, rue des Abbesses, F-75006-Paris (FR)**

(74) Mandataire: **Tonnellier, Marie-José et al, 102, route de Noisy Boîte postale no 9, F-93230 Romainville (FR)**

EP 0 033 259 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

ACTORUM AG

**Description pour les Etats contractants: AT, BE, CH, DE, FR, GB, IT, LI, LU, NL, SE**

La présente invention concerne de nouveaux dérivés 3-(buta-1', 3'-diényl) substitué de l'acide cyclopropane-1-carboxylique, leur application à la lutte contre les parasites des végétaux et des animaux à sang chaud, les compositions correspondantes ainsi que les compositions parfumantes les renfermant, leur préparation et les nouveaux intermédiaires obtenus.

On connaissait déjà par le brevet 2 144 336 de Sumitomo des composés de formule:

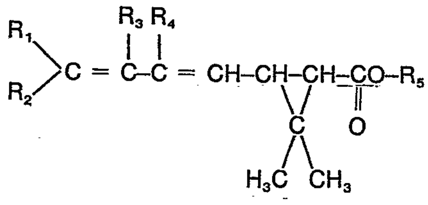

où $R_1$, $R_2$, $R_3$ et $R_4$ sont des atomes d'hydrogène ou des groupes méthyles. $R_5$ est un reste d'alcool utilisé dans la série pyréthrinoïde. Ces produits présentent une activité insecticide relativement faible.

On connaissait également le brevet allemand 2 638 356 citant entre autres à la page 33, lignes 53 à 56 un acide de formule:

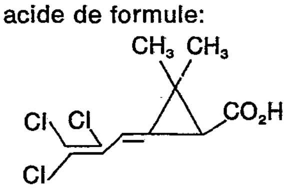

qui comporte deux doubles liaisons conjuguées mais comporte 3 atomes de chlore.

On connaissait aussi dans le brevet français 2 364 884 des composés de formule générale:

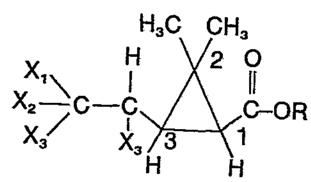

dont la chaîne latérale en position 3 est une chaîne saturée tétrahalogénée ne comportant donc aucune double liaison sur la chaîne portée par le carbone en 3 de la double liaison.

L'invention a pour objet, sous toutes leurs formes stéréoisomères possibles ou sous forme de mélanges de stéréoisomères, les composés de formule (I):

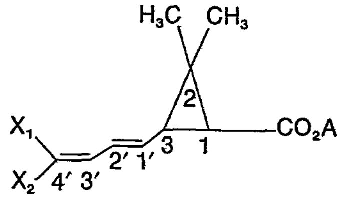

(I)

dans laquelle $X_1$ et $X_2$, identiques ou différents, représentent un atome d'halogène, et A représente:

– soit un atome d'hydrogène,

– soit un radical alcoyle renfermant de 1 à 18 atomes de carbone,

– soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylène dioxyle, le radical benzyle et les atomes d'halogènes,

– soit un groupement

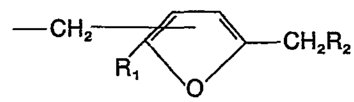

dans lequel le substituant $R_1$ représente un atome d'hydrogène ou un radical méthyle et le substituant $R_2$ un aryle monocyclique ou un groupement $-CH_2-C\equiv CH$ et notamment un groupement 5-benzyl 3-furyl méthyle,

– soit un groupement

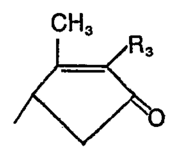

dans lequel $R_3$ représente un radical organique aliphatique comportant de 2 à 6 atomes de carbone et une ou plusieurs insaturations carbone-carbone et notamment le radical vinyle, propén-1-yle, buta-1,3-diényle ou buten-1-yle,

– soit un groupement

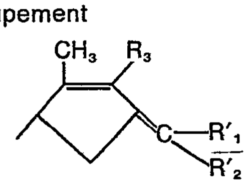

dans lequel $R_3$ conserve la même signification que précédemment, $R'_1$ et $R'_2$, identiques ou différents, représentent un atome d'hydrogène, un atome d'halogène, un radical alcoyle renfermant de 1 à 6 atomes de carbone, un radical aryle comportant de 6 à 10 atomes de carbone, un groupement alcoyl-oxycarbonyle comportant de 2 à 5 atomes de carbone, ou un groupement cyano,

– soit un groupement

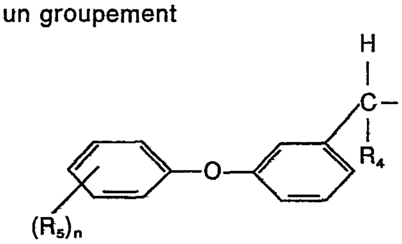

dans lequel $R_4$ représente un atome d'hydrogène, un groupement $-C\equiv N$, un radical méthyle, un radical $-CONH_2$, un radical $-CSNH_2$ ou un groupement $-C\equiv CH$, $R_5$ représente un atome de chlore ou un radical méthyle et n représente un nombre

égal à 0, 1 ou 2, et notamment le groupement 3-phénoxy benzylique, α-cyano 3-phénoxy benzylique ou α-éthynyl 3-phénoxy benzylique,

– soit un groupement

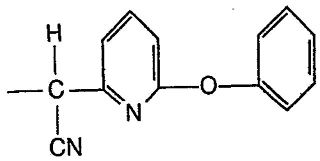

– soit un groupement

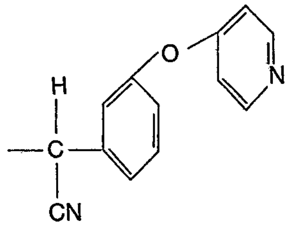

– soit un groupement

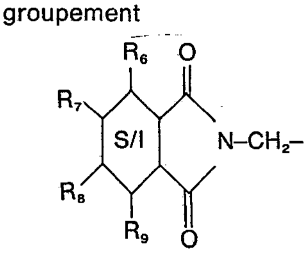

dans lequel les substituants $R_6$, $R_7$, $R_8$ et $R_9$ représentent un atome d'hydrogène, un atome de chlore ou un radical méthyle et dans lequel S/I symbolise un cycle aromatique ou un cycle analogue dihydro ou tétrahydro.

Les composés de formule (I) peuvent exister sous de nombreuses formes isomères, ils présentent tous, deux carbones asymétriques en position 1 et 3 du cycle cyclopropanique, ainsi qu'une possibilité d'isomérie E/Z au niveau de la double liaison 1', 2' et, lorsque $X_1$ et $X_2$ sont différents l'un de l'autre, il présentent également une possibilité d'isomérie E/Z au niveau de la double liaison 3', 4'.

$X_1$ et $X_2$ représentent de préférence un atome de chlore, de brome ou d'iode.

Le radical A peut posséder également 1 ou plusieurs centres d'asymétrie.

Lorsque A représente un radical alcoyle, il s'agit de préférence du radical méthyle, éthyle, n-propyle, iso-propyle ou n-butyle.

Lorsque A représente un radical benzyle substitué par un ou plusieurs radicaux alcoyle, il s'agit de préférence des radicaux méthyle ou éthyle.

Lorsque A représente un radical benzyle substitué par un ou plusieurs radicaux alcényle, il s'agit de préférence de radicaux vinyle, allyle, 2-méthylallyle, ou iso-butényle.

Lorsque A représente un radical benzyle substitué par un ou plusieurs radicaux alcényloxy, il s'agit de préférence de radicaux vinyloxy, allyloxy, 2-méthylallyloxy ou isobuténylоxy.

Lorsque A représente un radical benzyle substitué par un ou plusieurs radicaux alcadiényles, il s'agit de préférence de radicaux buta-1', 3'-diényle ou pentadiényle.

Lorsque A représente un radical benzyle substitué par un ou plusieurs atomes d'halogènes, il s'agit de préférence d'atomes de chlore ou de brome.

L'invention a notamment pour objet les composés de formule (I) pour lesquels la copule acide est de structure 1R cis ou 1R trans.

L'invention a plus particulièrement pour objet les composés de formule (I) pour lesquels $X_1$ et $X_2$ sont identiques et représentent tous les deux un atome de brome ou de chlore, et ceux pour lesquels A représente un atome d'hydrogène, ainsi que leurs sels.

Par «sels» on entend de préférence les sels formés avec les métaux alcalins, alcalino terreux, le cuivre, le zinc ou encore les sels d'amines.

L'invention a également plus particulièrement pour objet les composés de formule (I), pour lesquels A représente un radical alcoyle renfermant de 1 à 4 atomes de carbone et notamment le radical méthyle, ceux pour lesquels A représente un groupement α-cyano 3-phénoxybenzyle, ainsi que ceux pour lesquels A représente un groupement 3-allyl 2-méthyl 4-oxo cyclopent-2-en-1-yle.

L'invention a naturellement tout particulièrement pour objet les produits dont la préparation est donnée dans la partie expérimentale.

Les composés de formule (I) présentent d'intéressantes propriétés qui permettent leur utilisation dans la lutte contre les parasites, il peut s'agir par exemple de la lutte contre les parasites des végétaux et des animaux à sang chaud. C'est ainsi que l'on peut utiliser les produits de l'invention pour lutter contre les insectes, les nématodes et les acariens parasites des végétaux et des animaux.

Les produits de formule (I) peuvent donc être utilisés notamment pour lutter contre les insectes dans le domaine agricole, pour lutter par exemple contre les pucerons, les larves de lépidoptères et les coléoptères. Ils sont utilisés à des doses comprises entre 10 et 300 g de matière active à l'hectare.

Les produits de formule (I) peuvent également être utilisés pour lutter contre les insectes dans le domaine domestique, pour lutter notamment contre les mouches, les moustiques et les blattes.

Les produits de formule (I) peuvent aussi être utilisés pour lutter contre les acariens et les nématodes parasites des végétaux.

Les composés de formule (I) peuvent encore être utilisés pour lutter contre les acariens parasites des animaux, pour lutter par exemple contre les tiques et notamment les tiques de l'espèce Boophilus, ceux de l'espèce Hyalomma, ceux de l'espèce Amblyomma et ceux de l'espèce Rhipicephalus, ou pour lutter contre toutes sortes de gales et notamment la gale sarcoptique, la gale psoroptique et la gale chorioptique.

L'invention a donc également pour objet l'application des produits de formule I à la lutte contre les parasites des animaux à sang chaud et des végétaux ainsi que les compositions destinées à

cette lutte, caractérisées en ce qu'elles renferment comme principe actif au moins l'un des produits définis ci-dessus.

L'invention a notamment pour objet les compositions insecticides renfermant comme principe actif au moins l'un des produits définis ci-dessus.

Ces compositions sont préparées selon les procédés usuels de l'industrie agrochimique, de l'industrie vétérinaire ou de l'industrie des produits destinés à la nutrition animale.

Dans ces compositions la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre tel que le talc, les argiles, les silicates, le kieselguhr ou un solide combustible, tel que la poudre de tabu (ou marc de pyréthre).

Les compositions insecticides selon l'invention contiennent de préférence de 0,005 à 10% en poids de matière active.

Comme les compositions insecticides selon l'invention, les compositions acaricides et nématicides peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables pour pulvérisation foliaire, contenant de 1 à 80% de principe actif ou des liquides pour pulvérisation foliaire contenant de 1 à 500 g/litre de principe actif. On peut également employer des poudres pour poudrage foliaire, contenant de 0,05 à 3 pour cent de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention, on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo/2,2-1/-5-heptène-2,3-di carboximide, ou le pipéronyl-bis-2-(2′-n-butoxy éthoxy) ethylacétal (ou tropital).

Les composés de formule (I) présentent une excellente tolérance générale et l'invention a donc également pour objet les produits de formule (I), à titre de médicaments, pour lutter notamment contre les affections créées par les tiques et les gales.

Les médicaments de l'invention peuvent être utilisés tant en médecine humaine qu'en médecine vétérinaire.

Les médicaments de l'invention sont notamment utilisés en médecine humaine pour lutter contre les poux à titre préventif ou curatif et pour lutter contre la gale.

Les médicaments de l'invention peuvent être administrés par voie externe, par vaporisation, par bain ou badigeonnage.

Les médicaments de l'invention à usage vétérinaire peuvent être également administrés par badigeonnage de l'épine dorsale selon la méthode dite méthode «pour-on». Ils peuvent être également administrés par voie digestive ou parentérale.

L'invention a donc pour objet les compositions pharmaceutiques renfermant comme principe actif au moins l'un des médicaments définis précédemment.

Lorsqu'il s'agit de lutter contre les acariens parasites des animaux, on peut incorporer les produits de l'invention dans des compositions alimentaires en association avec un mélange nutritif adapté à l'alimentation animale. Le mélange nutritiel peut varier selon l'espèce animale, il peut renfermer des céréales, des sucres et des grains, des tourteaux de soja, d'arachide et de tournesol, des farines d'origine animale, par exemple des farines de poissons, des acides aminés de synthèse, des sels minéraux, des vitamines et des antioxydants.

L'invention a donc ainsi pour objet les compositions alimentaires définies ci-dessus.

On peut indiquer également que les produits de l'invention peuvent être utilisés comme biocides ou comme régulateurs de croissance.

Les produits de formule (I) pour lesquels A représente un atome d'hydrogène et leurs sels ainsi que les produits pour lesquels A représente un radical alcoyle renfermant de 1 à 18 atomes de carbone, sont non seulement des produits biologiquement actifs, ils sont également des intermédiaires utiles pour la synthèse de produits biologiquement actifs.

Les produits les plus intéressants d'un point de vue biologique sont les produits pour lesquels A ne représente ni un atome d'hydrogène, ni un radical alcoyle.

Les produits de formule (I) présentent d'intéressantes propriétés organoleptiques qui permettent de les utiliser notamment comme agent parfumant.

Les produits de formule (I) pour lesquels A représente un radical alcoyle renfermant de 1 à 4 atomes de carbone et notamment le radical méthyle sont ceux qui semblent le plus intéressants à utiliser en tant qu'agent parfumant. Ces produits sont doués d'une odeur florale remarquable.

En raison de leurs intéressantes propriétés olfactives, les produits de formule (I) peuvent être

utilisés comme agents odorants en parfumerie pour préparer des compositions odorantes qui peuvent servir elles-mêmes de base à des parfums.

L'invention a donc pour objet les compositions parfumantes renfermant comme principe actif au moins un produit de formule (I).

Les produits de formule (I) peuvent également être utilisés pour préparer des articles d'hygiène comme par exemple des savons, des talcs, des shampooings.

Les produits de formule (I) peuvent enfin être utilisés pour la préparation de produits détergents, comme par exemple les lessives, ou pour la préparation de produits d'entretien comme les cires.

L'invention a également pour objet un procédé de préparation des composés de formule (I), caractérisé en ce que l'on soumet le composé de formule (II)

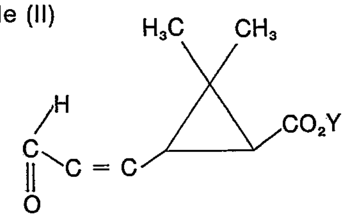

(II)

dans laquelle Y représente un atome d'hydrogène ou un radical alcoyle renfermant de 1 à 18 atomes de carbone, sous l'une quelconque de ses formes isomères ou sous forme d'un mélange d'isomères, à l'action d'un composé de formule:

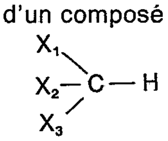

(III)

dans laquelle soit les substituants $X_1$, $X_2$ et $X_3$ sont identiques et représentent un atome de chlore ou de brome, soit au moins deux des substituants $X_1$, $X_2$ et $X_3$ sont différents, $X_1$, $X_2$, $X_3$ représentant alors un atome de fluor, de chlore ou de brome, $X_1$, $X_2$ et $X_3$ étant de poids atomiques égaux ou croissant de $X_1$ à $X_3$, pour obtenir le composé de formule

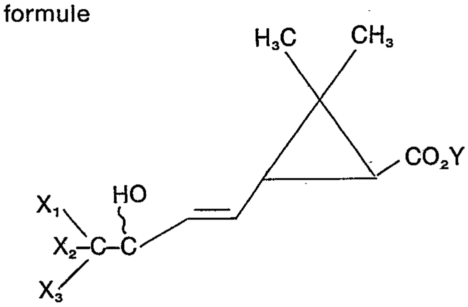

(IV)

dont on bloque l'hydroxyle par action d'un acide ou d'un dérivé fonctionnel d'acide $R-CO_2H$, puis soumet le produit obtenu de formule:

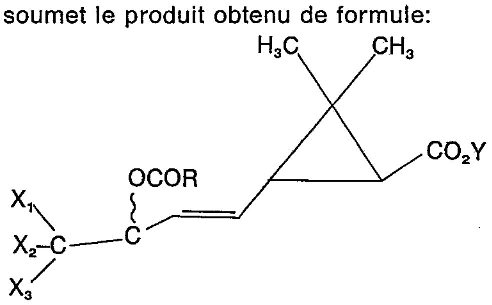

(V)

à l'action d'un agent de réduction pour obtenir après élimination du substituant $X_3$ qui est le substituant ou l'un des substituants de poids atomique le plus élevé le composé de formule

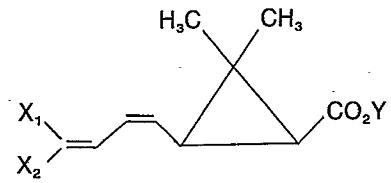

$(I_A)$

dans laquelle $X_1$ et $X_2$ soit sont identiques et représentent un atome de chlore ou de brome, soit sont différents et représentent un atome de fluor, de chlore ou de brome, puis soumet, si désiré, un composé de formule $I_A$, dans lequel Y représente un radical alcoyle renfermant de 1 à 18 atomes de carbone, à l'action d'un agent de saponification, pour obtenir un composé de formule (I) dans lequel A représente un atome d'hydrogène ou un sel de ce composé, ou soumet, si désiré, soit un composé de formule $(I_A)$ dans lequel Y est un radical alcoyle renfermant de 1 à 18 atomes de carbone, soit un composé de formule $(I_A)$ dans lequel Y représente un atome d'hydrogène, soit un dérivé fonctionnel de ce dernier, à l'action d'un alcool de formule $A_1OH$ ou d'un dérivé fonctionnel de cet alcool, formule dans laquelle $A_1$ a la même signification que A, à l'exception de l'hydrogène pour obtenir le composé de formule (I) correspondant.

Dans un mode de réalisation préféré de l'invention,

- Y représente un radical alcoyle,
- on bloque l'hydroxyle du composé de formule (IV) par action d'un anhydride, par exemple l'anhydride acétique, ou d'un halogénure d'acide comme par exemple un chlorure ou un bromure et notamment le chlorure ou le bromure d'acétyle,
- l'agent de réduction auquel on soumet le composé de formule (V) est le zinc en présence d'acide acétique ou le couple zinc-cuivre en présence d'un alcool,
- l'agent de saponification est la soude ou la potasse,
- le dérivé fonctionnel du composé $I_A$ dans lequel Y représente un atome d'hydrogène est un chlorure d'acide, mais il peut être également un sel de métal alcalin, par exemple un sel de sodium ou de potassium, la réaction étant alors effectuée sur un dérivé halogéné d'un alcool, par exemple un bromure ou un chlorure.

L'invention a également pour objet une variante du procédé décrit ci-dessus, caractérisée en ce que l'on soumet selon la réaction de Wittig le composé de formule (II) telle que définie précédemment, sous l'une quelconque de ses formes isomères ou sous forme d'un mélange d'isomères, à l'action d'un composé de formule

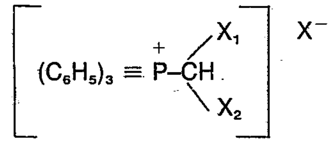

dans laquelle $X_1$, $X_2$ sont définis comme précédemment et $X^-$ représente un anion halogénure pour obtenir le composé de formule ($I_A$) puis, si désiré, poursuit la synthèse comme décrit précédemment.

L'invention a plus particulièrement pour objet un procédé de préparation des composés de formule (I), caractérisé en ce que le composé de formule (II) est de structure 1R cis ou 1R trans.

La réaction de Wittig ci-dessus est effectuée en présence d'une base forte qui peut être un hydrure alcalin, un amidure alcalin, un alcoyllithien ou un alcoolate alcalin, de préférence un terbutylate de sodium, de potassium ou de lithium.

Les composés de formule (II) utilisés comme produits de départ sont des produits connus d'une façon générale, de toute façon ils peuvent être préparés selon le procédé décrit par Elliott et Coll dans J. Chem. Soc. Perkin 1, p. 2470 et suivantes.

L'acide (1R cis) 2,2-diméthyl 3-(3'-oxo 1'-propényl) cyclopropane carboxylique est un produit nouveau et l'invention a pour objet ce produit à titre de produit industriel nouveau et plus particulièrement à titre de produit intermédiaire nécessaire à la mise en œuvre du procédé de l'invention.

Les composés de formule (IV) et (V) obtenus lors de la mise en œuvre du procédé sont des produits chimiques nouveaux et l'invention a également pour objet les composés de formule (IV) et (V), à titre de produits industriels nouveaux, et plus particulièrement à titre de produits intermédiaires nécessaires à la mise en œuvre du procédé de l'invention.

Les exemples suivants illustrent l'invention sans toutefois la limiter.

Préparation: Acide (1R; cis) 2,2-diméthyl 3-(3'-oxo 1'-propényl) cyclopropane carboxylique.

On mélange 76,5 g de ter-butylate de potassium, 374 $cm^3$ d'heptane, ajoute 63,8 $cm^3$ de ter-butanol et 66 $cm^3$ d'heptane sous atmosphère inerte. A 20 °C on introduit, peu à peu, 159,5 g de bromure de triphényl/(1,3-dioxolan-2-yl) méthyl/ phosphonium préparé ci-après. On refroidit à − 10 °C, ajoute 44 g de 1R, 5S) 6,6-diméthyl 4(R)-hydroxy 3-oxo bicyclo/3-1-0/hexane 2-one dans 180 $cm^3$ de tétrahydrofurane, maintient à − 10 °C pendant 1 heure puis 17 heures à température ambiante. On concentre à sec sous pression réduite, reprend le résidu par 1400 $cm^3$ de soude 0,25N et 320 $cm^3$ de chlorure de méthylène et on décante. A la phase aqueuse, traitée au charbon actif, on ajoute 176 $cm^3$ de tétrahydrofurane et 70 $cm^3$ d'acide chlorhydrique concentré, puis agite 3 heures 30 minutes à 20 °C. On extrait au chlorure de méthylène, lave à l'eau, sèche, concentre à sec, reprend le résidu par de l'éther isopropylique, agite pendant une demi-heure à 0 °C, essore et obtient 23,7 g de produit attendu F = 129 °C.

Le bromure de triphényl (1,3-dioxolan-2-yl mètyl) phosphonium utilisé au départ de la préparation a été préparé comme suit:

On chauffe à 80 °C pendant 36 heures, 180 g de 2(bromoéthyl) 1,3-dioxolan et 260 g de triphénylphosphine. On refroidit, dissout le produit obtenu dans le chlorure de méthylène et verse lentement sur 3000 $cm^3$ d'éther éthylique. Après agitation, on essore, empâte avec de l'éther éthylique et obtient 359 g de produit F = 206 °C.

Exemple 1

(Acide (1R,cis) 2,2-diméthyl 3-(4',4'-dichloro buta − 1',3'-diènyl) cyclopropane carboxylique.

Stade A: Acide (1R,cis) 2,2-diméthyl 3-(4',4',4'-trichloro 3'-hydroxy buta 1'-ènyl) cyclopropane carboxylique.

On mélange 10 g d'acide (1R,cis) 2,2-diméthyl 3-(3'-oxo 1'-propényl) cyclopropane carboxylique obtenu à la préparation 1 et 24 $cm^3$ de chloroforme, agite sous atmosphère inerte, refroidit à − 10 °C et introduit goutte à goutte une solution préparée avec 9,2 g de méthylate de potassium, 39 $cm^3$ de butanol et 32 $cm^3$ de tétrahydrofuranne. On agite pendant 2 heures le milieu réactionnel à une température voisine de − 5 °C–0 °C, puis, le verse dans 300 $cm^3$ d'un mélange eau-glace et 13 $cm^3$ d'acide chlorhydrique concentré. On décante, extrait au chloroforme, lave à l'eau, sèche, concentre à sec sous pression réduite. On reprend le résidu à l'éther de pétrole (eb 60–80 °C) essore et obtient 9,3 g de produit attendu F = 138 °C.

Stade B: Acide (1R,cis) 2,2-diméthyl 3-(4',4',4'-trichloro 3'-acétoxy but-1-ényl) cyclopropane carboxylique.

A température ambiante, on agite pendant 4 heures 8 g du produit obtenu au stade précédent dans 22,5 $cm^3$ de pyridine et 11,5 $cm^3$ d'anhydride acétique, ajoute de la glace, agite, puis verse la solution dans un mélange de 30 $cm^3$ d'acide chlorhydrique concentré et 150 $cm^3$ d'eau glacée. On extrait au chloroforme, lave à l'eau, sèche, traite au charbon actif, concentre à sec sous pression réduite et obtient 9,56 g de produit huileux utilisé tel quel au stade suivant.

Stade C: Acide (1R,cis) 2,2-diméthyl 3-(4',4'-dichloro buta 1',3'-diényl) cyclopropane carboxylique.

On mélange 9,5 g du produit obtenu ci-dessus et 95 $cm^3$ d'acide acétique à 10% d'eau et ajoute, sous agitation, 5,65 g de zinc en poudre. On agite pendant 4 heures à 35 °C environ, concentre à sec sous pression réduite, reprend le résidu par l'eau et extrait au chloroforme. Les phases organiques sont lavées à l'eau, traitées au charbon actif, séchées, concentrées à sec et lavées au toluène. On obtient 6,5 g de produit huileux que l'on chromatographie sur silice, élue par un mélange éther de pétrole (eb 40–70 °C) – éther éthylique (75–25) et récolte 2,75 g de produit attendu.

Spectre RMN ($CDCl_3$)

1,225–1,3 ppm pics attribués aux hydrogènes des méthyles.

1,66 à 2,08 ppm pics attribués aux hydrogènes du cyclopropyle.

6,17 à 6,58 ppm pics attribués aux hydrogènes éthyléniques.

Exemple 2

(1R,trans) 2,2-diméthyl 3-(4',4'-dichloro buta-1',3'-diènyl) cyclopropane carboxylate de méthyle.

Stade A: (1R,trans) 2,2-diméthyl 3-(4',4',4'-trichloro 3'-hydroxy but-1-ényl) cyclopropane carboxylate de méthyle.

On agite 11,6 g de (1R,trans) 2,2-diméthyl 3-(3'-oxo 1'-propényl)cyclopropane carboxylate de méthyle préparé selon le procédé décrit par Elliot et Coll (J. Chem. Soc. 1975 p. 2470 et suivantes), dans 15 $cm^3$ de chloroforme, refroidit à −5–−10 °C et introduit 5,25 g de méthylate de potassium dans 20 $cm^3$ de ter-butanol et 15 $cm^3$ de tétrahydrofuranne. Après 2 heures à −5°C sous agitation, la suspension est versée dans 100 $cm^3$ d'un mélange glace-eau et 7,5 $cm^3$ d'acide chlorhydrique concentré. On agite, décante, extrait au chloroforme, sèche, concentre à sec à 50 °C sous pression réduite, recristallise dans l'éther de pétrole (eb 60–80°C) et obtient 12,2 g du produit attendu F = ~20–25 °C.

Stade B: (1R,trans) 2,2-diméthyl 3-(4',4',4'-trichloro 3'-acétoxy but-1'-ényl) cyclopropane carboxylate de méthyle.

A température ambiante, on agite pendant 4 heures 33,5 $cm^3$ de pyridine, 12,2 g de produit obtenu ci-dessus et 16,8 $cm^3$ d'anhydride acétique. On ajoute 40 g de glace, agite pendant 30 minutes et verse le mélange réactionnel sur 60 g d'un mélange glace-eau et 39 $cm^3$ d'acide chlorhydrique concentré. Après 10 minutes on extrait au chlorure de méthylène, lave sèche, concentre à sec à 50 °C sous pression réduite et obtient 11,8 g de produit attendu.

Stade C: (1R,trans) 2,2-diméthyl 3-(4', 4'-dichloro buta-1',3'-diényl)cyclopropane carboxylate de méthyle.

On mélange 11,8 g du produit obtenu au stade B et 236 $cm^3$ d'acide acétique à 10% d'eau, ajoute 6,73 g de poudre de zinc et chauffe à 55 °C pendant 4 heures. On laisse refroidir à 20 °C, filtre l'insoluble et concentre à sec le filtrat à 60 °C sous pression réduite. On reprend le résidu par 125 $cm^3$ de chlorure de méthylène et 100 $cm^3$ d'eau, agite, décante, lave la phase organique par une solution aqueuse saturée de bicarbonate de soude et réextrait par du chlorure de méthylène, lave à l'eau, sèche, concentre à sec sous pression réduite et obtient 9 g de produit. On le purifie par chromatographie sur silice, élue par un mélange cyclohexane-acétate d'éthyle (9–1) et obtient 4,3 g de produit huileux.

Spectre RMN ($CDCl_3$)

1,18–1,28 ppm pics attribués aux hydrogènes des méthyles géminés.

1,58–1,67 ppm pics attribués à l'hydrogène en 1 du cyclopropyle.

2 à 2,21 ppm pics attribués à l'hydrogène en 3 du cyclopropyle.

3,68 ppm pic attribué aux hydrogènes du méthyle de l'ester.

5,3 à 5,18 ppm pics attribués à l'hydrogène éthylénique en position 1 de la chaîne latérale.

6,1 à 6,6 ppm pics attribués aux autres hydrogènes éthyléniques en position 2 et 3 de la chaîne latérale.

Exemple 3

(Acide (1R,trans) 2,2-diméthyl 3-(4',4'-dichloro buta 1',3'-diènyl) cyclopropane carboxylique).

On chauffe au reflux pendant 4 heures 4,3 g de (1R,trans) 2,2-diméthyl 3-(4',4'-dichloro buta 1',3'-diényl) cyclopropane carboxylate de méthyle obtenu à l'exemple 2, 114 $cm^3$ de méthanol et 17,2 $cm^3$ de soude 2N. On concentre à sec à 50 °C sous pression réduite, reprend l'extrait sec avec 50 $cm^3$ d'eau et extrait à l'éther éthylique. La phase aqueuse est amenée à pH 1 avec de l'acide chlorhydrique 2N et extrait à l'éther éthylique. On sèche la phase organique, amène à sec à 30 °C sous pression réduite, puis une heure à 50 °C. On obtient 3,8 g de produit.

Spectre RMN ($CDCl_3$)

1,16–1,28 ppm pics attribués aux hydrogènes des méthyles géminés.

1,56–1,65 ppm pics attribués à l'hydrogène en 1 du cyclopropyle.

2,02–2,11–2,15–2,24 ppm pics atribués à l'hydrogène en 3 du cyclopropyle.

5,3 à 5,8 ppm pics attribués à l'hydrogène éthylénique en 1 de la chaîne latérale.

6,2 à 6,4 ppm pics attribués aux 2 autres hydrogènes éthyléniques.

11,3 ppm pic attribué à l'hydroxyle.

Exemple 4

(Acide (1R,cis) 2,2-diméthyl 3-(4',4'-dibromo buta-1',3'-diényl) cyclopropane carboxylique.

On chauffe à 75 °C sous atmosphère inerte, 24 $cm^3$ de ter-butanol, 16 $cm^3$ d'heptane et 6,6 g de ter-butylate de potassium. A 20 °C on ajoute 14,7 g de bromure de dibromo méthyl triphényl phosphorium. Après refroidissement à 0 °C, on introduit 4 g d'acide (1R,cis) 2,2-diméthyl 3-(3'-oxo 1'-propényl) cyclopropane carboxylique obtenu à la préparation 1 et agite encore pendant 1 heure à cette température. On chauffe le milieu réactionnel à 50 °C pendant 1 heure puis concentre à sec sous pression réduite. On reprend le résidu par 30 $cm^3$ de soude N et 20 $cm^3$ de chlorure de méthylène et ajoute 96 $cm^3$ d'eau distillée. On décante, lave au chlorure de méthylène. On agite les phases organiques avec 20 $cm^3$ de soude 0,25N. On réunit les phases aqueuses, les traite au charbon actif et ajoute 4 $cm^3$ d'acide chlorhydrique concentré. Le produit gommeux obtenu est dissous dans le chlorure de méthylène. On concentre à sec, à 40 °C, sous pression réduite et obtient 5,68 g de produit huileux qui, chromatographié sur silice en éluant par un mélange éther de pétrole (eb 40–70 °C)-éther éthylique (75–25) donne 3 g de produit.

Spectre RMN ($CDCl_3$)

1,23–1,3 ppm pics attribués aux hydrogènes des méthyles géminés.

1,75 à 2,33 ppm pics attribués aux hydrogènes du cyclopropyle.

6,2 à 6,4 et 6,9 à 7,1 ppm pics attribués aux hydrogènes éthyléniques.

Exemple 5

(1R,trans) 2,2-diméthyl 3-(4',4'-dibromo buta 1',3'-diényl) cyclopropane carboxylate de méthyle.

Sous agitation et sous atmosphère inerte, on introduit à 20 °C, 9,74 g de ter-butylate de potassium dans 100 $cm^3$ d'heptane, ainsi que 7,95 $cm^3$ de ter-butanol. On ajoute ensuite lentement et sous agitation 43,5 g de bromure de dibromo méthyl triphényl phosphonium et agite pendant 1 heure. On refroidit à −10 °C, ajoute 12,8 g de (1R,trans) 2,2-diméthyl 3-(3'-oxo 1'-propényl) cyclopropane carboxylate de méthyle dans 40 $cm^3$ de tétrahydrofuranne et agite pendant une heure à −10 °C et pendant 2 jours à température ambiante. On concentre à sec sous pression réduite, reprend le résidu par un mélange glace-eau, extrait au chlorure de méthylène, lave avec une solution saturée de phosphate monosodique, sèche, amène à sec sous pression réduite. On chromatographie le résidu sur silice, élue au chlorure de méthylène, concentre à sec et obtient 19,8 g de produit attendu $n_D^{23}$ = 1,561.

Spectre RMN ($CDCl_3$)

1,15–1,25 ppm pics attribués aux hydrogènes des méthyles géminés.

1,58–1,675 ppm attribués à l'hydrogène en 1 du cyclopropyle.

3,7 ppm pic attribué aux hydrogènes du méthyle (ester).

1,99–2,08 ppm et 2,12–2,21 ppm pics attribués à l'hydrogène en 3 du cyclopropyle.

5,4–5,5 et 5,6–5,8 ppm pics attribués à l'hydrogène éthylénique de la chaîne latérale.

6,0–6,2 ppm 6,3–6,5 ppm pics attribués à l'hydrogène en 2 de la chaîne latérale.

6,8–7,0 ppm pics attribués à l'hydrogène en 3 de la chaîne latérale.

Exemple 6

Acide (1R,trans) 2,2-diméthyl 3-(4',4'-dibromo buta-1',3'-diényl) cyclopropane carboxylique.

On opère comme à l'exemple 3 en partant de 14 g de (1R,trans) 2,2-diméthyl 3-(4',4'-dibromo buta-1',3'-diényl) cyclopropane carboxylate de méthyle obtenu à l'exemple 5 et en extrayant à l'éther isopropylique. On obtient, après chromatographie sur silice, en éluant par un mélange chlorure de méthylène-acétate d'éthyle (9–1) 6,62 g de produit attendu.

Spectre RMN ($CDCl_3$)

1,3–1,32 ppm pics attribués aux hydrogènes des méthyles géminés.

1,6–1,68 ppm pics attribués à l'hydrogène en 1 du cyclopropyle.

2 à 2,25 ppm pics attribués à l'hydrogène en 3 du cyclopropyle.

5,4 à 5,8 ppm pics attribués à l'hydrogène éthylénique en 1 sur la chaîne latérale.

6,1–6,3 ppm – 6,3–6,5 ppm pics attribués à l'hydrogène éthylénique en 2 sur la chaîne latérale.

6,8–7,0 ppm pics attribués à l'hydrogène éthylénique en 3 sur la chaîne latérale.

Exemple 7

(1R,cis) 2,2-diméthyl 3-(4',4'-dichloro buta 1',3'-diényl) cyclopropane carboxylate de (S) α-cyano 3-phénoxybenzyle.

Stade A: chlorure d'acide (1R,cis) 2,2-diméthyl 3-(4',4'-dichloro buta 1',3'-diényl) cyclopropane carboxylique.

Pendant 4 heures on porte au reflux 2,75 g d'acide (1R,cis) 2,2-diméthyl 3-(4'4'-dichloro buta 1',3'-diényl) cyclopropane carboxylique obtenu à l'exemple 1, 28 $cm^3$ d'éther de pétrole (eb 40–70 °C) et 8,25 $cm^3$ de chlorure de thionyle. On concentre à sec à 35 °C sous pression réduite, et obtient 2,91 g de produit attendu.

Stade B: (1R,cis) 2,2-diméthyl 3-(4',4'-dichloro buta 1',3'-diényl cyclopropane carboxylate de (S) α-cyano 3-phénoxybenzyle.

Au produit obtenu précédemment on ajoute 30 $cm^3$ de benzène et 2,7 g d'alcool (S) α-cyano 3-phénoxybenzylique. A 15 °C on introduit, goutte à goutte, un mélange de 4,44 $cm^3$ de pyridine et 9 $cm^3$ de benzène et agite pendant 17 heures à température ambiante. On verse le mélange réactionnel dans 100 $cm^3$ d'acide chlorhydrique 2N, agite, décante. On lave à l'eau la phase organique, traite au charbon actif, sèche, concentre à sec à 35–40 °C et obtient 4,87 g de produit.

Après chromatographie sur silice, on recristallise le produit dans un mélange hexane-éther isopropylique (1–1) et obtient 1,5 g du produit attendu F = 65 °C.

$\alpha_D$ = +53° ± 2,5 (c = 0,5% benzène).

Spectre RMN ($CDCl_3$)

1,23 ppm pic attribué aux hydrogènes des méthyles géminés.

1,77 à 2,2 ppm pics attribués aux hydrogènes du cyclopropyle.

5,8 à 6,7 ppm pics attribués aux hydrogènes éthyliques.

6,5 ppm pic attribué à l'hydrogène porté par le même carbone que CN.

7 à 7,6 ppm pics attribués aux hydrogènes aromatiques.

Exemple 8

(1R,trans) 2,2-diméthyl 3-(4',4'-dichloro buta 1',3'-diényl) cyclopropane carboxylate de (RS) α-cyano 3-phénoxybenzyle.

Stade A: Chlorure d'acide (1R,trans) 2,2-diméthyl 3-(4',4'-dichloro buta 1',3'-diényl) cyclopropane carboxylique.

On opère comme au stade A de l'exemple 7 en partant de 2,32 g d'acide (1R,trans) 2,2-diméthyl 3-(4',4'-dichloro buta 1',3'-diényl) cyclopropane carboxylique obtenu à l'exemple 3 et obtient 2,5 g de produit attendu.

Stade B: (1R,trans) 2,2-diméthyl 3-(4',4'-dichloro buta 1',3'-diényl) cyclopropane carboxylate de (RS) α-cyano 3-phénoxybenzyle.

D'une manière analogue à celle décrite au stade B de l'exemple 7, on obtient 4,90 g de produit brut, en partant du produit obtenu ci-dessus et de 2,3 g d'alcool (RS) α-cyano 3-phénoxybenzylique. Après chromatographie sur silice (éluant

éther de pétrole (eb 40–70 °C) éther éthylique (75/25)) on obtient 2,46 g de produit attendu.

$\alpha_D$ = −25°6 ± 2,5 (c = 0,6% benzène).

Spectre RMN ($CDCl_3$)

1,17–1,2–1,3 ppm pics attribués aux hydrogènes des méthyles géminés.

1,55 à 1,72 ppm pics attribués à l'hydrogène en position 1 du cyclopropyle.

2 à 2,33 ppm pics attribués à l'hydrogène en position 3 du cyclopropyle.

6,3 ppm pic attribué à l'hydrogène porté par le même carbone que CN et aux hydrogènes en position 2 et 3 de la chaîne latérale éthylénique.

5,3 à 5,8 ppm pics attribués à l'hydrogène en position 1 de la chaîne latérale éthylénique.

6,9 à 7,7 ppm pics attribués aux hydrogènes aromatiques.

Exemple 9

(1R,trans) 2,2-diméthyl 3-(4',4'-dichloro buta 1',3'-diényl) cyclopropane carboxylate de (S) α-cyano 3-phénoxybenzyle.

On opère comme au stade B de l'exemple 7 en partant de 1,4 g de chlorure d'acide (1R,trans) 2,2-diméthyl 3-(4',4'-dichloro buta 1',3'-diényl) cyclopropane carboxylique obtenu au stade A de l'exemple 8 et de 1,35 g d'alcool (S) α-cyano 3-phénoxybenzylique. On obtient 2,54 g de produit huileux. On le chromatographie sur silice, élue par un mélange cyclohexane, acétate d'éthyle (85–15) et isole 1,86 g de produit, qui recristallisé dans l'éther de pétrole (eb 40–70 °C) donne 1,3 g de produit attendu F = 78 °C.

$\alpha_D$ = +6° ± 2 (c = 0,6% benzène).

Spectre RMN ($CDCl_3$)

1,17–1,23 ppm pics attribués aux hydrogènes des méthyles géminés.

1,66–1,75 ppm pics attribués à l'hydrogène en position 1 du cyclopropyle.

2,1–2,19–2,24–2,33 ppm pics attribués à l'hydrogène en position 3 du cyclopropyle.

5,2 à 5,8 ppm pics attribués à l'hydrogène en position 3 de la chaîne latérale éthylénique.

6,2 à 6,6 ppm pics attribués aux hydrogènes en position 1 et 2 de la chaîne latérale éthylénique.

6,3 à 6,5 ppm pics attribués à l'hydrogène porté par le même carbone que le CN.

7,0 à 7,6 ppm pics attribués aux hydrogènes aromatiques.

Exemple 10

(1R,cis) 2,2-diméthyl 3-(4',4'-dibromo buta 1',3'-diényl) cyclopropane carboxylate de (S) α-cyano 3-phénoxybenzyle.

Stade A: chlorure d'acide (1R,cis) 2,2-diméthyl 3-(4',4'-dibromo buta 1',3'-diényl) cyclopropane carboxylique.

On opère comme à l'exemple 7, stade A, en partant de 7 g d'acide (1R,cis) 2,2-diméthyl 3-(4',4'-dibromo buta 1',3'-diényl) cyclopropane carboxylique obtenu à l'exemple 4 et obtient 7,6 g de produit attendu.

Stade B: (1R,cis) 2,2-diméthyl 3-(4',4'-dibromo buta 1',3'-diényl) cyclopropane carboxylate de (S) α-cyano 3-phénoxy benzyle.

De la même façon qu'au stade B de l'exemple 7 on prépare 5,83 g de produit brut à partir du produit obtenu ci-dessus et de 2,9 g d'alcool (S) α-cyano 3-phénoxybenzylique. On chromatographie sur silice (éluant: éther de pétrole – éther éthylique (75/25)) et obtient 1,61 g de produit attendu.

$\alpha_D$ = +46°3 ± 2 (c = 0,4% benzène).

Spectre RMN ($CDCl_3$)

1,22–1,23 ppm pics attribués aux hydrogènes des méthyles géminés.

6,4 ppm pic attribué à l'hydrogène porté par le même carbone que le CN.

6,2 à 6,5 ppm pics attribués aux hydrogènes éthyléniques.

6,9 à 7,7 ppm pics attribués aux hydrogènes aromatiques.

Exemple 11

(1R,trans) 2,2-diméthyl 3-(4',4'-dibromo buta 1',3'-diényl) cyclopropane carboxylate de (RS) α-cyano 3-phénoxybenzyle.

Stade A: Chlorure d'acide (1R,trans) 2,2-diméthyl 3-(4',4'-dibromo buta 1',3'-diényl) cyclopropane carboxylique.

On opère comme au stade A de l'exemple 7 en partant de 1,8 g d'acide (1R,trans) 2,2-diméthyl 3-(4',4'-dibromo buta 1',3'-diényl) cyclopropane carboxylique obtenu à l'exemple 6 et obtient 1,9 g de produit attendu.

Stade B: (1R,trans) 2,2-diméthyl 3-(4',4'-dibromo buta 1',3'-diényl) cyclopropane carboxylate de (RS) α-cyano 3-phénoxybenzyle.

On traite le produit obtenu ci-dessus de la même façon qu'au stade B de l'exemple 7 avec 1,4 g d'alcool (RS) α-cyano phénoxybenzylique et obtient 2,7 g de produit brut. On chromatographie ce dernier sur silice, élue par le système hexane-éther isopropylique (8–2) et recueille 1,33 g de produit attendu.

$\alpha_D$ = −14° ± 2,5 (c = 0,5% benzène). Spectre RMN ($CDCl_3$)

1,18–1,23 ppm pics attribués aux hydrogènes des méthyles géminés du produit S.

1,23–1,32 ppm pics attribués aux hydrogènes des méthyles géminés du produit R.

1,65 à 1,75 ppm et 2,05 à 2,38 ppm pics attribués aux hydrogènes du cyclopropyle,

de 6,9 à 7,1 ppm pics attribués à l'hydrogène dédoublé en 3 de la chaîne latérale.

5,4 à 5,9 ppm pics attribués à l'hydrogène en 1 de la chaîne latérale,

de 6,1 à 6,6 ppm pics attribués à l'hydrogène dédoublé en 2 de la chaîne latérale.

6,4 ppm pic attribué à l'hydrogène porté par le même carbone que le $-C\equiv N$.

6,9 à 7,6 ppm pics attribués aux hydrogènes aromatiques.

Exemple 12

(1R,trans) 2,2-diméthyl 3-(4',4'-dibromo buta 1',3'-diényl) cyclopropane carboxylate de (S) α-cyano 3-phénoxybenzyle.

On opère comme au début du stade B de l'exemple 7 à partir du chlorure d'acide (1R,trans) 2,2-diméthyl 3-(4',4'-dibromo buta 1',3'-diényl) cyclopropane carboxylique obtenu à l'exemple 11 et de 6,11 g d'alcool (S) α-cyano 3-phénoxy-benzylique.

Après agitation pendant 17 heures à 20 °C on verse le mélange réactionnel dans 200 $cm^3$ d'eau, agite, décante et extrait au benzène. Les phases organiques sont lavées avec de l'acide chlorhydrique 0,1N puis à l'eau. On sèche, concentre à sec sous pression réduite et obtient 15,4 g de produit. Après 2 chromatographies sur silice (1er éluant hexane-acétate d'éthyle (9–1); 2[e] éluant hexane-éther isopropylique (8–2) on isole 4,4 g de produit que l'on recristallise dans le mélange hexane-éther isopropylique (1–1). On obtient 2,2 g de produit F = 110 °C.

$\alpha_D$ = +16° ± 1° (c = 1% benzène).

Spectre RMN ($CDCl_3$)

1,2–1,16 ppm pics attribués aux hydrogènes des méthyles géminés,

de 2,1 à 2,3 ppm pics attribués à l'hydrogène en position 3 du cyclopropane.

166–1,75 ppm pics attribués à l'hydrogène en position 1 du cyclopropane,

de 5,4 à 5,8 ppm pics attribués à l'hydrogène éthylénique en position 1 de la chaîne latérale,

de 6,1 à 6,5 ppm pics attribués à l'hydrogène éthylénique en position 2 de la chaîne latérale.

6,8–7 ppm pics attribués à l'hydrogène éthylénique en position 3 de la chaîne latérale.

6,4 ppm pic attribué à l'hydrogène porté par le même carbone que le CN,

de 6,9 à 7,6 ppm pics attribués aux hydrogènes aromatiques.

Exemple 13

(1R,cis) 2,2-diméthyl 3-(4',4'-dichloro buta 1',3'-diényl) cyclopropane carboxylate de (S) 3-allyl 2-méthyl 4-oxo cyclopent 2-en-1-yle.

On opère selon la technique employée à l'exemple 7 à partir de 2,5 g d'acide (1R,cis) 2,2-diméthyl 3-(4',4'-dichloro buta-1',3'-diényl) cyclopropane carboxylique préparé à l'exemple 1 et 1,8 g de (S) 3-allyl 1-hydroxy 2-méthyl 4-oxo cyclopent-2-ène. On obtient 3,23 g de produit brut et après chromatographie sur silice (éluant: hexane-acétate d'éthyle 9/1) 600 mg de produit pur.

$\alpha_D$ = +14° ± 2° (c = 0,4% benzène).

Spectre RMN ($CDCl_3$)

1,22–1,28 ppm pics attribués aux hydrogènes des méthyles géminés.

2 ppm pic attribué aux hydrogènes du mèthyle de l'allethrone.

2,93 –3,0 ppm pics attribués aux hydrogènes en position 1 de l'allyle.

4,7 à 5,2 ppm pics attribués aux hydrogènes en position 3 de l'allyle.

5,6–5,7 ppm pics attribués à l'hydrogène porté par le même carbone que CN.

5,4 à 6,3 ppm pics attribués à l'hydrogène en position 2 de l'allyle.

6 à 6,5 ppm pics attribués aux hydrogènes éthyléniques sur la chaîne latérale en 3 du cyclopropyle.

Exemple 14

(1R,trans) 2,2-diméthyl 3-(4',4'-dichloro buta 1',3'-diényl) cyclopropane carboxylate de (S) 3-allyl 2-méthyl 4-oxo cyclopent-2-en-1-yle.

On opère comme à l'exemple 7 à partir de 1,53 g d'acide (1R,trans) 2,2-diméthyl 3-(4',4'-dichloro buta-1',3'-diényl) cyclopropane carboxylique, obtenu à l'exemple 3 et 1,07 g de (S) 3-allyl 1-hydroxy 2-méthyl 4-oxo cyclopent-2-ène. On obtient 2,17 g de produit brut et après chromatographie sur silice (éluant benzène – acétate d'éthyle – 9/1) 850 mg de produit pur.

$n_D^{23,5}$ = 1,5539 $\alpha_D$ = −76° ± 2,5 (c = 0,4% benzène).

Spectre RMN ($CDCl_3$)

1,17–1,25 ppm pics attribués aux hydrogères des méthyles géminés.

2,0 ppm pic attribué aux hydrogènes du méthyle de l'allethrolone.

1,6–1,69 ppm pics attribués à l'hydrogène en position 1 du cyclopropyl.

4,7 à 5,2 pics attribués aux hydrogènes en position 3 de l'allyle.

5,3 à 6,2 ppm pics attribués à l'hydrogène en position 2 de l'allyle et à l'hydrogène en position 1 de la chaîne latérale éthylénique du cyclopropyle.

6,2 à 6,5 ppm pics attribués aux hydrogènes éthyléniques en position 2 et 3 de la chaîne latérale du cyclopropyle.

Exemple 15

(1R,trans) 2,2-diméthyl 3-(4',4'-dibromo buta 1',3'-diényl) cyclopropane carboxylate de (S) 3-allyl 2-méthyl 4-oxo cyclopent-2-en-1-yle.

On opère selon la technique employée à l'exemple 7 à partir de 2,0 g d'acide (1R,trans) 2,2-diméthyl 3-(4',4'-dibromo buta 1',3'-diényl) cyclopropane carboxylique préparé à l'exemple 6 et de 1 g de (S) 3-allyl 1-hydroxy 2-méthyl 4-oxo cyclopent-2-ène. On obtient 2,5 g de produit brut et après chromatographie sur silice (système: hexane éther isopropylique – 8/2) 1,5 g de produit pur.

$\alpha_D$ = −55°5 ± 3° (c = 0,3% benzène).

Spectre RMN ($CDCl_3$)

1,2–1,28 ppm pics attribués aux hydrogènes des méthyles géminés.

1,62–1,71 ppm pics attribués à l'hydrogène en position 1 du cyclopropyle.

2,03 ppm pic attribué aux hydrogènes du méthyle de l'alléthrolone.

4,8–5,2 ppm pics attribués aux hydrogènes en position 3 de l'allyle.

5,4 à 6,5 ppm pics attribués à l'hydrogène en position 2 de l'allyle.

6,9–7,0 ppm pics attribués à l'hydrogène en position 3 de la chaîne éthylénique latérale du cyclopropyle,

de 6,1 à 6,5 ppm pics attribués à l'hydrogène en position 2 de la chaîne éthylénique latérale du cyclopropyle.

5,43 à 5,83 ppm pics attribués à l'hydrogène éthylénique en position 1 de la chaîne latérale du cyclopropyle.

Exemple 16

(1R,cis) 2,2-diméthyl 3-(4',4'-dibromo buta 1',3'-diényl) cyclopropane carboxylate de (S) 3-allyl 2-méthyl 4-oxo cyclopent-2-en-1-yle.

On opère selon la technique employée à l'exemple 7 à partir de 6,2 g d'acide (1R,cis) 2,2-diméthyl 3-(4',4'-dibromo buta 1',3'-diényl) cyclopropane carboxylique préparé à l'exemple 4 et 0,98 g de (S) 3-allyl 1-hydroxy 2-méthyl 4-oxo cyclopent-2-ène. On obtient 2,2 g de produit brut que l'on chromatographie sur silice (éluant hexane – éther isopropylique 8/2) pour isoler 760 mg de produit attendu.

$\alpha_D = +1,25° \pm 1$ (c = 1,5% benzène).

Spectre RMN ($CDCl_3$)

1,22–1,28 ppm pics attribués aux hydrogènes des méthyles géminés.

2,0 ppm pic attribué aux hydrogènes du méthyle de l'allethrolone.

4,8 à 5,2 ppm pics attribués aux hydrogènes en position 3 de l'allyle.

5,7 ppm pic attribué à l'hydrogène en position 1 de l'allethrolone.

5,5 à 7,1 ppm pics attribués aux hydrogènes éthyléniques.

Préparation

(1R,cis,ΔZ) 3-(2'-formyl éthényl) 2,2-diméthyl cyclopropane carboxylate de terbutyle.

On mélange 3,4 g de (1R,cis) 2,2-diméthyl 3-(3',3'-diéthoxy prop-1-èn-1-yl) cyclopropane carboxylate de terbutyle (préparé selon H.J. Bestmann, K. Roth et M. Ettinger. Angew. Chem. Int. 18 [1979] n° 9), 5 cm³ d'eau et 20 cm³ d'acétone, refroidit à 0, +5°C et ajoute quelques cristaux d'acide paratoluène sulfonique. On agite à (0, +5°C) pendant 45 minutes, verse le mélange sur une solution aqueuse glacée saturée de bicarbonate de sodium, extrait au pentane, sèche, évapore le solvant et obtient 2,42 g de produit attendu.

Spectre RMN ($CDCl_3$)

1,28–1,33 ppm pics attribués aux $CH_3$ géminés.

2,47–2,78 ppm pics attribués à l'hydrogène en 3.

1,87–2,01 ppm pics attribués à l'hydrogène en 1.

1,45 ppm pic attribué aux hydrogènes du terbutyle.

6,85–7,3 ppm pics attribués à l'hydrogène en 1'.

5,93–6,06 et 6,13–6,27 ppm pics attribués à l'hydrogène en 2'.

10,1–10,2 ppm pics attribués à l'hydrogène en 3'.

Exemple 17

Acide (1R,cis,ΔZ) 2,2-diméthyl 3-(4',4'-dibromo buta 1',3'-diényl) cyclopropane carboxylique.

Stade A: (1R,cis,ΔZ) 2,2-diméthyl 3-(4',4'-dibromo buta 1',3'-diényl) cyclopropane carboxylate de terbutyle.

On mélange 3 cm³ d'heptane, 4,5 cm³ de terbutanol et 0,28 g de terbutylate de lithium, agite à 20°C pendant 15 minutes puis ajoute 2,27 g de bromure de dibromométhyltriphénylphosphonium. On agite à 20°C pendant 1 heure, refroidit à −60°C et ajoute goutte à goutte une solution de 0,64 g de (1R,cis,ΔZ) 3-(2'-formyl éthényl) 2,2-diméthyl cyclopropane carboxylate de terbutyle dans 6 cm³ d'heptane. On laisse la température remonter jusqu'à 0°C et agite pendant ½ heure puis verse le mélange sur un mélange de 60 cm³ d'eau glacée et 60 cm³ d'éther isopropylique. On décante, sèche la phase organique, traite au charbon actif, évapore le solvant, reprend le résidu à l'éther isopropylique, filtre, évapore le solvant et obtient 1,48 g de produit attendu brut. On chromatographie ce produit sur silice en éluant au mélange hexane-éther isopropylique (95–5) et obtient 0,53 g de produit attendu.

Stade B: acide (1R,cis,ΔZ) 2,2-diméthyl 3-(4',4'-dibromo buta 1',3'-diényl) cyclopropane carboxylique.

On mélange 0,48 g de produit obtenu au stade A, 5 cm³ de toluène et 0,030 g d'acide paratoluène sulfonique, porte au reflux pendant 15 minutes puis concentre à sec. On obtient 0,5 g de produit attendu utilisé tel quel dans l'exemple 18.

Exemple 18

(1R,cis,ΔZ) 2,2-diméthyl 3-(4',4'-dibromo buta 1',3'-diényl) cyclopropane carboxylate de (S) α-cyano 3-phénoxy benzyle.

On mélange 0,42 g de produit obtenu à l'exemple 17, 2 cm³ de chlorure de méthylène et 0,04 g de diméthylaminopyridine. On agite pendant 10 minutes, refroidit à 0°C et ajoute goutte à goutte un mélange de 0,27 g de dicyclohexylcarbodiimide dans 2 cm³ de chlorure de méthylène. On agite la suspension obtenue pendant 15 minutes à 0°C puis ajoute 0,34 g d'alcool (S) α-cyano 3-phénoxybenzylique dans 3 cm³ de chlorure de méthylène. On laisse la température remonter à 20°C, agite pendant 1 heure, filtre l'insoluble, concentre le filtrat à sec, chromatographie le résidu sur silice en éluant au mélange hexane-éther isopropylique (8–2) et obtient 0,57 g de produit attendu.

Spectre IR ($CHCl_3$)

Absorption à 1743 $cm^{-1}$ (c = O ester), à 1590–1490 $cm^{-1}$ (c = c aromatique), à 1390–1380 $cm^{-1}$ (gem diméthyl).

Spectre RMN ($CDCl_3$)

6,83 à 7,66 ppm pics attribués aux hydrogènes éthyléniques en 3' et aromatiques.

5,66 à 6,5 ppm pics attribués aux hydrogènes en 1', 2'.

6,41 ppm pic attribué à l'hydrogène porté par le même carbone que le CN.

1,23 ppm pic attribué aux $CH_3$ géminés.

Etude de l'activité des composés de l'invention

L'activité insecticide des exemples 7, 10 et 12 dénommés ci-après produits A, B, C a été recherchée sur les larves d'Epilachna Varivestris.

Les résultats expérimentaux, exprimés en DL 50 sont résumés dans le tableau suivant:

| | DL 50 ng par insecte |
|---|---|
| Composé A | 1,8 |
| Composé B | 1,9 |
| Composé C | 2,9 |

Conclusion: Les composés A, B et C sont doués d'une forte activité insecticide vis-à-vis d'Epilachna Varivestris.

Exemple 19

Préparation d'un concentré émulsifiable.

On effectue un mélange homogène de:

| | |
|---|---|
| Produit de l'exemple 7 | 0,25 g |
| Butoxyde de pipéronyle | 1 g |
| Tween 80 | 0,25 g |
| Topanol A | 0,1 g |
| Eau | 98,4 g |

Exemple 20

Préparation d'un concentré émulsifiable.

On mélange intimement:

| | |
|---|---|
| Produit de l'exemple 7 | 0,015 g |
| Butoxyde de pipéronyle | 0,5 g |
| Topanol A | 0,1 g |
| Xylène | 99,385 g |

Exemple 21

Préparation d'un concentré émulsifiable.

On effectue un mélange homogène de:

| | |
|---|---|
| Produit de l'exemple 7 | 1,5 g |
| Tween 80 | 20 g |
| Topanol A | 0,1 g |
| Xylène | 78,4 g |

Exemple 22

Préparation d'une composition fumigène.

On mélange d'une façon homogène:

| | |
|---|---|
| Produit de l'exemple 7 | 0,25 g |
| Poudre de tabu | 25 g |
| Poudre de feuilles de cèdre | 40 g |
| Poudre de bois de pin | 33,75 g |
| Vert brillant | 0,5 g |
| p-nitrophénol | 0,5 g |

**Description pour les Etats contractants: BE, CH, DE, FR, GB, LI, NL**

On connaissait aussi dans le brevet français 2 364 884 des composés de formule générale:

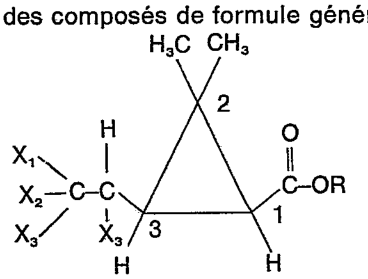

dont la chaîne latérale en position 3 est une chaîne saturée tétrahalogénée ne comportant donc aucune double liaison sur la chaîne portée par le carbone en 3 de la double liaison.

L'invention a pour objet, sous toutes leurs formes stéréoisomères possibles ou sous forme de mélanges de stéréoisomères, les composés de formule (I):

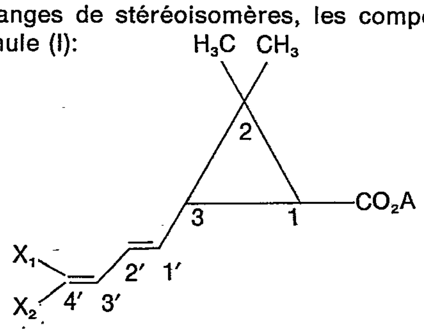

(I)

dans laquelle $X_1$ et $X_2$, identiques ou différents, représentent un atome d'halogène, et A représente,

– soit un radical benzyle éventuellement substitué par un ou plusieurs radicaux choisis dans le groupe constitué par les radicaux alcoyles comportant de 1 à 4 atomes de carbone, les radicaux alcényles comportant de 1 à 6 atomes de carbone, les radicaux alcadiényles comportant de 4 à 8 atomes de carbone, le radical méthylène dioxyle, le radical benzyle et les atomes d'halogène,

– soit un groupement

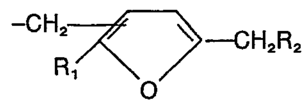

dans lequel le substituant $R_1$ représente un atome d'hydrogène ou un radical méthyle et le substituant $R_2$ un aryle monocyclique ou un groupement $-CH_2-C\equiv CH$ et notamment un groupement 5-benzyl 3-furyl méthyle,

– soit un groupement

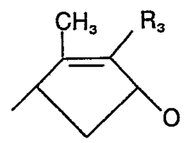

dans lequel $R_3$ représente un radical organique aliphatique comportant de 2 à 6 atomes de carbone et une ou plusieurs insaturations carbone-carbone et notamment le radical vinyle, propen-1-yle, buta-1,3-diényle ou buten-1-yle,

– soit un groupement

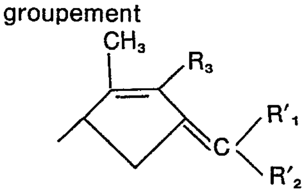

dans lequel $R_3$ conserve la même signification que précédemment, $R'_1$ et $R'_2$ identiques ou différents représentent un atome d'hydrogène, un atome d'halogène, un radical alcoyle renfermant de 1 à 6 atomes de carbone, un radical aryle comportant de 6 à 10 atomes de carbone, un groupement alcoyl-oxycarbonyle comportant de 2 à 5 atomes de carbone, ou un groupement cyano,

– soit un groupement

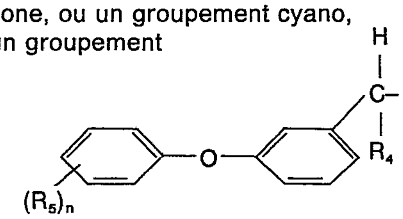

dans lequel $R_4$ représente un atome d'hydrogène, un groupement $-C\equiv N$, un radical méthyle, un radical $-CONH_2$, un radical $-CSNH_2$, ou un groupement $-C\equiv CH$, $R_5$ représente un atome de chlore ou un radical méthyle et n représente un nombre égal à 0, 1 ou 2, à la condition que si n = O, $R_4$ ne représente ni un atome d'hydrogène, ni un groupement $C\equiv N$, ni un groupement $C\equiv CH$,

– soit un groupement

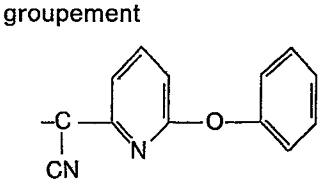

– soit un groupement

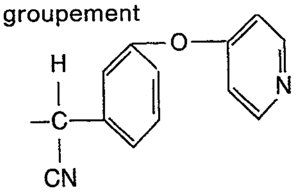

– soit un groupement

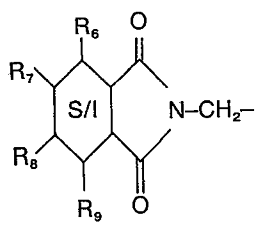

dans lequel les substituants $R_6$, $R_7$, $R_8$ et $R_9$ représentent un atome d'hydrogène, un atome de chlore ou un radical méthyle et dans lequel S/I symbolise un cycle aromatique ou un cycle analogue dihydro ou tétrahydro.

L'invention a notamment pour objet sous toutes leurs formes stéréoisomères possibles ou sous forme de mélanges de stéréoisomères, les composés de formule ($I_a$):

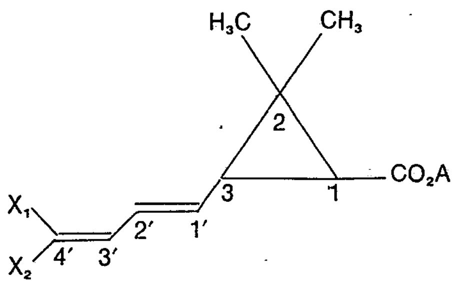

($I_a$)

dans laquelle la copule acide cyclopropanique est de structure 1R cis ou 1R trans et dans laquelle $X_1$ et $X_2$, identiques ou différents, représentent un atome d'halogène, et A représente,
– soit un atome d'hydrogène,
– soit un radical alcoyle renfermant de 1 à 18 atomes de carbone,
– soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylène dioxyle, le radical benzyle et les atomes d'halogène,
– soit un groupement

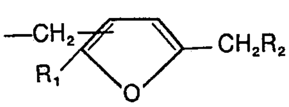

dans lequel le substituant $R_1$ représente un atome d'hydrogène ou un radical méthyle et le substituant $R_2$ un aryle monocyclique ou un groupement $-CH_2-C{\equiv}CH$ et notamment un groupement 5-benzyl 3-furyl méthyle,
– soit un groupement

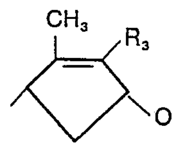

dans lequel $R_3$ représente un radical organique aliphatique comportant de 2 à 6 atomes de carbone et une ou plusieurs insaturations carbone-carbone et notamment le radical vinyle, propen-1-yle, buta-1,3-diényle ou buten-1-yle,
– soit un groupement

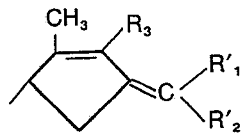

dans lequel $R_3$ conserve la même signification que précédemment, $R'_1$ et $R'_2$ identiques ou différents représentent un atome d'hydrogène, un atome d'halogène, un radical alcoyle renfermant de 1 à 6 atomes de carbone, un radical aryle comportant de 6 à 10 atomes de carbone, un groupement alcoyl-oxycarbonyle comportant de 2 à 5 atomes de carbone, ou un groupement cyano,
– soit un groupement

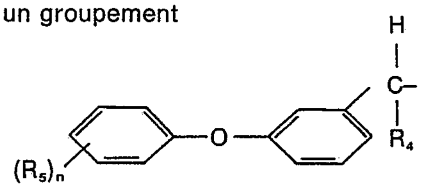

dans lequel $R_4$ représente un atome d'hydrogène, un groupement $-C{\equiv}N$, un radical méthyle, un radical $-CONH_2$, un radical $-CSNH_2$, ou un groupement $-C{\equiv}CH$, $R_5$ représente un atome de chlore ou un radical méthyle et n représente un nombre égal à 0, 1 ou 2, et notamment le groupement 3-phénoxybenzylique, α-cyano 3-phénoxybenzylique ou α-éthynyl 3-phénoxybenzylique,
– soit un groupement

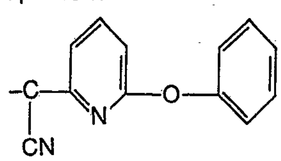

– soit un groupement

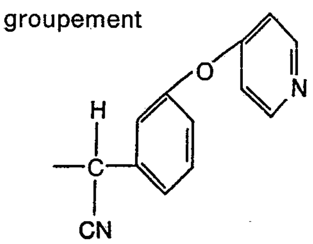

– soit un groupement

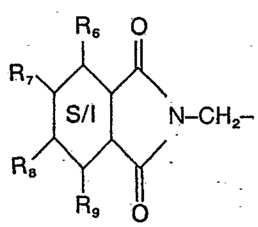

dans lequel les substituants $R_6$, $R_7$, $R_8$ et $R_9$ représentent un atome d'hydrogène, un atome de chlore ou un radical méthyle et dans lequel S/I symbolise un cycle aromatique ou un cycle analogue dihydro ou tétrahydro.

Les composés de formule (I) ou ($I_a$) peuvent exister sous de nombreuses formes isomères, ils présentent tous deux carbones asymétriques en position 1 et 3 du cycle cyclopropanique, ainsi qu'une possibilité d'isomérie E/Z au niveau de la double liaison 1′, 2′ et, lorsque $X_1$ et $X_2$ sont différents l'un de l'autre, ils présentent également une possibilité d'isomérie E/Z au niveau de la double liaison 3′, 4′.

$X_1$ et $X_2$ représentent de préférence un atome de chlore, de brome ou d'iode.

Le radical a peut posséder également 1 ou plusieurs centres d'asymétrie.

Lorsque A représente un radical alcoyle, il s'agit de préférence du radical méthyle, éthyle, n-propyle, iso-propyle ou n-butyle.

Lorsque A représente un radical benzyle substitué par un ou plusieurs radicaux alcoyle, il s'agit de préférence des radicaux méthyle ou éthyle.

Lorsque A représente un radical benzyle substitué par un ou plusieurs radicaux alcényle, il s'agit de préférence de radicaux vinyle, allyle, 2-méthylallyle, ou isobutényle.

Lorsque A représente un radical benzyle substitué par un ou plusieurs radicaux alcényloxy, il s'agit de préférence de radicaux vinyloxy, allyloxy, 2-méthylallyloxy ou isobuténylоxy.

Lorsque A représente un radical benzyle substitué par un ou plusieurs radicaux alcadiényles, il s'agit de préférence de radicaux buta-1′,3′-diényle ou pentadiényle.

Lorsque A représente un radical benzyle substitué par un ou plusieurs atomes d'halogènes, il s'agit de préférence d'atomes de chlore ou de brome.

L'invention a notamment pour objet les composés de formule (I) pour lesquels la copule acide est de structure 1R cis ou 1R trans.

L'invention a plus particulièrement pour objet les composés de formules (I) et $I_a$) pour lesquels $X_1$ et $X_2$ sont identiques et représentent tous les deux un atome de brome ou de chlore, et ceux pour lesquels A représente un atome d'hydrogène, ainsi que leurs sels.

Par «sels» ont entend de préférence les sels formés avec les métaux alcalins, alcalino terreux, le cuivre, le zinc ou encore les sels d'amines.

L'invention a également plus particulièrement pour objet les composés de formule ($I_a$) pour lesquels A représente un radical alcoyle renfermant de 1 à 4 atomes de carbone et notamment le radical méthyle, ceux pour lesquels A représente un groupement α-cyano 3-phénoxybenzyle, ainsi que ceux pour lesquels A représente un groupement 3-allyl 2-méthyl 4-oxo cyclopent-2-en-1-yle.

L'invention a naturellement tout particulièrement pour objet les produits dont la préparation est donnée dans la partie expérimentale.

Les composés de formules (I) et ($I_a$) présentent d'intéressantes propriétés qui permettent leur utilisation dans la lutte contre les parasites, il peut s'agir par exemple de la lutte contre les parasites des végétaux. C'est ainsi que l'on peut utiliser les produits de l'invention pour lutter contre les insectes, les nématodes et les acariens parasites des végétaux et des animaux.

Les produits de formules (I) et ($I_a$) peuvent dont être utilisés notamment pour lutter contre les insectes dans le domaine agricole, pour lutter par exemple contre les pucerons, les larves de lépidoptères et les coléoptères. Ils sont utilisés à des doses comprises entre 10 et 300 g de matière active à l'hectare.

Les produits de formules (I) et ($I_a$) peuvent également être utilisés pour lutter contre les insectes dans le domaine domestique, pour lutter notamment contre les mouches, les moustiques et les blattes.

Les produits de formules (I) et ($I_a$) peuvent aussi être utilisés pour lutter contre les acariens et les nématodes parasites des végétaux.

Les composés de formules (I) et ($I_a$) peuvent encore être utilisés pour lutter contre les acariens parasites des animaux, pour lutter par exemple contre les tiques et notamment les tiques de l'espèce Boophilus, ceux de l'espèce Hyalomma, ceux de l'espèce Amblyomma et ceux de l'espèce Rhipicephalus, ou pour lutter contre toutes sortes de gales et notamment la gale sarcoptique, la gale psoroptique et la gale chorioptique.

L'invention a donc également pour objet l'application des produits de formules (I) et ($I_a$) à la lutte contre les parasites des végétaux ainsi que les compositions destinées à la lutte contre les parasites des animaux à sang chaud et des végétaux, caractérisées en ce qu'elles renferment comme principe actif au moins l'un des produits définis ci-dessus.

L'invention a notamment pour objet les compositions insecticides renfermant comme principe actif au moins l'un des produits définis ci-dessus.

Ces compositions sont préparées selon les procédés usuels de l'industrie agrochimique, de l'industrie vétérinaire ou de l'industrie des produits destinés à la nutrition animale.

Dans ces compositions la ou les matières actives peuvent être additionnées éventuellement d'un ou plusieurs autres agents pesticides. Ces compositions peuvent se présenter sous forme de poudres, granulés, suspensions, émulsions, solutions, solutions pour aérosols, bandes combustibles, appâts ou autres préparations employés classiquement pour l'utilisation de ce genre de composés.

Outre le principe actif, ces compositions contiennent, en général, un véhicule et/ou un agent tensio-actif, non ionique, assurant, en outre, une dispersion uniforme des substances constitutives du mélange. Le véhicule utilisé peut être un liquide, tel que l'eau, l'alcool, les hydrocarbures ou autres solvants organiques, une huile minérale, animale ou végétale, une poudre tel que le talc, les argiles, les silicates, le kieselguhr ou un solide combustible, tel que la poudre de tabu (ou marc de pyréthre).

Les compositions insecticides selon l'invention contiennent de préférence de 0,005 à 10% en poids de matière active.

Comme les compositions insecticides selon l'invention, les compositions acaricides et nématicides peuvent être additionnées éventuellement d'un ou plusieurs autres agents pesticides. Les compositions acaricides et nématicides peuvent se présenter notamment sous forme de poudre, granulés, suspensions, émulsions, solutions.

Pour l'usage acaricide, on utilise de préférence des poudres mouillables pour pulvérisation foliaire, contenant de 1 à 80% de principe actif ou des liquides pour pulvérisation foliaire contenant de 1 à 500 g/litre de principe actif. On peut également employer des poudres pour poudrage foliaire, contenant de 0,05 à 3% de matière active.

Pour l'usage nématicide, on utilise de préférence des liquides pour traitement des sols contenant de 300 à 500 g/l de principe actif.

Les composés acaricides et nématicides selon l'invention sont utilisés, de préférence, à des doses comprises entre 1 et 100 g de matière active à l'hectare.

Pour exalter l'activité biologique des produits de l'invention, on peut les additionner à des synergistes classiques utilisés en pareil cas tel que le 1-(2,5,8-trioxadodécyl) 2-propyl 4,5-méthylènedioxy benzène (ou butoxyde de pipéronyle) ou la N-(2-éthyl heptyl) bicyclo/2,2-1/-5-heptène-2,3-di carboximide, ou le pipéronyl-bis-2-(2'-n-butoxy éthoxy) ethylacétal (ou tropital).

Les composés de formules (I) et ($I_a$) présentent une excellente tolérance générale et l'invention a donc également pour objet les produits de formules (I) et ($I_a$) à titre de médicaments, pour lutter notamment contre les affections créées par les tiques et les gales.

Les médicaments de l'invention peuvent être utilisés tant en médecine humaine qu'en médecine vétérinaire.

Les médicaments de l'invention sont notamment utilisés en médecine humaine pour lutter contre les poux à titre préventif ou curatif et pour lutter contre la gale.

Les médicaments de l'invention peuvent être administrés par voie externe, par vaporisation, par bain ou badigeonnage.

Les médicaments de l'invention à usage vétérinaire peuvent être également administrés par badigeonnage de l'épine dorsale selon la méthode dite méthode «pour-on». Ils peuvent être également administrés par voie digestive ou parentérale.

L'invention a donc pour objet les compositions pharmaceutiques renfermant comme principe actif au moins l'un des médicaments définis précédemment.

Lorsqu'il s'agit de lutter contre les acariens parasites des animaux, on peut incorporer les produits de l'invention dans des compositions alimentaires en association avec un mélange nutritif adapté à l'alimentation animale. Le mélange nutritiel peut varier selon l'espèce animale, il peut renfermer des céréales, des sucres et des grains, des tourteaux de soja, d'arachide et de tournesol, des farines d'origine animale, par exemple des farines de poissons, des acides aminés de synthèse, des sels minéraux, des vitamines et des antioxydants.

L'invention a donc ainsi pour objet les compositions alimentaires définies ci-dessus.

On peut indiquer également que les produits de l'invention peuvent être utilisés comme biocides ou comme régulateurs de croissance.

Les produits de formules (I) et ($I_a$) pour lesquels A représente un atome d'hydrogène et leurs sels ainsi que les produits pour lesquels A représente un radical alcoyle renfermant de 1 à 18 atomes de carbone, sont non seulement des produits biologiquement actifs, ils sont également des intermédiaires utiles pour la synthèse de produits biologiquement actifs.

Les produits les plus intéressants d'une point de vue biologique sont les produits pour lesquels A ne représente ni un atome d'hydrogène, ni un radical alcoyle.

Les produits de formules (I) et ($I_a$) présentent d'intéressantes propriétés organoleptiques qui permettent de les utiliser notamment comme agent parfumant.

L'invention a pour objet les compositions parfumantes renfermant comme principe actif au moins un produit de formule (I) sous toutes ses formes stéréoisomères possibles ainsi que sous forme de mélanges de stéréoisomères:

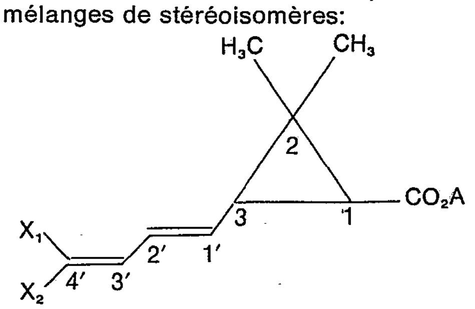

(I)

dans laquelle $X_1$ et $X_2$, identiques ou diffèrents, représentent un atome d'halogène, et A représente,

– soit un atome d'hydrogène,

– soit un radical alcoyle renfermant de 1 à 18 atomes de carbone,

– soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylène dioxyle, le radical benzyle et les atomes d'halogène,

– soit un groupement

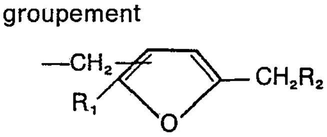

dans lequel le substituant $R_1$ représente un atome d'hydrogène ou un radical méthyle et le substituant $R_2$ un aryle monocyclique ou un groupement

$-CH_2-C{\equiv}CH$ et notamment un groupement 5-benzyl 3-furyl méthyle,

– soit un groupement

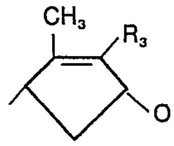

dans lequel $R_3$ représente un radical organique aliphatique comportant de 2 à 6 atomes de carbone et une ou plusieurs insaturations carbone-carbone et notamment le radical vinyle, propen-1-yle, buta-1,3-diényle ou buten-1-yle,

– soit un groupement

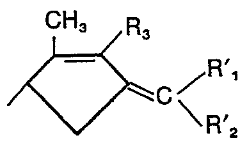

dans lequel $R_3$ conserve la même signification que précédemment, $R'_1$ et $R'_2$ identiques ou différents représentent un atome d'hydrogène, un atome d'halogène, un radical alcoyle renfermant de 1 à 6 atomes de carbone, un radical aryle comportant de 6 à 10 atomes de carbone, un groupement alcoyl-oxycarbonyle comportant de 2 à 5 atomes de carbone, ou un groupement cyano,

– soit un groupement

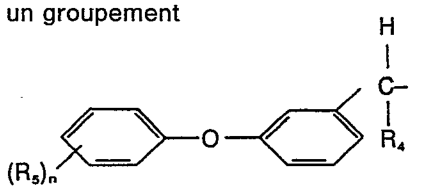

dans lequel $R_4$ représente un atome d'hydrogène, un groupement $-C{\equiv}N$, un radical méthyle, un radical $-CONH_2$, un radical $-CSNH_2$, ou un groupement $-C{\equiv}CH$, $R_5$ représente un atome de chlore ou un radical méthyle et n représente un nombre égal à 0, 1 ou 2, et notamment le groupement 3-phénoxybenzylique, α-cyano 3-phénoxybenzylique ou α-éthyl 3-phénoxybenzylique,

– soit un groupement

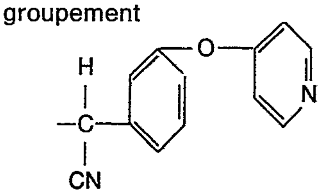

– soit un groupement

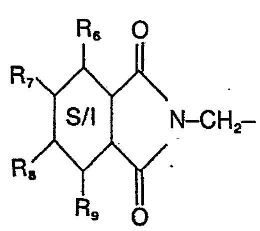

dans lequel les substituants $R_6$, $R_7$, $R_8$ et $R_9$ représentent un atome d'hydrogène, un atome de chlore ou un radical méthyle et dans lequel S/I symbolise un cycle aromatique ou un cycle analogue dihydro ou tétrahydro.

Les produits de formule (I) pour lesquels A représente un radical alcoyle renfermant de 1 à 4 atomes de carbone et notamment le radical méthyle sont ceux qui semblent le plus intéressants à utiliser en tant qu'agent parfumant. Ces produits sont doués d'une odeur florale remarquable.

En raison de leurs intéressantes propriétés olfactives, les produits de formule (I) peuvent être utilisés comme agents odorants en parfumerie pour préparer des compositions odorantes qui peuvent servir elles-mêmes de base à des parfums.

L'invention a donc pour objet les compositions parfumantes renfermant comme principe actif au moins un produit de formule (I).

Les produits de formule (I) peuvent également être utilisés pour préparer des articles d'hygiène comme par exemple des savons, des talcs, des shampooings.

Les produits de formule (I) peuvent enfin être utilisés pour la préparation de produits détergents, comme par exemple les lessives, ou pour la préparation de produits d'entretien comme les cires.

L'invention a également pour objet un procédé de préparation des composés de formule (I) ou $(I_A)$, caractérisé en ce que l'on soumet le composé de formule (II):

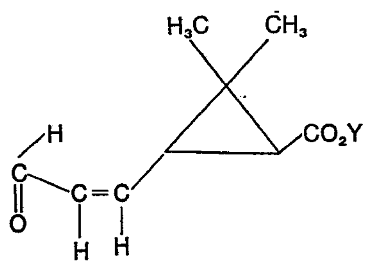
(II)

dans laquelle Y représente un atome d'hydrogène ou un radical alcoyle renfermant de 1 à 18 atomes de carbone, sous l'une quelconque de ses formes isomères ou sous forme d'un mélange d'isomères, à l'action d'un composé de formule:

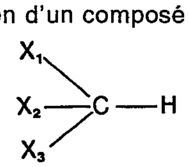
(III)

dans laquelle soit les substituants $X_1$, $X_2$ et $X_3$ sont identiques et représentent un atome de chlore ou de brome, soit au moins deux des substituants $X_1$, $X_2$ et $X_3$ sont différents, $X_1$, $X_2$, $X_3$ représentant alors un atome de fluor, de chlore ou de brome, $X_1$, $X_2$ et $X_3$ étant de poids atomiques égaux ou croissant de $X_1$ à $X_3$, pour obtenir le composé de formule

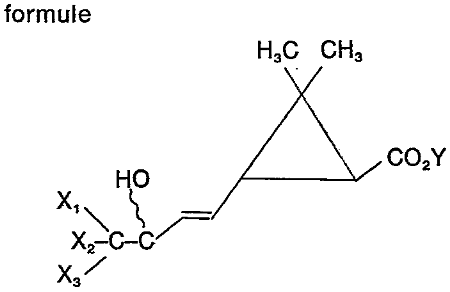
(IV)

dont on bloque l'hydroxyle par action d'un acide ou d'un dérivé fonctionnel d'acide $R–CO_2H$, puis soumet le produit obtenu de formule:

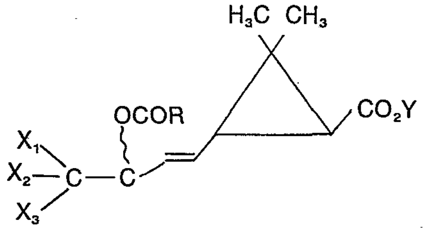

(V)

à l'action d'un agent de réduction pour obtenir après élimination du substituant $X_3$ qui est le substituant ou l'un des substituants de poids atomique le plus élevé le composé de formule

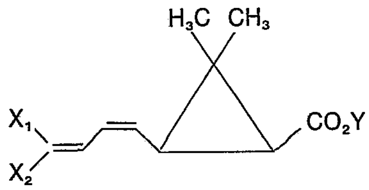

($I_A$)

dans laquelle $X_1$ et $X_2$ soit sont identiques et représentent un atome de chlore ou de brome, soit sont différents et représentent un atome de fluor, de chlore ou de brome, puis soumet, si désiré, un composé de formule $I_A$, dans lequel Y représente un radical alcoyle renfermant de 1 à 18 atomes de carbone, à l'action d'un agent de saponification, pour obtenir un composé de formule (I) dans lequel A représente un atome d'hydrogène ou un sel de ce composé, ou soumet, si désiré, soit un composé de formule ($I_A$) dans lequel Y est un radical alcoyle renfermant de 1 à 18 atomes de carbone, soit un composé de formule ($I_A$) dans lequel Y représente un atome d'hydrogène, soit un dérivé fonctionnel de ce dernier, à l'action d'un alcool de formule $A_1OH$ ou d'un dérivé fonctionnel de cet alcool, formule dans laquelle $A_1$ a la même signification que A, à l'exception de l'hydrogène pour obtenir le composé de formule (I) correspondant.

Dans un mode de réalisation préféré de l'invention,

- Y représente un radical alcoyle,
- on bloque l'hydroxyle du composé de formule (IV) par action d'un anhydride, par exemple l'anhydride acétique, ou d'un halogénure d'acide comme par exemple un chlorure ou un bromure et notamment le chlorure ou le bromure d'acétyle,
- l'agent de réduction auquel on soumet le composé de formule (V) est le zinc en présence d'acide acétique ou le couple zinc-cuivre en présence d'un alcool,
- l'agent de saponification est la soude ou la potasse,
- le dérivé fonctionnel du composé $I_A$ dans lequel Y représente un atome d'hydrogène est un chlorure d'acide, mais il peut être également un sel de métal alcalin, par exemple un sel de sodium ou de potassium, la réaction étant alors effectuée sur un dérivé halogéné d'un alcool, par exemple un bromure ou un chlorure.

L'invention a également pour objet une variante du procédé décrit ci-dessus, caractérisée en ce que l'on soumet selon la réaction de Wittig le composé de formule (II) telle que définie précédemment, sous l'une quelconque de ses formes isomères ou sous forme d'un mélange d'isomères, à l'action d'un composé de formule

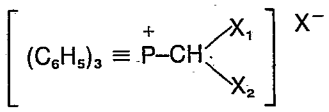

dans laquelle $X_1$, $X_2$ sont définis comme précédemment et $X^-$ représente un anion halogénure pour obtenir le composé de formule ($I_A$) puis, si désiré, poursuit la synthèse comme décrit précédemment.

L'invention a plus particulièrement pour objet un procédé de préparation des composés de formule (I), caractérisé en ce que le composé de formule (II) est de structure 1R,cis ou 1R,trans.

La réaction de Wittig ci-dessus est effectuée en présence d'une base forte qui peut être un hydrure alcalin, un amidure alcalin, un alcoyllithien ou un alcoolate alcalin, de préférence un terbutylate de sodium, de potassium ou de lithium.

Les composés de formule (II) utilisés comme produits de départ sont des produits connus d'une façon générale, de toute façon ils peuvent être préparés selon le procédé décrit par Elliott et Coll dans J. Chem. Soc. Perkin 1, p. 2470 et suivantes.

L'acide (1R cis) 2,2-diméthyl 3-(3'-oxo 1'-propényl) cyclopropane carboxylique est un produit nouveau et l'invention a pour objet ce produit à titre de produit industriel nouveau et plus particulièrement à titre de produit intermédiaire nécessaire à la mise en œuvre du procédé de l'invention.

Les composés de formule (IV) et (V) obtenus lors de la mise en œuvre du procédé sont des produits chimiques nouveaux et l'invention a également pour objet les composés de formule (IV) et (V), à titre de produits industriels nouveaux, et plus particulièrement à titre de produits intermédiaires nécessaires à la mise en œuvre du procédé de l'invention.

Les exemples suivants illustrent l'invention sans toutefois la limiter.

Préparation: Acide (1R,cis) 2,2-diméthyl 3-(3'-oxo 1'-propényl) cyclopropane carboxylique.

On mélange 76,5 g de ter-butylate de potassium, 374 cm³ d'heptane, ajoute 63,8 cm³ de terbutanol et 66 cm³ d'heptane sous atmosphère inerte. A 20 °C on introduit, peu à peu, 159,5 g de bromure de triphényl/(1,3-dioxolan-2-yl) méthyl/ phosphonium préparé ci-après. On refroidit à −10 °C, ajoute 44 g de (1R, 5S) 6,6-diméthyl 4(R)-hydroxy 3-oxo bicyclo/3-1-0/hexane 2-one dans 180 cm³ de tétrahydrofurane, maintient à −10 °C pendant 1 heure puis 17 heures à température ambiante. On concentre à sec sous pression réduite, reprend le résidu par 1400 cm³ de soude 0,25N et 320 cm³ de chlorure de méthylène et on décante. A la phase aqueuse, traitée au charbon actif, on ajoute 176 cm³ de tétrahydrofurane et

70 cm³ d'acide chlorhydrique concentré, puis agite 3 heures 30 minutes à 20 °C. On extrait au chlorure de méthylène, lave à l'eau, sèche, concentre à sec, reprend le résidu par de l'éther isopropylique, agite pendant une demi-heure à 0 °C, essore et obtient 23,7 g de produit attendu F = 129 °C.

Le bromure de triphényl (1,3-dioxolan-2-yl méthyl) phosphonium utilisé au départ de la préparation a été préparé comme suit:

On chauffe à 80 °C pendant 36 heures, 180 g de 2(bromoéthyl) 1,3-dioxolan et 260 g de triphénylphosphine. On refroidit, dissout le produit obtenu dans le chlorure de méthylène et verse lentement sur 3000 cm³ d'éther éthylique. Après agitation, on essore, empâte avec de l'éther éthylique et obtient 359 g de produit F = 206 °C.

Exemple 1

(Acide (1R,cis) 2,2-diméthyl 3-(4',4'-dichloro buta – 1',3'-diènyl) cyclopropane carboxylique.

Stade A: Acide (1R,cis) 2,2-diméthyl 3-(4',4',4'-trichloro 3'-hydroxy buta 1'-ènyl) cyclopropane carboxylique.

On mélange 10 g d'acide (1R,cis) 2,2-diméthyl 3-(3'-oxo 1'-propényl) cyclopropane carboxylique obtenu à la préparation 1 et 24 cm³ de chloroforme, agite sous atmosphère inerte, refroidit à −10 °C et introduit goutte à goutte une solution préparée avec 9,2 g de méthylate de potassium, 39 cm³ de butanol et 32 cm³ de tétrahydrofuranne. On agite pendant 2 heures le milieu réactionnel à une température voisine de −5 °C–0 °C, puis, le verse dans 300 cm³ d'un mélange eau-glace et 13 cm³ d'acide chlorhydrique concentré. On décante, extrait au chloroforme, lave à l'eau, sèche, concentre à sec sous pression réduite. On reprend le résidu à l'éther de pétrole (eb 60–80 °C) essore et obtient 9,3 g de produit attendu F = 138 °C.

Stade B: Acide (1R,cis) 2,2-diméthyl 3-(4',4',4'-trichloro 3'-acétoxy but-1-ényl) cyclopropane carboxylique.

A température ambiante, on agite pendant 4 heures 8 g du produit obtenu au stade précédent dans 22,5 cm³ de pyridine et 11,5 cm³ d'anhydride acétique, ajoute de la glace, agite, puis verse la solution dans un mélange de 30 cm³ d'acide chlorhydrique concentré et 150 cm³ d'eau glacée. On extrait au chloroforme, lave à l'eau, sèche, traite au charbon actif, concentre à sec sous pression réduite et obtient 9,56 g de produit huileux utilisé tel quel au stade suivant.

Stade C: Acide (1R,cis) 2,2-diméthyl 3-(4',4'-dichloro buta 1',3'-diényl) cyclopropane carboxylique.

On mélange 9,5 g du produit obtenu ci-dessus et 95 cm³ d'acide acétique à 10% d'eau et ajoute, sous agitation, 5,65 g de zinc en poudre. On agite pendant 4 heures à 35 °C environ, concentre à sec sous pression réduite, reprend le résidu par l'eau et extrait au chloroforme. Les phases organiques sont lavées à l'eau, traitées au charbon actif, séchées, concentrées à sec et lavées au toluène. On obtient 6,5 g de produit huileux que l'on chromatographie sur silice, élue par un mélange éther de pétrole (eb 40–70 °C) – éther éthylique (75–25) et récolte 2,75 g de produit attendu.

Spectre RMN ($CDCl_3$)

1,225–1,3 ppm pics attribués aux hydrogènes des méthyles.

1,66 à 2,08 ppm pics attribués aux hydrogènes du cyclopropyle.

6,17 à 6,58 ppm pics attribués aux hydrogènes éthyléniques.

Exemple 2

(1R,trans) 2,2-diméthyl 3-(4',4'-dichloro buta-1',3'-diènyl) cyclopropane carboxylate de méthyle.

Stade A: (1R,trans) 2,2-diméthyl 3-(4',4',4'-trichloro 3'-hydroxy but-1-ényl) cyclopropane carboxylate de méthyle.

On agite 11,6 g de (1R,trans) 2,2-diméthyl 3-(3'-oxo 1'-propényl)cyclopropane carboxylate de méthyle préparé selon le procédé décrit par Elliot et Coll (J. Chem. Soc. 1975 p. 2470 et suivantes), dans 15 cm³ de chloroforme, refroidit à −5–−10 °C et introduit 5,25 g de méthylate de potassium dans 20 cm³ de ter-butanol et 15 cm³ de tétrahydrofuranne. Après 2 heures à −5°C sous agitation, la suspension est versée dans 100 cm³ d'un mélange glace-eau et 7,5 cm³ d'acide chlorhydrique concentré. On agite, décante, extrait au chloroforme, sèche, concentre à sec à 50 °C sous pression réduite, recristallise dans l'éther de pétrole (eb 60–80°C) et obtient 12,2 g du produit attendu F = ~20–25 °C.

Stade B: (1R,trans) 2,2-diméthyl 3-(4',4',4'-trichloro 3'-acétoxy but-1'-ényl) cyclopropane carboxylate de méthyle.

A température ambiante, on agite pendant 4 heures 33,5 cm³ de pyridine, 12,2 g de produit obtenu ci-dessus et 16,8 cm³ d'anhydride acétique. On ajoute 40 g de glace, agite pendant 30 minutes et verse le mélange réactionnel sur 60 g d'un mélange glace-eau et 39 cm³ d'acide chlorhydrique concentré. Après 10 minutes on extrait au chlorure de méthylène, lave sèche, concentre à sec à 50 °C sous pression réduite et obtient 11,8 g de produit attendu.

Stade C: (1R,trans) 2,2-diméthyl 3-(4', 4'-dichloro buta-1',3'-diényl)cyclopropane carboxylate de méthyle.

On mélange 11,8 g du produit obtenu au stade B et 236 cm³ d'acide acétique à 10% d'eau, ajoute 6,73 g de poudre de zinc et chauffe à 55 °C pendant 4 heures. On laisse refroidir à 20 °C, filtre l'insoluble et concentre à sec le filtrat à 60 °C sous pression réduite. On reprend le résidu par 125 cm³ de chlorure de méthylène et 100 cm³ d'eau, agite, décante, lave la phase organique par une solution aqueuse saturée de bicarbonate de soude et réextrait par du chlorure de méthylène, lave à l'eau, sèche, concentre à sec sous pression réduite et obtient 9 g de produit. On le purifie par chromatographie sur silice, élue par un mélange cyclohexane-acétate d'éthyle (9–1) et obtient 4,3 g de produit huileux.

Spectre RMN ($CDCl_3$)

1,18–1,28 ppm pics attribués aux hydrogènes des méthyles géminés.

1,58–1,67 ppm pics attribués à l'hydrogène en 1 du cyclopropyle.
2 à 2,21 ppm pics attribués à l'hydrogène en 3 du cyclopropyle.
3,68 ppm pic attribué aux hydrogènes du méthyle de l'ester.
5,3 à 5,18 ppm pics attribués à l'hydrogène éthylénique en position 1 de la chaîne latérale.
6,1 à 6,6 ppm pics attribués aux autres hydrogènes éthyléniques en position 2 et 3 de la chaîne latérale.

Exemple 3

(Acide (1R,trans) 2,2-diméthyl 3-(4',4'-dichloro buta 1',3'-diènyl) cyclopropane carboxylique).

On chauffe au reflux pendant 4 heures 4,3 g de (1R,trans) 2,2-diméthyl 3-(4',4'-dichloro buta 1',3'-diényl) cyclopropane carboxylate de méthyle obtenu à l'exemple 2, 114 $cm^3$ de méthanol et 17,2 $cm^3$ de soude 2N. On concentre à sec à 50 °C sous pression réduite, reprend l'extrait sec avec 50 $cm^3$ d'eau et extrait à l'éther éthylique. La phase aqueuse est amenée à pH 1 avec de l'acide chlorhydrique 2N et extrait à l'éther éthylique. On sèche la phase organique, amène à sec à 30 °C sous pression réduite, puis une heure à 50 °C. On obtient 3,8 g de produit.

Spectre RMN ($CDCl_3$)
1,16–1,28 ppm pics attribués aux hydrogènes des méthyles géminés.
1,56–1,65 ppm pics attribués à l'hydrogène en 1 du cyclopropyle.
2,02–2,11–2,15–2,24 ppm pics atribués à l'hydrogène en 3 du cyclopropyle.
5,3 à 5,8 ppm pics attribués à l'hydrogène éthylénique en 1 de la chaîne latérale.
6,2 à 6,4 ppm pics attribués aux 2 autres hydrogènes éthyléniques.
11,3 ppm pic attribué à l'hydroxyle.

Exemple 4

(Acide (1R,cis) 2,2-diméthyl 3-(4',4'-dibromo buta-1',3'-diényl) cyclopropane carboxylique.

On chauffe à 75 °C sous atmosphère inerte, 24 $cm^3$ de ter-butanol, 16 $cm^3$ d'heptane et 6,6 g de ter-butylate de potassium. A 20 °C on ajoute 14,7 g de bromure de dibromo méthyl triphényl phosphorium. Après refroidissement à 0 °C, on introduit 4 g d'acide (1R,cis) 2,2-diméthyl 3-(3'-oxo 1'-propényl) cyclopropane carboxylique obtenu à la préparation 1 et agite encore pendant 1 heure à cette température. On chauffe le milieu réactionnel à 50 °C pendant 1 heure puis concentre à sec sous pression réduite. On reprend le résidu par 30 $cm^3$ de soude N et 20 $cm^3$ de chlorure de méthylène et ajoute 96 $cm^3$ d'eau distillée. On décante, lave au chlorure de méthylène. On agite les phases organiques avec 20 $cm^3$ de soude 0,25N. On réunit les phases aqueuses, les traite au charbon actif et ajoute 4 $cm^3$ d'acide chlorhydrique concentré. Le produit gommeux obtenu est dissous dans le chlorure de méthylène. On concentre à sec, à 40 °C, sous pression réduite et obtient 5,68 g de produit huileux qui, chromatographié sur silice en éluant par un mélange éther de pétrole (eb 40–70 °C)-éther éthylique (75–25) donne 3 g de produit.

Spectre RMN ($CDCl_3$)
1,23–1,3 ppm pics attribués aux hydrogènes des méthyles géminés.
1,75 à 2,33 ppm pics attribués aux hydrogènes du cyclopropyle.
6,2 à 6,4 et 6,9 à 7,1 ppm pics attribués aux hydrogènes éthyléniques.

Exemple 5

(1R,trans) 2,2-diméthyl 3-(4',4'-dibromo buta 1',3'-diényl) cyclopropane carboxylate de méthyle.

Sous agitation et sous atmosphère inerte, on introduit à 20 °C, 9,74 g de ter-butylate de potassium dans 100 $cm^3$ d'heptane, ainsi que 7,95 $cm^3$ de ter-butanol. On ajoute ensuite lentement et sous agitation 43,5 g de bromure de dibromo méthyl triphényl phosphonium et agite pendant 1 heure. On refroidit à −10 °C, ajoute 12,8 g de (1R,trans) 2,2-diméthyl 3-(3'-oxo 1'-propényl) cyclopropane carboxylate de méthyle dans 40 $cm^3$ de tétrahydrofuranne et agite pendant une heure à −10 °C et pendant 2 jours à température ambiante. On concentre à sec sous pression réduite, reprend le résidu par un mélange glace-eau, extrait au chlorure de méthylène, lave avec une solution saturée de phosphate monosodique, sèche, amène à sec sous pression réduite. On chromatographie le résidu sur silice, élue au chlorure de méthylène, concentre à sec et obtient 19,8 g de produit attendu $n_D^{23} = 1,561$.

Spectre RMN ($CDCl_3$)
1,15–1,25 ppm pics attribués aux hydrogènes des méthyles géminés.
1,58–1,675 ppm attribués à l'hydrogène en 1 du cyclopropyle.
3,7 ppm pic attribué aux hydrogènes du méthyle (ester).
1,99–2,08 ppm et 2,12–2,21 ppm pics attribués à l'hydrogène en 3 du cyclopropyle.
5,4–5,5 et 5,6–5,8 ppm pics attribués à l'hydrogène éthylénique de la chaîne latérale.
6,0–6,2 ppm 6,3–6,5 ppm pics attribués à l'hydrogène en 2 de la chaîne latérale.
6,8–7,0 ppm pics attribués à l'hydrogène en 3 de la chaîne latérale.

Exemple 6

Acide (1R,trans) 2,2-diméthyl 3-(4',4'-dibromo buta-1',3'-diényl) cyclopropane carboxylique.

On opère comme à l'exemple 3 en partant de 14 g de (1R,trans) 2,2-diméthyl 3-(4',4'-dibromo buta-1',3'-diényl) cyclopropane carboxylate de méthyle obtenu à l'exemple 5 et en extrayant à l'éther isopropylique. On obtient, après chromatographie sur silice, en éluant par un mélange chlorure de méthylène-acétate d'éthyle (9–1) 6,62 g de produit attendu.

Spectre RMN ($CDCl_3$)
1,3–1,32 ppm pics attribués aux hydrogènes des méthyles géminés.
1,6–1,68 ppm pics attribués à l'hydrogène en 1 du cyclopropyle.
2 à 2,25 ppm pics attribués à l'hydrogène en 3 du cyclopropyle.
5,4 à 5,8 ppm pics attribués à l'hydrogène éthylénique en 1 sur la chaîne latérale.

6,1–6,3 ppm – 6,3–6,5 ppm pics attribués à l'hydrogène éthylénique en 2 sur la chaîne latérale.
6,8–7,0 ppm pics attribués à l'hydrogène éthylénique en 3 sur la chaîne latérale.

Exemple 7

(1R,cis) 2,2-diméthyl 3-(4',4'-dichloro buta 1',3'-diényl) cyclopropane carboxylate de (S) α-cyano 3-phénoxybenzyle.

Stade A: chlorure d'acide (1R,cis) 2,2-diméthyl 3-(4',4'-dichloro buta 1',3'-diényl) cyclopropane carboxylique.

Pendant 4 heures on porte au reflux 2,75 g d'acide (1R,cis) 2,2-diméthyl 3-(4'4'-dichloro buta 1',3'-diényl) cyclopropane carboxylique obtenu à l'exemple 1, 28 $cm^3$ d'éther de pétrole (eb 40–70 °C) et 8,25 $cm^3$ de chlorure de thionyle. On concentre à sec à 35 °C sous pression réduite, et obtient 2,91 g de produit attendu.

Stade B: (1R,cis) 2,2-diméthyl 3-(4',4'-dichloro buta 1',3'-diényl cyclopropane carboxylate de (S) α-cyano 3-phénoxybenzyle.

Au produit obtenu précédemment on ajoute 30 $cm^3$ de benzène et 2,7 g d'alcool (S) α-cyano 3-phénoxybenzylique. A 15 °C on introduit, goutte à goutte, un mélange de 4,44 $cm^3$ de pyridine et 9 $cm^3$ de benzène et agite pendant 17 heures à température ambiante. On verse le mélange réactionnel dans 100 $cm^3$ d'acide chlorhydrique 2N, agite, décante. On lave à l'eau la phase organique, traite au charbon actif, sèche, concentre à sec à 35–40 °C et obtient 4,87 g de produit.

Après chromatographie sur silice, on recristallise le produit dans un mélange hexane-éther isopropylique (1–1) et obtient 1,5 g du produit attendu F = 65 °C.

$\alpha_D = +53° \pm 2,5$ (c = 0,5% benzène).

Spectre RMN ($CDCl_3$)
1,23 ppm pic attribué aux hydrogènes des méthyles géminés.
1,77 à 2,2 ppm pics attribués aux hydrogènes du cyclopropyle.
5,8 à 6,7 ppm pics attribués aux hydrogènes éthyliques.
6,5 ppm pic attribué à l'hydrogène porté par le même carbone que CN.
7 à 7,6 ppm pics attribués aux hydrogènes aromatiques.

Exemple 8

(1R,trans) 2,2-diméthyl 3-(4',4'-dichloro buta 1',3'-diényl) cyclopropane carboxylate de (RS) α-cyano 3-phénoxybenzyle.

Stade A: Chlorure d'acide (1R,trans) 2,2-diméthyl 3-(4',4'-dichloro buta 1',3'-diényl) cyclopropane carboxylique.

On opère comme au stade A de l'exemple 7 en partant de 2,32 g d'acide (1R,trans) 2,2-diméthyl 3-(4',4'-dichloro buta 1',3'-diényl) cyclopropane carboxylique obtenu à l'exemple 3 et obtient 2,5 g de produit attendu.

Stade B: (1R,trans) 2,2-diméthyl 3-(4',4'-dichloro buta 1',3'-diényl) cyclopropane carboxylate de (RS) α-cyano 3-phénoxybenzyle.

D'une manière analogue à celle décrite au stade B de l'exemple 7, on obtient 4,90 g de produit brut, en partant du produit obtenu ci-dessus et de 2,3 g d'alcool (RS) α-cyano 3-phénoxybenzylique. Après chromatographie sur silice (éluant éther de pétrole (eb 40–70 °C) éther éthylique (75/25)) on obtient 2,46 g de produit attendu.

$\alpha_D = -25°6 \pm 2,5$ (c = 0,6% benzène).

Spectre RMN ($CDCl_3$)
1,17–1,2–1,3 ppm pics attribués aux hydrogènes des méthyles géminés.
1,55 à 1,72 ppm pics attribués à l'hydrogène en position 1 du cyclopropyle.
2 à 2,33 ppm pics attribués à l'hydrogène en position 3 du cyclopropyle.
6,3 ppm pic attribué à l'hydrogène porté par le même carbone que CN et aux hydrogènes en position 2 et 3 de la chaîne latérale éthylénique.
5,3 à 5,8 ppm pics attribués à l'hydrogène en position 1 de la chaîne latérale éthylénique.
6,9 à 7,7 ppm pics attribués aux hydrogènes aromatiques.

Exemple 9

(1R,trans) 2,2-diméthyl 3-(4',4'-dichloro buta 1',3'-diényl) cyclopropane carboxylate de (S) α-cyano 3-phénoxybenzyle.

On opère comme au stade B de l'exemple 7 en partant de 1,4 g de chlorure d'acide (1R,trans) 2,2-diméthyl 3-(4',4'-dichloro buta 1',3'-diényl) cyclopropane carboxylique obtenu au stade A de l'exemple 8 et de 1,35 g d'alcool (S) α-cyano 3-phénoxybenzylique. On obtient 2,54 g de produit huileux. On le chromatographie sur silice, élue par un mélange cyclohexane, acétate d'éthyle (85–15) et isole 1,86 g de produit, qui recristallisé dans l'éther de pétrole (eb 40–70 °C) donne 1,3 g de produit attendu F = 78 °C.

$\alpha_D = +6° \pm 2$ (c = 0,6% benzène).

Spectre RMN ($CDCl_3$)
1,17–1,23 ppm pics attribués aux hydrogènes des méthyles géminés.
1,66–1,75 ppm pics attribués à l'hydrogène en position 1 du cyclopropyle.
2,1–2,19–2,24–2,33 ppm pics attribués à l'hydrogène en position 3 du cyclopropyle.
5,2 à 5,8 ppm pics attribués à l'hydrogène en position 3 de la chaîne latérale éthylénique.
6,2 à 6,6 ppm pics attribués aux hydrogènes en position 1 et 2 de la chaîne latérale éthylénique.
6,3 à 6,5 ppm pics attribués à l'hydrogène porté par le même carbone que le CN.
7,0 à 7,6 ppm pics attribués aux hydrogènes aromatiques.

Exemple 10

(1R,cis) 2,2-diméthyl 3-(4',4'-dibromo buta 1',3'-diényl) cyclopropane carboxylate de (S) α-cyano 3-phénoxybenzyle.

Stade A: chlorure d'acide (1R,cis) 2,2-diméthyl 3-(4',4'-dibromo buta 1',3'-diényl) cyclopropane carboxylique.

On opère comme à l'exemple 7, stade A, en partant de 7 g d'acide (1R,cis) 2,2-diméthyl 3-(4',4'-dibromo buta 1',3'-diényl) cyclopropane carboxylique obtenu à l'exemple 4 et obtient 7,6 g de produit attendu.

Stade B: (1R,cis) 2,2-diméthyl 3-(4',4'-dibromo buta 1',3'-diényl) cyclopropane carboxylate de (S) α-cyano 3-phénoxy benzyle.

De la même façon qu'au stade B de l'exemple 7 on prépare 5,83 g de produit brut à partir du produit obtenu ci-dessus et de 2,9 g d'alcool (S) α-cyano 3-phénoxybenzylique. On chromatographie sur silice (éluant: éther de pétrole – éther éthylique (75/25)) et obtient 1,61 g de produit attendu.

$\alpha_D$ = +46°3 ± 2 (c = 0,4% benzène).

Spectre RMN ($CDCl_3$)

1,22–1,23 ppm pics attribués aux hydrogènes des méthyles géminés.

6,4 ppm pic attribué à l'hydrogène porté par le même carbone que le CN.

6,2 à 6,5 ppm pics attribués aux hydrogènes éthyléniques.

6,9 à 7,7 ppm pics attribués aux hydrogènes aromatiques.

Exemple 11

(1R,trans) 2,2-diméthyl 3-(4',4'-dibromo buta 1',3'-diényl) cyclopropane carboxylate de (RS) α-cyano 3-phénoxybenzyle.

Stade A: Chlorure d'acide (1R,trans) 2,2-diméthyl 3-(4',4'-dibromo buta 1',3'-diényl) cyclopropane carboxylique.

On opère comme au stade A de l'exemple 7 en partant de 1,8 g d'acide (1R,trans) 2,2-diméthyl 3-(4',4'-dibromo buta 1',3'-diényl) cyclopropane carboxylique obtenu à l'exemple 6 et obtient 1,9 g de produit attendu.

Stade B: (1R,trans) 2,2-diméthyl 3-(4',4'-dibromo buta 1',3'-diényl) cyclopropane carboxylate de (RS) α-cyano 3-phénoxybenzyle.

On traite le produit obtenu ci-dessus de la même façon qu'au stade B de l'exemple 7 avec 1,4 g d'alcool (RS) α-cyano phénoxybenzylique et obtient 2,7 g de produit brut. On chromatographie ce dernier sur silice, élue par le système hexane-éther isopropylique (8–2) et recueille 1,33 g de produit attendu.

$\alpha_D$ = −14° ± 2,5 (c = 0,5% benzène). Spectre RMN ($CDCl_3$)

1,18–1,23 ppm pics attribués aux hydrogènes des méthyles géminés du produit S.

1,23–1,32 ppm pics attribués aux hydrogènes des méthyles géminés du produit R.

1,65 à 1,75 ppm et 2,05 à 2,38 ppm pics attribués aux hydrogènes du cyclopropyle,

de 6,9 à 7,1 ppm pics attribués à l'hydrogène dédoublé en 3 de la chaîne latérale.

5,4 à 5,9 ppm pics attribués à l'hydrogène en 1 de la chaîne latérale,

de 6,1 à 6,6 ppm pics attribués à l'hydrogène dédoublé en 2 de la chaîne latérale.

6,4 ppm pic attribué à l'hydrogène porté par le même carbone que le –C≡N.

6,9 à 7,6 ppm pics attribués aux hydrogènes aromatiques.

Exemple 12

(1R,trans) 2,2-diméthyl 3-(4',4'-dibromo buta 1',3'-diényl) cyclopropane carboxylate de (S) α-cyano 3-phénoxybenzyle.

On opère comme au début du stade B de l'exemple 7 à partir du chlorure d'acide (1R,trans) 2,2-diméthyl 3-(4',4'-dibromo buta 1',3'-diényl) cyclopropane carboxylique obtenu à l'exemple 11 et de 6,11 g d'alcool (S) α-cyano 3-phénoxy-benzylique.

Après agitation pendant 17 heures à 20 °C on verse le mélange réactionnel dans 200 cm³ d'eau, agite, décante et extrait au benzène. Les phases organiques sont lavées avec de l'acide chlorhydrique 0,1N puis à l'eau. On sèche, concentre à sec sous pression réduite et obtient 15,4 g de produit. Après 2 chromatographies sur silice (1er éluant hexane-acétate d'éthyle (9–1); 2[e] éluant hexane-éther isopropylique (8–2) on isole 4,4 g de produit que l'on recristallise dans le mélange hexane-éther isopropylique (1–1). On obtient 2,2 g de produit F = 110 °C.

$\alpha_D$ = +16° ± 1° (c = 1% benzène).

Spectre RMN ($CDCl_3$)

1,2–1,16 ppm pics attribués aux hydrogènes des méthyles géminés,

de 2,1 à 2,3 ppm pics attribués à l'hydrogène en position 3 du cyclopropane.

166–1,75 ppm pics attribués à l'hydrogène en position 1 du cyclopropane,

de 5,4 à 5,8 ppm pics attribués à l'hydrogène éthylénique en position 1 de la chaîne latérale,

de 6,1 à 6,5 ppm pics attribués à l'hydrogène éthylénique en position 2 de la chaîne latérale.

6,8–7 ppm pics attribués à l'hydrogène éthylénique en position 3 de la chaîne latérale.

6,4 ppm pic attribué à l'hydrogène porté par le même carbone que le CN,

de 6,9 à 7,6 ppm pics attribués aux hydrogènes aromatiques.

Exemple 13

(1R,cis) 2,2-diméthyl 3-(4',4'-dichloro buta 1',3'-diényl) cyclopropane carboxylate de (S) 3-allyl 2-méthyl 4-oxo cyclopent 2-en-1-yle.

On opère selon la technique employée à l'exemple 7 à partir de 2,5 g d'acide (1R,cis) 2,2-diméthyl 3-(4',4'-dichloro buta-1',3'-diényl) cyclopropane carboxylique préparé à l'exemple 1 et 1,8 g de (S) 3-allyl 1-hydroxy 2-méthyl 4-oxo cyclopent-2-ène. On obtient 3,23 g de produit brut et après chromatographie sur silice (éluant: hexane-acétate d'éthyle 9/1) 600 mg de produit pur.

$\alpha_D$ = +14° ± 2° (c = 0,4% benzène).

Spectre RMN ($CDCl_3$)

1,22–1,28 ppm pics attribués aux hydrogènes des méthyles géminés.

2 ppm pic attribué aux hydrogènes du méthyle de l'allethrone.

2,93 –3,0 ppm pics attribués aux hydrogènes en position 1 de l'allyle.

4,7 à 5,2 ppm pics attribués aux hydrogènes en position 3 de l'allyle.

5,6–5,7 ppm pics attribués à l'hydrogène porté par le même carbone que CN.

5,4 à 6,3 ppm pics attribués à l'hydrogène en position 2 de l'allyle.

6 à 6,5 ppm pics attribués aux hydrogènes éthyléniques sur la chaîne latérale en 3 du cyclopropyle.

Exemple 14

(1R,trans) 2,2-diméthyl 3-(4',4'-dichloro buta 1',3'-diényl) cyclopropane carboxylate de (S) 3-allyl 2-méthyl 4-oxo cyclopent-2-en-1-yle.

On opère comme à l'exemple 7 à partir de 1,53 g d'acide (1R,trans) 2,2-diméthyl 3-(4',4'-dichloro buta-1',3'-diényl) cyclopropane carboxylique, obtenu à l'exemple 3 et 1,07 g de (S) 3-allyl 1-hydroxy 2-méthyl 4-oxo cyclopent-2-ène. On obtient 2,17 g de produit brut et après chromatographie sur silice (éluant benzène – acétate d'éthyle – 9/1) 850 mg de produit pur.

$n_D^{23,5} = 1,5539$ $\alpha_D = -76° \pm 2,5$ (c = 0,4% benzène).

Spectre RMN ($CDCl_3$)

1,17–1,25 ppm pics attribués aux hydrogènes des méthyles géminés.

2,0 ppm pic attribué aux hydrogènes du méthyle de l'allethrolone.

1,6–1,69 ppm pics attribués à l'hydrogène en position 1 du cyclopropyl.

4,7 à 5,2 pics attribués aux hydrogènes en position 3 de l'allyle.

5,3 à 6,2 ppm pics attribués à l'hydrogène en position 2 de l'allyle et à l'hydrogène en position 1 de la chaîne latérale éthylénique du cyclopropyle.

6,2 à 6,5 ppm pics attribués aux hydrogènes éthyléniques en position 2 et 3 de la chaîne latérale du cyclopropyle.

Exemple 15

(1R,trans) 2,2-diméthyl 3-(4',4'-dibromo buta 1',3'-diényl) cyclopropane carboxylate de (S) 3-allyl 2-méthyl 4-oxo cyclopent-2-en-1-yle.

On opère selon la technique employée à l'exemple 7 à partir de 2,0 g d'acide (1R,trans) 2,2-diméthyl 3-(4',4'-dibromo buta 1',3'-diényl) cyclopropane carboxylique préparé à l'exemple 6 et de 1 g de (S) 3-allyl 1-hydroxy 2-méthyl 4-oxo cyclopent-2-ène. On obtient 2,5 g de produit brut et après chromatographie sur silice (système: hexane éther isopropylique – 8/2) 1,5 g de produit pur.

$\alpha_D = -55°5 \pm 3°$ (c = 0,3% benzène).

Spectre RMN ($CDCl_3$)

1,2–1,28 ppm pics attribués aux hydrogènes des méthyles géminés.

1,62–1,71 ppm pics attribués à l'hydrogène en position 1 du cyclopropyle.

2,03 ppm pic attribué aux hydrogènes du méthyle de l'alléthrolone.

4,8–5,2 ppm pics attribués aux hydrogènes en position 3 de l'allyle.

5,4 à 6,5 ppm pics attribués à l'hydrogène en position 2 de l'allyle.

6,9–7,0 ppm pics attribués à l'hydrogène en position 3 de la chaîne éthylénique latérale du cyclopropyle,

de 6,1 à 6,5 ppm pics attribués à l'hydrogène en position 2 de la chaîne éthylénique latérale du cyclopropyle.

5,43 à 5,83 ppm pics attribués à l'hydrogène éthylénique en position 1 de la chaîne latérale du cyclopropyle.

Exemple 16

(1R,cis) 2,2-diméthyl 3-(4',4'-dibromo buta 1',3'-diényl) cyclopropane carboxylate de (S) 3-allyl 2-méthyl 4-oxo cyclopent-2-en-1-yle.

On opère selon la technique employée à l'exemple 7 à partir de 6,2 g d'acide (1R,cis) 2,2-diméthyl 3-(4',4'-dibromo buta 1',3'-diényl) cyclopropane carboxylique préparé à l'exemple 4 et 0,98 g de (S) 3-allyl 1-hydroxy 2-méthyl 4-oxo cyclopent-2-ène. On obtient 2,2 g de produit brut que l'on chromatographie sur silice (éluant hexane – éther isopropylique 8/2) pour isoler 760 mg de produit attendu.

$\alpha_D = +1,25° \pm 1$ (c = 1,5% benzène).

Spectre RMN ($CDCl_3$)

1,22–1,28 ppm pics attribués aux hydrogènes des méthyles géminés.

2,0 ppm pic attribué aux hydrogènes du méthyle de l'allethrolone.

4,8 à 5,2 ppm pics attribués aux hydrogènes en position 3 de l'allyle.

5,7 ppm pic attribué à l'hydrogène en position 1 de l'allethrolone.

5,5 à 7,1 ppm pics attribués aux hydrogènes éthyléniques.

Préparation

(1R,cis,ΔZ) 3-(2'-formyl éthényl) 2,2-diméthyl cyclopropane carboxylate de terbutyle.

On mélange 3,4 g de (1R,cis) 2,2-diméthyl 3-(3',3'-diéthoxy prop-1-èn-1-yl) cyclopropane carboxylate de terbutyle (préparé selon H.J. Bestmann, K. Roth et M. Ettinger. Angew. Chem. Int. 18 [1979] n° 9), 5 $cm^3$ d'eau et 20 $cm^3$ d'acétone, refroidit à 0, +5 °C et ajoute quelques cristaux d'acide paratoluène sulfonique. On agite à (0, +5 °C) pendant 45 minutes, verse le mélange sur une solution aqueuse glacée saturée de bicarbonate de sodium, extrait au pentane, sèche, évapore le solvant et obtient 2,42 g de produit attendu.

Spectre RMN ($CDCl_3$)

1,28–1,33 ppm pics attribués aux $CH_3$ géminés.

2,47–2,78 ppm pics attribués à l'hydrogène en 3.

1,87–2,01 ppm pics attribués à l'hydrogène en 1.

1,45 ppm pic attribué aux hydrogènes du terbutyle.

6,85–7,3 ppm pics attribués à l'hydrogène en 1'.

5,93–6,06 et 6,13–6,27 ppm pics attribués à l'hydrogène en 2'.

10,1–10,2 ppm pics attribués à l'hydrogène en 3'.

Exemple 17

Acide (1R,cis,ΔZ) 2,2-diméthyl 3-(4',4'-dibromo buta 1',3'-diényl) cyclopropane carboxylique.

Stade A: (1R,cis,ΔZ) 2,2-diméthyl 3-(4',4'-dibromo buta 1',3'-diényl) cyclopropane carboxylate de terbutyle.

On mélange 3 $cm^3$ d'heptane, 4,5 $cm^3$ de terbutanol et 0,28 g de terbutylate de lithium, agite à 20 °C pendant 15 minutes puis ajoute 2,27 g de bromure de dibromométhyltriphénylphosphonium. On agite à 20 °C pendant 1 heure, refroidit à −60 °C et ajoute goutte à goutte une solution de 0,64 g de (1R,cis,ΔZ) 3-(2'-formyl éthényl) 2,2-diméthyl cyclopropane carboxylate de terbutyle dans 6 $cm^3$ d'heptane. On laisse la température remonter jusqu'à 0°C et agite pendant ½ heure puis verse le mélange sur un mélange de 60 $cm^3$ d'eau glacée et 60 $cm^3$ d'éther isopropylique. On décante, sèche la phase organique, traite au charbon actif, évapore le solvant, reprend le résidu à l'éther isopropylique, filtre, évapore le solvant et

obtient 1,48 g de produit attendu brut. On chromatographie ce produit sur silice en éluant au mélange hexane-éther isopropylique (95–5) et obtient 0,53 g de produit attendu.

Stade B: acide (1R,cis,ΔZ) 2,2-diméthyl 3-(4′,4′-dibromo buta 1′,3′-diényl) cyclopropane carboxylique.

On mélange 0,48 g de produit obtenu au stade A, 5 $cm^3$ de toluène et 0,030 g d'acide paratoluène sulfonique, porte au reflux pendant 15 minutes puis concentre à sec. On obtient 0,5 g de produit attendu utilisé tel quel dans l'exemple 18.

Exemple 18

(1R,cis,ΔZ) 2,2-diméthyl 3-(4′,4′-dibromo buta 1′,3′-diényl) cyclopropane carboxylate de (S) α-cyano 3-phénoxy benzyle.

On mélange 0,42 g de produit obtenu à l'exemple 17, 2 $cm^3$ de chlorure de méthylène et 0,04 g de diméthylaminopyridine. On agite pendant 10 minutes, refroidit à 0 °C et ajoute goutte à goutte un mélange de 0,27 g de dicyclohexylcarbodiimide dans 2 $cm^3$ de chlorure de méthylène. On agite la suspension obtenue pendant 15 minutes à 0 °C puis ajoute 0,34 g d'alcool (S) α-cyano 3-phénoxybenzylique dans 3 $cm^3$ de chlorure de méthylène. On laisse la température remonter à 20 °C, agite pendant 1 heure, filtre l'insoluble, concentre le filtrat à sec, chromatographie le résidu sur silice en éluant au mélange hexane-éther isopropylique (8–2) et obtient 0,57 g de produit attendu.

Spectre IR ($CHCl_3$)

Absorption à 1743 $cm^{-1}$ (c = O ester), à 1590–1490 $cm^{-1}$ (c = c aromatique), à 1390–1380 $cm^{-1}$ (gem diméthyl).

Spectre RMN ($CDCl_3$)

6,83 à 7,66 ppm pics attribués aux hydrogènes éthyléniques en 3′ et aromatiques.

5,66 à 6,5 ppm pics attribués aux hydrogènes en 1′, 2′.

6,41 ppm pic attribué à l'hydrogène porté par le même carbone que le CN.

1,23 ppm pic attribué aux $CH_3$ géminés.

Etude de l'activité des composés de l'invention

L'activité insecticide des exemples 7, 10 et 12 dénommés ci-après produits A, B, C a été recherchée sur les larves d'Epilachna Varivestris.

Les résultats expérimentaux, exprimés en DL 50 sont résumés dans le tableau suivant:

| | DL 50 ng par insecte |
|---|---|
| Composé A | 1,8 |
| Composé B | 1,9 |
| Composé C | 2,9 |

Conclusion: Les composés A, B et C sont doués d'une forte activité insecticide vis-à-vis d'Epilachna Varivestris.

Exemple 19

Préparation d'un concentré émulsifiable.

On effectue un mélange homogène de:

| | |
|---|---|
| Produit de l'exemple 7 | 0,25 g |
| Butoxyde de pipéronyle | 1 g |
| Tween 80 | 0,25 g |
| Topanol A | 0,1 g |
| Eau | 98,4 g |

Exemple 20

Préparation d'un concentré émulsifiable.

On mélange intimement:

| | |
|---|---|
| Produit de l'exemple 7 | 0,015 g |
| Butoxyde de pipéronyle | 0,5 g |
| Topanol A | 0,1 g |
| Xylène | 99,385 g |

Exemple 21

Préparation d'un concentré émulsifiable.

On effectue un mélange homogène de:

| | |
|---|---|
| Produit de l'exemple 7 | 1,5 g |
| Tween 80 | 20 g |
| Topanol A | 0,1 g |
| Xylène | 78,4 g |

Exemple 22

Préparation d'une composition fumigène.

On mélange d'une façon homogène:

| | |
|---|---|
| Produit de l'exemple 7 | 0,25 g |
| Poudre de tabu | 25 g |
| Poudre de feuilles de cèdre | 40 g |
| Poudre de bois de pin | 33,75 g |
| Vert brillant | 0,5 g |
| p-nitrophénol | 0,5 g |

**Revendications pour les Etats contractants: IT, LU, SE**

1. Sous toutes leurs formes stéréoisomères possibles ou sous forme de mélanges de stéréoisomères, les composés de formule (I):

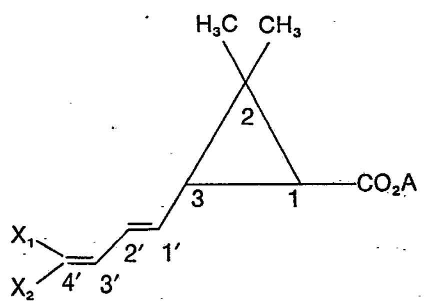
(I)

dans laquelle $X_1$ et $X_2$, identiques ou différents, représentent un atome d'halogène, et A représente,

– soit un atome d'hydrogène,

– soit un radical alcoyle renfermant de 1 à 18 atomes de carbone,

– soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylène dioxyle, le radical benzyle et les atomes d'halogène,

– soit un groupement

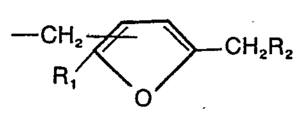

dans lequel le substituant $R_1$ représente un atome d'hydrogène ou un radical méthyle et le substituant $R_2$ un aryle monocyclique ou un groupement $-CH_2-C\equiv CH$ et notamment un groupement 5-benzyl 3-furyl méthyle,

– soit un groupement

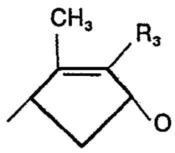

dans lequel $R_3$ représente un radical organique aliphatique comportant de 2 à 6 atomes de carbone et une ou plusieurs insaturations carbone-carbone et notamment le radical vinyle, propen-1-yle, buta-1,3-diényle ou buten-1-yle,

– soit un groupement

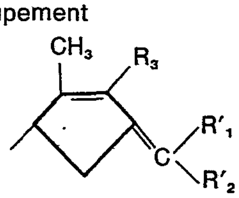

dans lequel $R_3$ conserve la même signification que précédemment, $R'_1$ et $R'_2$ identiques ou différents représentent un atome d'hydrogène, un atome d'halogène, un radical alcoyle renfermant de 1 à 6 atomes de carbone, un radical aryle comportant de 6 à 10 atomes de carbone, un groupement alcoyl-oxycarbonyle comportant de 2 à 5 atomes de carbone, ou un groupement cyano,

– soit un groupement

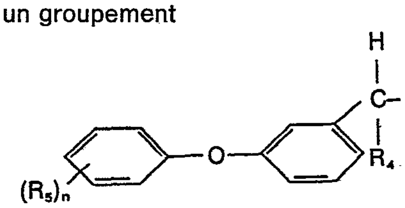

dans lequel $R_4$ représente un atome d'hydrogène, un groupement $-C\equiv N$, un radical méthyle, un radical $-CONH_2$, un radical $-CSNH_2$, ou un groupement $-C\equiv CH$, $R_5$ représente un atome de chlore ou un radical méthyle et n représente un nombre égal à 0, 1 ou 2, et notamment le groupement 3-phénoxybenzylique, α-cyano 3-phénoxybenzylique ou α-éthynyl 3-phénoxybenzylique,

– soit un groupement

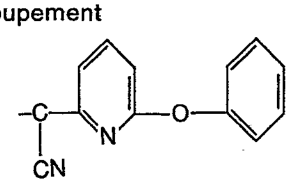

– soit un groupement

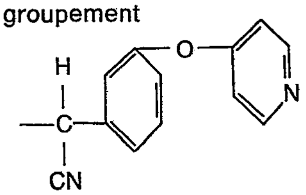

– soit un groupement

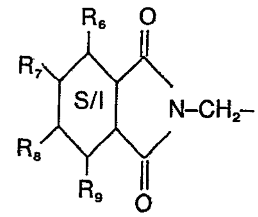

dans lequel les substituants $R_6$, $R_7$, $R_8$ et $R_9$ représentent un atome d'hydrogène, un atome de chlore ou un radical méthyle et dans lequel S/I symbolise un cycle aromatique ou un cycle analogue dihydro ou tétrahydro.

2. Les composés de formule (I) tels que définis à la revendication 1, pour lesquels la copule acide est de structure 1R,cis ou 1R,trans.

3. Les composés de formule (I) tels que définis à la revendication 1 ou 2, pour lesquels $X_1$ et $X_2$ sont identiques et représentent tous les deux un atome de brome ou de chlore.

4. Les composés de formule (I) tels que définis à l'une quelconque des revendications 1 à 3, pour lesquels A représente un atome d'hydrogène, ainsi que leurs sels.

5. Les composés de formule (I) tels que définis à l'une quelconque des revendications 1 à 3, pour lesquels A représente un radical alcoyle renfermant de 1 à 4 atomes de carbone et notamment le radical méthyle.

6. Les composés de formule (I) tels que définis à l'une quelconque des revendications 1 à 3, pour lesquels A représente un groupement α-cyano 3-phénoxy benzyle.

7. Les composés de formule (I) tels que définis à l'une quelconque des revendications 1 à 3 pour lesquels A représente un groupement 3-allyl 2-méthyl 4-oxo cyclopent 2-en-1-yle.

8. Procédé de préparation des composés de formule (I) tels que définis à la revendication 1, caractérisé en ce que l'on soumet le composé de formule (II)

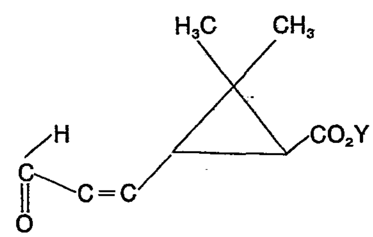
(II)

dans laquelle Y représente un atome d'hydrogène ou un radical alcoyle renfermant de 1 à 18 atomes de carbone, sous l'une quelconque de ses formes isomères ou sous forme d'un mélange d'isomères, à l'action d'un composé de formule (III):

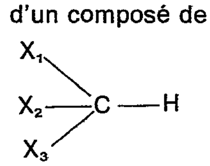
(III)

dans laquelle, soit les substituants $X_1$, $X_2$ et $X_3$ sont identiques et représentent un atome de chlore ou de brome, soit au moins deux des substituants $X_1$, $X_2$ et $X_3$ sont différents $X_1$, $X_2$ et $X_3$ représentant alors un atome de fluor, de chlore ou de brome,

$X_1$, $X_2$ et $X_3$ étant de poids atomiques égaux ou croissants de $X_1$ à $X_3$, pour obtenir le composé de formule (IV)

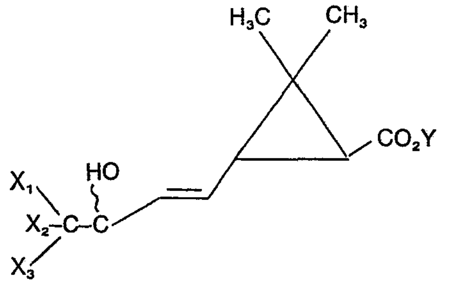

(IV)

dont on bloque l'hydroxyle par action d'un acide ou d'un dérivé fonctionnel d'acide $RCO_2H$, puis soumet le produit obtenu de formule:

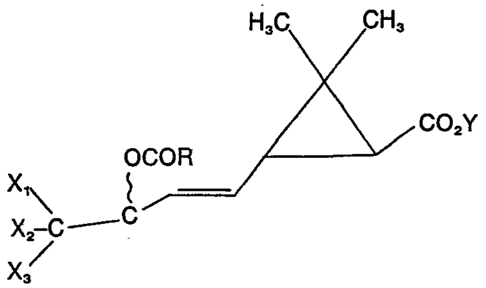

(V)

à l'action d'un agent de réduction, pour obtenir après élimination du substituant $X_3$ qui est le substituant ou l'un des substituants de poids atomique le plus élevé, le composé de formule ($I_A$)

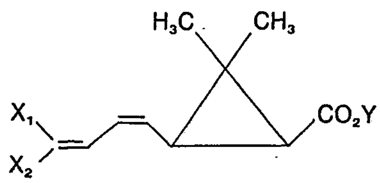

($I_A$)

dans laquelle $X_1$ et $X_2$ soit sont identiques et représentent un atome de chlore ou de brome, soit sont différents et représentent un atome de fluor, de chlore ou de brome, puis soumet, si désiré, un composé de formule $I_A$, dans lequel Y représente un radical alcoyle renfermant de 1 à 18 atomes de carbone, à l'action d'un agent de saponification, pour obtenir un composé de formule (I) dans lequel A représente un atome d'hydrogène, ou un sel de ce composé, ou soumet, si désiré, soit un composé de formule ($I_A$) dans lequel Y est un radical alcoyle renfermant de 1 à 18 atomes de carbone, soit un composé de formule ($I_A$) dans lequel Y représente un atome d'hydrogène, soit un dérivé fonctionnel de ce dernier, à l'action d'un alcool de formule $A_1OH$ ou d'un dérivé fonctionnel de cet alcool, formule dans laquelle $A_1$ a la même signification que A dans la revendication 1, à l'exception de l'hydrogène, pour obtenir le composé de formule (I) correspondant.

9. Variante du procédé selon la revendication 8, caractérisé en ce que l'on soumet selon la réaction de Wittig le composé de formule (II) telle que définie à la revendication 8, sous l'une quelconque de ses formes isomères ou sous forme d'un mélange d'isomères, à l'action d'un composé de formule

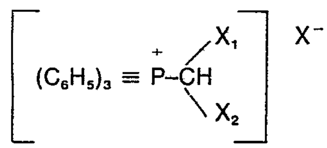

dans laquelle $X_1$, $X_2$ sont définis comme à la revendication 1 et $X^-$ représente un anion halogénure pour obtenir le composé de formule ($I_A$), puis, si désiré, poursuit la synthèse comme à la revendication 8.

10. Utilisation des composés de formule (I) tels que définis à l'une quelconque des revendications 1 à 7, à la lutte contre les parasites des végétaux.

11. Les compositions destinées à la lutte contre les parasites des végétaux et des animaux à sang chaud, caractérisées en ce qu'elles renferment comme principe actif, au moins l'un des produits définis à l'une quelconque des revendications 1 à 7.

12. Les compositions insecticides selon la revendication 11, renfermant comme principe actif au moins l'un des produits définis à l'une quelconque des revendications 1 à 7.

13. Les compositions parfumantes renfermant comme principe actif au moins un produit de formule (I) tel que défini à la revendication 1.

14. Les compositions parfumantes renfermant comme principe actif au moins un produit de formule (I) tel que défini à la revendication 5.

15. A titre de médicaments, les composés définis à l'une quelconque des revendications 1 à 7.

16. Les compositions pharmaceutiques renfermant comme principe actif au moins l'un des médicaments définis à la revendication 15.

17. Les compositions destinées à l'alimentation animale renfermant comme principe actif au moins l'un des produits définis à l'une quelconque des revendications 1 à 7, associé à un aliment destiné aux animaux.

**Revendications pour l'Etat contractant: AT**

1. Procédé pour préparer sous toutes leurs formes stéréoisomères possibles ou sous forme de mélanges de stéréoisomères, les composés de formule (I):

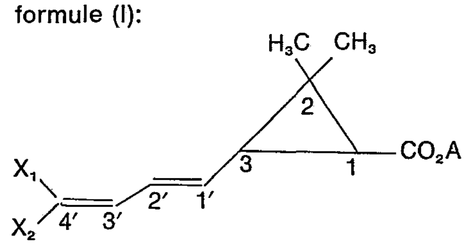

(I)

dans laquelle $X_1$ et $X_2$, identiques ou différents, représentent un atome d'halogène, et A représente,

– soit un atome d'hydrogène,

– soit un radical alcoyle renfermant de 1 à 18 atomes de carbone,

– soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylène dioxyle, le radical benzyle et les atomes d'halogène,

– soit un groupement

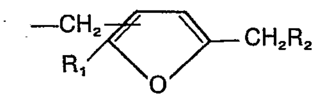

dans lequel le substituant $R_1$ représente un atome d'hydrogène ou un radical méthyle et le substituant $R_2$ un aryle monocyclique ou un groupement $-CH_2-C\equiv CH$ et notamment un groupement 5-benzyl 3-furyl méthyle,

– soit un groupement

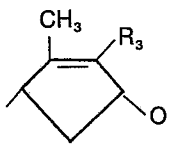

dans lequel $R_3$ représente un radical organique aliphatique comportant de 2 à 6 atomes de carbone et une ou plusieurs insaturations carbone-carbone et notamment le radical vinyle, propen-1-yle, buta-1,3-diényle ou buten-1-yle,

– soit un groupement

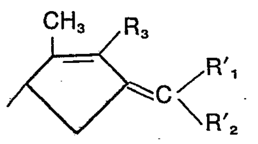

dans lequel $R_3$ conserve la même signification que précédemment, $R'_1$ et $R'_2$ identiques ou différents représentent un atome d'hydrogène, un atome d'halogène, un radical alcoyle renfermant de 1 à 6 atomes de carbone, un radical aryle comportant de 6 à 10 atomes de carbone, un groupement alcoyl-oxycarbonyle comportant de 2 à 5 atomes de carbone, ou un groupement cyano,

– soit un groupement

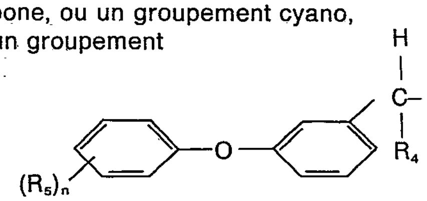

dans lequel $R_4$ représente un atome d'hydrogène, un groupement $-C\equiv N$, un radical méthyle, un radical $-CONH_2$, un radical $-CSNH_2$, ou un groupement $-C\equiv CH$, $R_5$ représente un atome de chlore ou un radical méthyle et n représente un nombre égal à 0, 1 ou 2, et notamment le groupement 3-phénoxybenzylique, α-cyano 3-phénoxybenzylique ou α-éthyl 3-phénoxybenzylique,

– soit un groupement

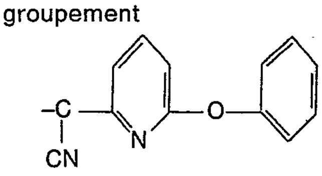

– soit un groupement

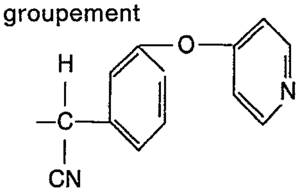

– soit un groupement

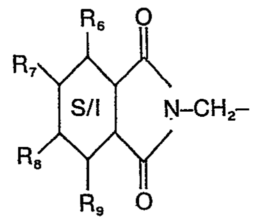

dans lequel les substituants $R_6$, $R_7$, $R_8$ et $R_9$ représentent un atome d'hydrogène, un atome de chlore ou un radical méthyle et dans lequel S/I symbolise un cycle aromatique ou un cycle analogue dihydro ou tétrahydro, caractérisé en ce que l'on soumet le composé de formule (II):

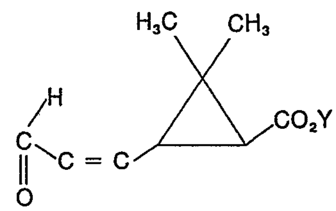

(II)

dans laquelle Y représente un atome d'hydrogène ou un radical alcoyle renfermant de 1 à 18 atomes de carbone, sous l'une quelconque de ses formes isomères ou sous forme d'un mélange d'isomères, à l'action d'un composé de formule (III):

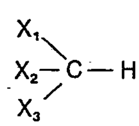

(III)

dans laquelle, soit les substituants $X_1$, $X_2$ et $X_3$ sont identiques et représentent un atome de chlore ou de brome, soit au moins deux des substituants $X_1$, $X_2$ et $X_3$ sont différents $X_1$, $X_2$ et $X_3$ représentant alors un atome de fluor, de chlore ou de brome, $X_1$, $X_2$ et $X_3$ étant de poids atomiques égaux ou croissants de $X_1$ à $X_3$, pour obtenir le composé de formule (IV)

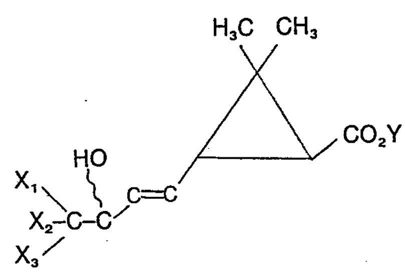

(IV)

dont on bloque l'hydroxyle par action d'un acide ou d'un dérivé fonctionnel d'acide $RCO_2H$, puis soumet le produit obtenu de formule:

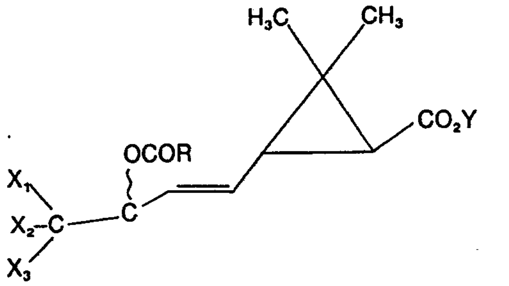

(V)

à l'action d'un agent de réduction, pour obtenir après élimination du substituant $X_3$ qui est le substituant ou l'un des substituants de poids atomique le plus élevé, le composé de formule ($I_A$)

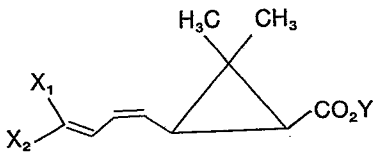

($I_A$)

dans laquelle $X_1$ et $X_2$ soit sont identiques et représentent un atome de chlore ou de brome, soit sont différents et représentent un atome de fluor, de chlore ou de brome, puis soumet, si désiré, un composé de formule $I_A$, dans lequel Y représente un radical alcoyle renfermant de 1 à 18 atomes de carbone, à l'action d'un agent de saponification, pour obtenir un composé de formule (I) dans lequel A représente un atome d'hydrogène, ou un sel de ce composé, ou soumet, si désiré, soit un composé de formule ($I_A$) dans lequel Y est un radical alcoyle renfermant de 1 à 18 atomes de carbone, soit un composé de formule ($I_A$) dans lequel Y représente un atome d'hydrogène, soit un dérivé fonctionnel de ce dernier, à l'action d'un alcool de formule $A_1$ OH ou d'un dérivé fonctionnel de cet alcool, formule dans laquelle $A_1$ a la même signification que A, à l'exception de l'hydrogène, pour obtenir le composé de formule (I) correspondant.

2. Variante du procédé selon la revendication 1, caractérisé en ce que l'on soumet selon la réaction de Wittig le composé de formule (II) telle que définie à la revendication 1, sous l'une quelconque de ses formes isomères ou sous forme d'un mélange d'isomères, à l'action d'un composé de formule

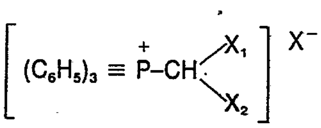

dans laquelle $X_1$, $X_2$ sont définis comme à la revendication 1 et $X^-$ représente un anion halogénure pour obtenir le composé de formule ($I_A$), puis, si désiré, poursuit la synthèse comme à la revendication 1.

3. Procédé selon la revendication 1 ou 2, caractérisé en ce que l'on utilise au départ un composé de formule (II), de structure 1R,cis ou 1R,trans.

4. Procédé selon l'une quelconque des revendications 1 à 3, caractérisé en ce que l'on utilise au départ un composé de formule (III), dans laquelle $X_1$ et $X_2$ sont identiques et représentent tous les deux un atome de brome ou de chlore.

5. Procédé selon l'une quelconque des revendications 1 à 4, caractérisé en ce que l'on utilise au départ un composé de formule $A_1OH$, dans laquelle $A_1$ représente un radical alcoyle renfermant de 1 à 4 atomes de carbone et notamment le radical méthyle.

6. Procédé selon l'une quelconque des revendications 1 à 4, caractérisé en ce que l'on utilise au départ un composé de formule $A_1OH$, dans laquelle $A_1$ représente un groupement α-cyano 3-phénoxybenzyle.

7. Procédé selon l'une quelconque des revendications 1 à 4, caractérisé en ce que l'on utilise au départ un composé de formule $A_1OH$, dans laquelle $A_1$ représente un groupement 3-allyl 2-méthyl 4-oxo cyclopent-2-en-1-yle.

8. Application comme pesticides des composés de formule (I), tels que définis à l'une quelconque des revendications 1 et 3 à 7.

9. Les compositions pesticides destinées à la lutte contre les parasites, caractérisées en ce qu'elles renferment comme principe actif au moins l'un des produits définis à l'une quelconque des revendications 1 et 3 à 7.

10. Les compositions insecticides selon la revendication 9, renfermant comme principe actif au moins l'un des produits définis à l'une quelconque des revendications 1 et 3 à 7.

11. Application comme agents parfumants des composés de formule (I), tels que définis à la revendication 1.

12. Les compositions parfumantes renfermant comme principe actif au moins un produit de formule (I) tel que défini à la revendication 1.

13. Les compositions parfumantes renfermant comme principe actif au moins un produit de formule (I) tel que défini à la revendication 5.

**Revendications pour les Etats contractants: BE, CH, DE, FR, GB, LI, NL**

1. Sous toutes leurs formes stéréoisomères possibles ou sous forme de mélanges de stéréoisomères, les composés de formule (I):

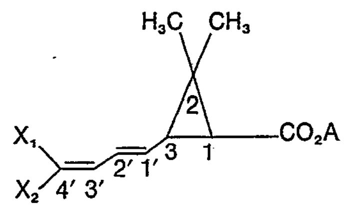

(I)

dans laquelle $X_1$ et $X_2$, identiques ou différents, représentent un atome d'halogène, et A représente,

– soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylène dioxyle, le radical benzyle et les atomes d'halogène,

– soit un groupement

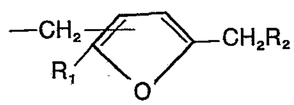

dans lequel le substituant $R_1$ représente un atome d'hydrogène ou un radical méthyle et le substituant $R_2$ un aryle monocyclique ou un groupement $-CH_2-C \equiv CH$ et notamment un groupement 5-benzyl 3-furyl méthyle,

– soit un groupement

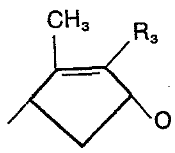

dans lequel $R_3$ représente un radical organique aliphatique comportant de 2 à 6 atomes de carbone et une ou plusieurs insaturations carbone-carbone et notamment le radical vinyle, propen-1-yle, buta-1,3-diényle ou buten-1-yle,

– soit un groupement

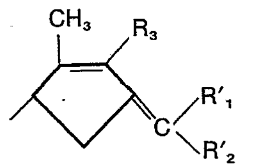

dans lequel $R_3$ conserve la même signification que précédemment, $R'_1$ et $R'_2$ identiques ou différents représentent un atome d'hydrogène, un atome d'halogène, un radical alcoyle renfermant de 1 à 6 atomes de carbone, un radical aryle comportant de 6 à 10 atomes de carbone, un groupement alcoyl-oxycarbonyle comportant de 2 à 5 atomes de carbone, ou un groupement cyano,

– soit un groupement

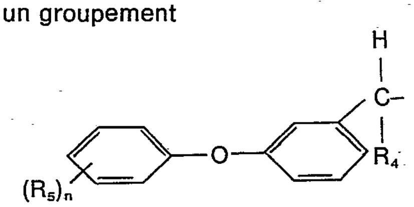

dans lequel $R_4$ représente un atome d'hydrogène, un groupement $-C \equiv N$, un radical méthyle, un radical $-CONH_2$, un radical $-CSNH_2$, ou un groupement $-C \equiv CH$, $R_5$ représente un atome de chlore ou un radical méthyle et n représente un nombre égal à 0, 1 ou 2, à la condition que si n = O, $R_4$ ne représente ni un atome d'hydrogène, ni un groupement $C \equiv N$, ni un groupement $C \equiv CH$,

– soit un groupement

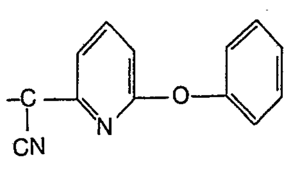

– soit un groupement

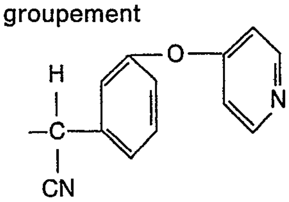

– soit un groupement

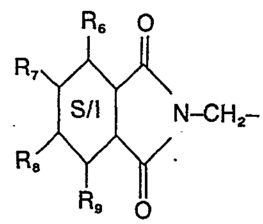

dans lequel les substituants $R_6$, $R_7$, $R_8$ et $R_9$ représentent un atome d'hydrogène, un atome de chlore ou un radical méthyle et dans lequel S/I symbolise un cycle aromatique ou un cycle analogue dihydro ou tétrahydro.

2. Sous toutes leurs formes stéréoisomères possibles ou sous forme de mélanges de stéréoisomères, les composés de formule ($I_a$):

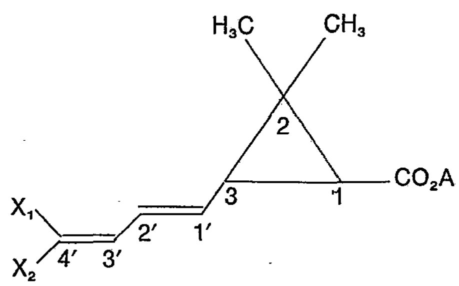

($I_a$)

dans laquelle la copule acide cyclopropanique est de structure 1R,cis ou 1R,trans et dans laquelle $X_1$ et $X_2$, identiques ou différents, représentent un atome d'halogène, et A représente,

– soit un atome d'hydrogène,

– soit un radical alcoyle renfermant de 1 à 18 atomes de carbone,

– soit un radical benzyle éventuellement substitué par un ou plusieurs radicaux choisis dans le groupe constitué par les radicaux alcoyles comportant de 1 à 4 atomes de carbone les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylène dioxyle, le radical benzyle et les atomes d'halogène,

– soit un groupement

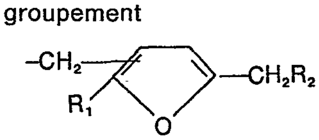

dans lequel le substituant $R_1$ représente un atome d'hydrogène ou un radical méthyle et le substituant $R_2$ un aryle monocyclique ou un groupement $-CH_2-C \equiv CH$ et notamment un groupement 5-benzyl 3-furyl méthyle,

– soit un groupement

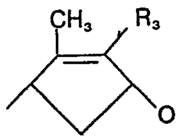

dans lequel $R_3$ représente un radical organique aliphatique comportant de 2 à 6 atomes de carbone et une ou plusieurs insaturations carbone-carbone et notamment le radical vinyle, propen-1-yle, buta-1,3-diényle ou buten-1-yle,

– soit un groupement

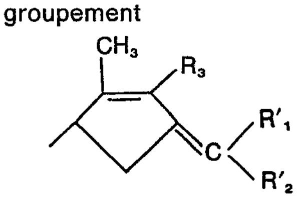

dans lequel $R_3$ conserve la même signification que précédemment, $R'_1$ et $R'_2$ identiques ou différents représentent un atome d'hydrogène, un atome d'halogène, un radical alcoyle renfermant de 1 à 6 atomes de carbone, un radical aryle comportant de 6 à 10 atomes de carbone, un groupement alcoyl-oxycarbonyle comportant de 2 à 5 atomes de carbone, ou un groupement cyano,

– soit un groupement

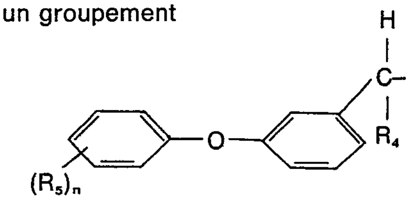

dans lequel $R_4$ représente un atome d'hydrogène, un groupement $-C\equiv N$, un radical méthyle, un radical $-CONH_2$, un radical $-CSNH_2$, ou un groupement $-C\equiv CH$, $R_5$ représente un atome de chlore ou un radical méthyle et n représente un nombre égal à 0, 1 ou 2, et notamment le groupement 3-phénoxybenzylique, α-cyano 3-phénoxybenzylique ou α-éthynyl 3-phénoxybenzylique,

– soit un groupement

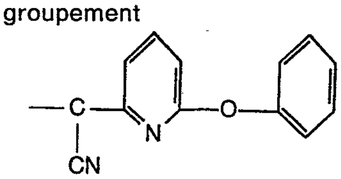

– soit un groupement

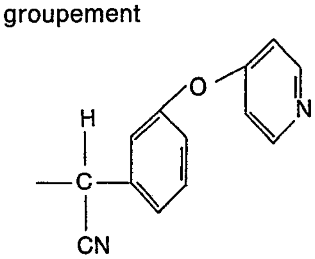

– soit un groupement

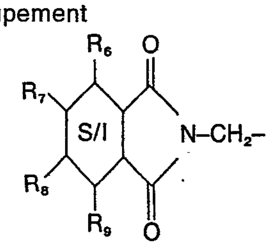

dans lequel les substituants $R_6$, $R_7$, $R_8$ et $R_9$ représentent un atome d'hydrogène, un atome de chlore ou un radical méthyle et dans lequel S/I symbolise un cycle aromatique ou un cycle analogue dihydro ou tétrahydro.

3. Les composés de formule (I) et ($I_a$) tels que définis à la revendication 1 ou 2, pour lesquels $X_1$ et $X_2$ sont identiques et représentent tous les deux un atome de brome ou de chlore.

4. Les composés de formule ($I_a$) tels que définis à la revendication 2 ou 3, pour lesquels A représente un atome d'hydrogène, ainsi que leurs sels.

5. Les composés de formule ($I_a$) tels que définis à la revendication 2 ou 3, pour lesquels A représente un radical alcoyle renfermant de 1 à 4 atomes de carbone et notamment le radical méthyle.

6. Les composés de formule ($I_a$) tels que définis à l'une quelconque des revendications 2 ou 3 pour lesquels A représente un groupement α-cyano 3-phénoxybenzyle.

7. Les composés de formule (I) tels que définis à l'une quelconque des revendications 1, 2 ou 3 pour lesquels A représente un groupement 3-allyl 2-méthyl 4-oxo cyclopent-2-en-1-yle.

8. Procédé de préparation des composés de formule (I) tels que définis à la revendication 1, caractérisé en ce que l'on soumet le composé de formule (II):

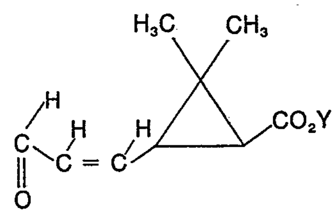

(II)

dans laquelle Y représente un atome d'hydrogène ou un radical alcoyle renfermant de 1 à 18 atomes de carbone, sous l'une quelconque de ses formes isomères ou sous forme d'un mélange d'isomères, à l'action d'un composé de formule (III):

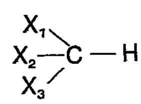

(III)

dans laquelle, soit les substituants $X_1$, $X_2$ et $X_3$ sont identiques et représentent un atome de chlore ou de brome, soit au moins deux des substituants $X_1$, $X_2$ et $X_3$ sont différents $X_1$, $X_2$ et $X_3$ représentant alors un atome de fluor, de chlore ou de brome, $X_1$, $X_2$ et $X_3$ étant de poids atomiques égaux ou croissants de $X_1$ à $X_3$, pour obtenir le composé de formule (IV)

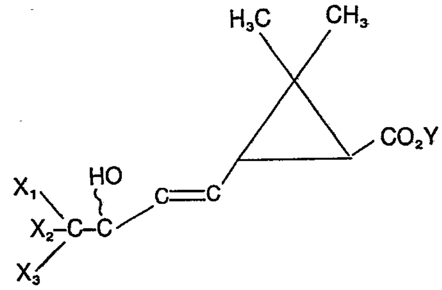

(IV)

dont on bloque l'hydroxyle par action d'un acide ou d'un dérivé fonctionnel d'acide $RCO_2H$, puis soumet le produit obtenu de formule

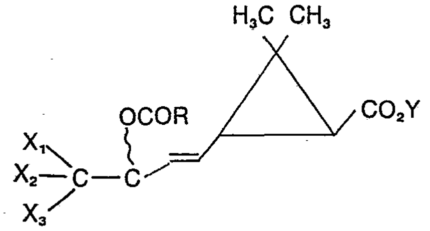

(V)

à l'action d'un agent de réduction, pour obtenir après élimination du substituant $X_3$ qui est le substituant ou l'un des substituants de poids atomique le plus élevé, le composé de formule ($I_A$)

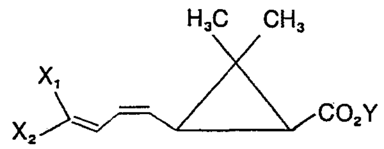

($I_A$)

dans laquelle $X_1$ et $X_2$ soit sont identiques et représentent un atome de chlore ou de brome, soit sont différents et représentent un atome de fluor, de chlore ou de brome, puis soumet, un composé de formule $I_A$, dans lequel Y représente un radical alcoyle renfermant de 1 à 18 atomes de carbone, à l'action d'un agent de saponification, pour obtenir un composé de formule (I) dans lequel A représente un atome d'hydrogène, ou un sel de ce composé, ou soumet, soit un somposé de formule ($I_A$) dans lequel Y est un radical alcoyle renfermant de 1 à 18 atomes de carbone, soit un composé de formule ($I_A$) dans lequel Y représente un atome d'hydrogène, soit un dérivé fonctionnel de ce dernier, à l'action d'un alcool de formule $A_1OH$ ou d'un dérivé fonctionnel de cet alcool, formule dans laquelle A a la même signification que dans la revendication 1, pour obtenir le composé de formule (I) correspondant.

9. Procédé de préparation des composés de formule ($I_a$) tels que définis à la revendication 2, caractérisé en ce que l'on soumet le composé de formule ($II_A$):

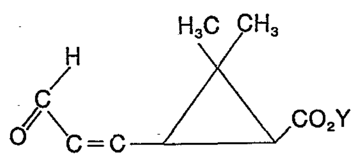

($II_A$)

dans laquelle Y représente un atome d'hydrogène ou un radical alcoyle renfermant de 1 à 18 atomes de carbone, sous l'une quelconque de ses formes isomères ou sous forme d'un mélange d'isomères, à l'action d'un composé de formule (III):

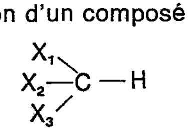

(III)

dans laquelle, soit les substituants $X_1$, $X_2$ et $X_3$ sont identiques et représentent un atome de chlore ou de brome, soit au moins deux des substituants $X_1$, $X_2$ et $X_3$ sont différents $X_1$, $X_2$ et $X_3$ représentant alors un atome de fluor, de chlore ou de brome, $X_1$, $X_2$ et $X_3$ étant de poids atomiques égaux ou croissants de $X_1$ à $X_3$, pour obtenir le composé de formule ($IV_A$)

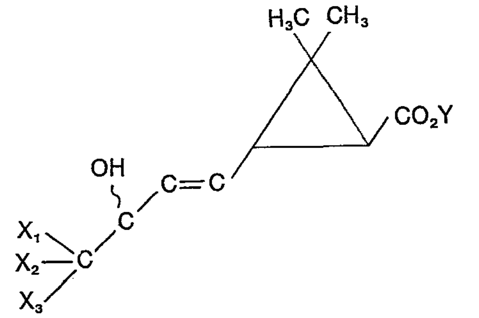

($IV_A$)

dont on bloque l'hydroxyle par action d'un acide ou d'un dérivé fonctionnel d'acide $RCO_2H$, puis soumet le produit obtenu de formule

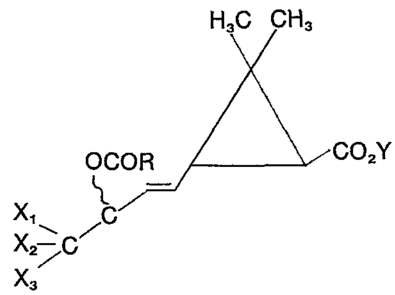

($V_A$)

à l'action d'un agent de réduction, pour obtenir après élimination du substituant $X_3$ qui est le substituant ou l'un des substituants de poids atomique le plus élevé, le composé de formule ($I_A$)

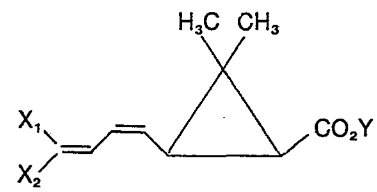

($I_A$)

dans laquelle $X_1$ et $X_2$ soit sont identiques et représentent un atome de chlore ou de brome, soit sont différents et représentent un atome de fluor, de chlore ou de brome, puis soumet, si désiré, un composé de formule $I_A$, dans lequel Y représente un radical alcoyle renfermant de 1 à 18 atomes de carbone, à l'action d'un agent de saponification, pour obtenir un composé de formule ($I_a$) dans lequel A représente un atome d'hydrogène, ou un sel de ce composé, puis soumet, si désiré, soit un composé de formule ($I_A$) dans lequel Y est un radical alcoyle renfermant de 1 à 18 atomes de carbone, soit un composé de formule ($I_A$) dans lequel Y représente un atome d'hydrogène, soit un dérivé fonctionnel de ce dernier, à l'action d'un alcool de formule $A_1OH$ ou d'un dérivé fonctionnel de cet alcool, formule dans laquelle $A_1$ a la même signification que A dans la revendication 1, à l'exception de l'hydrogène, pour obtenir le composé de formule ($I_a$) correspondant.

10. Variante du procédé selon la revendication 8 ou 9, caractérisé en ce que l'on soumet selon la réaction de Wittig le composé de formule (II) ou ($II_A$), telle que définie à la revendication 8 ou 9, sous l'une quelconque de ses formes isomères ou sous forme d'un mélange d'isomères, à l'action d'un composé de formule

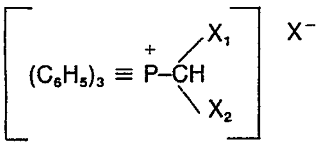

dans laquelle $X_1$, $X_2$ sont définis comme à la revendication 1 et $X^-$ représente un anion halogénure pour obtenir le composé de formule ($I_A$), puis, si désiré, poursuit la synthèse comme à la revendication 8 ou 9.

11. Utilisation des composés de formule (I) tels que définis à l'une quelconque des revendications 1 à 7, à la lutte contre les parasites des végétaux.

12. Les compositions destinées à la lutte contre les parasites des végétaux et des animaux à sang chaud, caractérisées en ce qu'elles renferment comme principe actif, au moins l'un des produits définis à l'une quelconque des revendications 1 à 7.

13. Les compositions insecticides selon la revendication 11, renfermant comme principe actif au moins l'un des produits définis à l'une quelconque des revendications 1 à 7.

14. Les compositions parfumantes renfermant comme principe actif au moins un produit de formule (I) sous toutes ses formes stéréoisomères possibles ainsi que sous forme de mélanges de stéréoisomères:

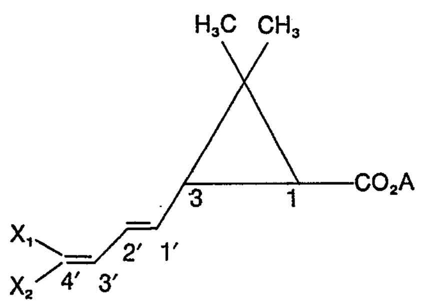

(I)

dans laquelle $X_1$ et $X_2$, identiques ou différents, représentent un atome d'halogène, et A représente,

– soit un atome d'hydrogène,

– soit un radical alcoyle renfermant de 1 à 18 atomes de carbone,

– soit un radical benzyle éventuellement substitué par un ou plusieurs radicaux choisis dans le groupe constitué par les radicaux alcoyles comportant de 1 à 4 atomes de carbone, les radicaux alcényles comportant de 2 à 6 atomes de carbone, les radicaux alcényloxy comportant de 2 à 6 atomes de carbone, les radicaux alcadiényles comportant de 4 à 8 atomes de carbone, le radical méthylène dioxyle, le radical benzyle et les atomes d'halogène,

– soit un groupement

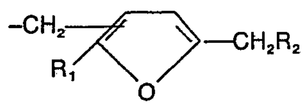

dans lequel le substituant $R_1$ représente un atome d'hydrogène ou un radical méthyle et le substituant $R_2$ un aryle monocyclique ou un groupement $-CH_2-C\equiv CH$ et notamment un groupement 3-benzyl 3-furyl méthyle,

– soit un groupement

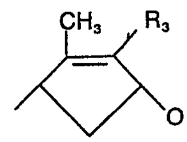

dans lequel $R_3$ représente un radical organique aliphatique comportant de 2 à 6 atomes de carbone et une ou plusieurs insaturations carbone-carbone et notamment le radical vinyle, propen-1-yle, buta-1,3-diényle ou buten-1-yle,

– soit un groupement

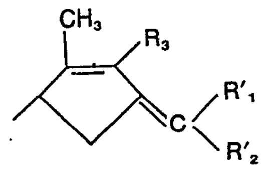

dans lequel $R_3$ conserve la même signification que précédemment, $R'_1$ et $R'_2$ identiques ou différents représentent un atome d'hydrogène, un atome d'halogène, un radical alcoyle renfermant de 1 à 6 atomes de carbone, un radical aryle comportant de 6 à 10 atomes de carbone, un groupement alcoyl-oxycarbonyle comportant de 2 à 5 atomes de carbone, ou un groupement cyano,

– soit un groupement

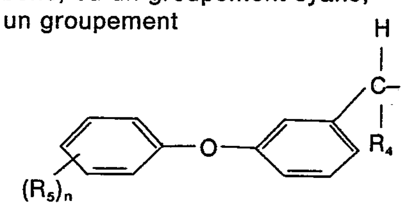

dans lequel $R_4$ représente un atome d'hydrogène, un groupement $-C\equiv N$, un radical méthyle, un radical $-CONH_2$, un radical $-CSNH_2$, ou un groupement $-C\equiv CH$, $R_5$ représente un atome de chlore ou un radical méthyle et n représente un nombre égal à 0, 1 ou 2, et notamment le groupement 3-phénoxybenzylique, α-cyano 3-phénoxybenzylique ou α-éthynyl 3-phénoxybenzylique,

– soit un groupement

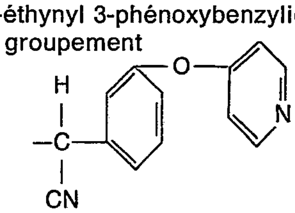

– soit un groupement

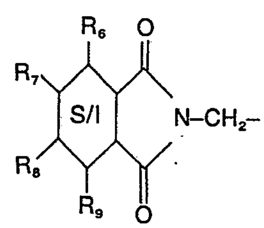

dans lequel les substituants $R_6$, $R_7$, $R_8$ et $R_9$ représentent un atome d'hydrogène, un atome de chlore ou un radical méthyle et dans lequel S/I symbolise un cycle aromatique ou un cycle analogue dihydro ou tétrahydro.

15. Les compositions parfumantes renfermant comme principe actif au moins un produit de formule (I) tel que défini à la revendication 5.

16. A titre de médicaments, les composés de formule (I) telle que définie à la revendication 14.

17. Les compositions pharmaceutiques renfermant comme principe actif au moins l'un des médicaments définis à la revendication 16.

18. Les compositions destinées à l'alimentation animale renfermant comme principe actif au moins l'un des produits définis de formule (I) telle que définie à la revendication 14, associé à un aliment destiné aux animaux.

**Claims for the contracting States, IT, LU, SE**

1. In all their possible stereo-isomeric forms or in the form of mixtures of stereo-isomers, the compounds with the formula (I):

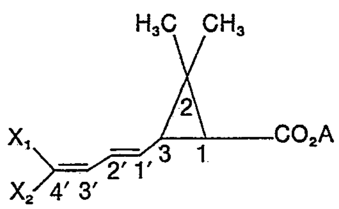

(I)

in which $X_1$ and $X_2$, identical or different, each represents a halogen atom, and A represents,
- either a hydrogen atom,
- or an alkyl radical containing from 1 to 18 carbon atoms,
- or a benzyl radical possibly substituted by one or more radicals chosen from the group constituted by the alkyl radicals containing from 1 to 4 carbon atoms, the alkenyl radicals containing from 2 to 6 carbon atoms, the alkenyloxy radicals containing from 2 to 6 carbon atoms, the alkadienyl radicals containing from 4 to 8 carbon atoms, the methylenedioxyl radical, the benzyl radical and the halogen atoms,
- or a group,

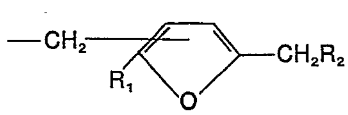

in which the substituent $R_1$ represents a hydrogen atom or a methyl radical and the substituent $R_2$ a monocyclic aryl or a group $-CH_2-C\equiv CH$ and in particular a 5-benzyl-3-furyl-methyl group,
- or a group,

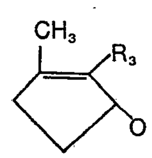

in which $R_3$ represents an aliphatic organic radical containing from 2 to 6 carbon atoms and one or more carbon-carbon unsaturations, and in particular the vinyl, propen-1-yl, buta-1,3-dienyl or buten-1-yl radical,
- or a group,

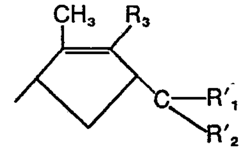

in which $R_3$ retains the same significance as previously, of $R'_1$ and $R'_2$, identical or different, each represents a hydrogen atom, a halogen atom, an alkyl radical containing from 1 to 6 carbon atoms, an aryl radical containing from 6 to 10 carbon atoms, an alkyloxycarbonyl group containing from 2 to 5 carbon atoms or a cyano group,
- or a group,

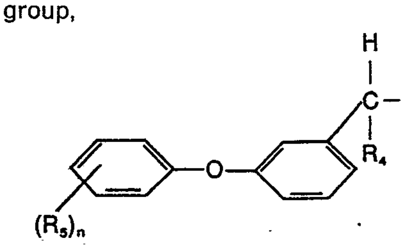

in which $R_4$ represents a hydrogen atom, a $C\equiv N$ group, a methyl radical, a $-CONH_2$ radical, a $-CSNH_2$ radical, or a $-C\equiv CH$ group, $R_5$ represents a chlorine atom or a methyl radical and n represents a numeral 0, 1 or 2, and in particular the 3-phenoxybenzyl group, the α-cyano-3-phenoxybenzyl group or the α-ethynyl-3-phenoxybenzyl group,
- or a group,

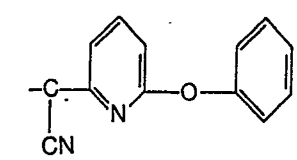

- or a group,

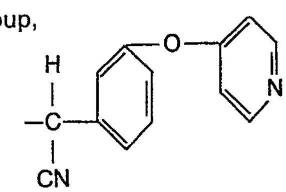

- or a group,

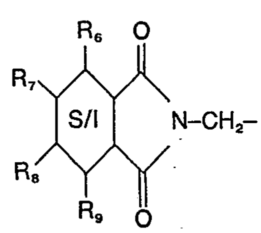

in which each of the substituents $R_6$, $R_7$, $R_8$ and $R_9$ represents a hydrogen atom, a chlorine atom or a methyl radical, and in which S/I symbolizes an aromatic ring or a similar dihydro or tetrahydro ring.

2. The compounds with the formula (I) as defined in Claim 1, for which the acid copula is of 1R cis or 1R trans structure.

3. The compounds with the formula (I) as defined in Claim 1 or 2 for which $X_1$ and $X_2$ are identical and each represents a bromine or a chlorine atom.

4. The compounds with the formula (I) as defined in any one of the Claims 1 to 3, for which A represents a hydrogen atom, as well as their salts.

5. The compounds with the formula (I) as defined in any one of the Claims 1 to 3, for which A represents an alkyl radical containing from 1 to 4 carbon atoms, and notably the methyl radical.

6. The compounds with the formula (I) as defined in any one of the Claims 1 to 3, for which A represents an α-cyano-3-phenoxybenzyl group.

7. The compounds with the formula (I) as defined in any one of the Claims 1 to 3, for which A represents a 3-allyl-2-methyl-4-oxocyclopent-2-en-1-yl group.

8. Preparation process for the compounds with the formula (I) as defined in Claim 1, characterized in that the compound with the formula (II):

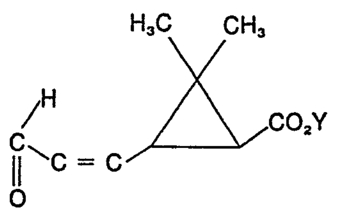

(II)

in which Y represents a hydrogen atom or an alkyl radical containing from 1 to 18 carbon atoms in any of its isomeric forms or in the form of a mixture of isomers, is submitted to the action of a compound with the formula (III):

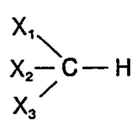

(III)

in which, either the substituents $X_1$, $X_2$ and $X_3$ are identical and each represents a chlorine or a bromine atom, or at least two of the substituents $X_1$, $X_2$ and $X_3$ are different, $X_1$ $X_2$ and $X_3$ then each representing a fluorine, chlorine or bromine atom, $X_1$, $X_2$ and $X_3$ being of equal atomic weight or of atomic weight increasing grom $X_1$ to $X_3$, so as to obtain the compound with the formula (IV):

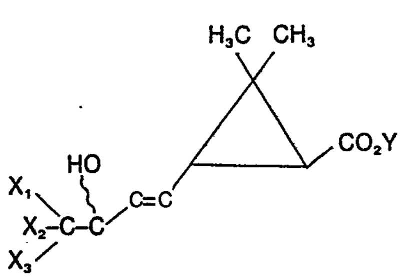

(IV)

of which the hydroxyl is blocked by the action of an acid or of a functional derivative of an acid $RCO_2H$, then the product obtained with the formula:

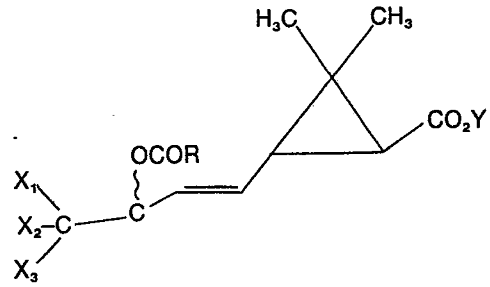

(V)

is submitted to the action of a reducing agent, so as to obtain, after elimination of the substituent $X_3$, which is the substituent or one of the substituents of highest atomic weight, the compound with the formula ($I_A$)

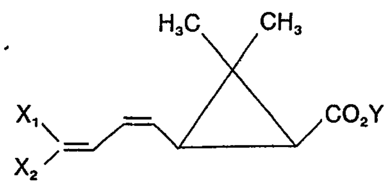

($I_A$)

in which $X_1$ and $X_2$ either are identical and each represents a chlorine or a bromine atom, or are different and each represents a fluorine, chlorine or bromine atom, then, if desired, a compound with the formula ($I_A$) in which Y represents an alkyl radical containing from 1 to 18 carbon atoms, is submitted to the action of a saponifying agent, so as to obtain a compound with the formula (I) in which A represents a hydrogen atom, or a salt of this compound, or else, if desired, either a compound with the formula ($I_A$) in which Y is an alkyl radical containing from 1 to 18 carbon atoms, or a compound with the formula ($I_A$) in which Y represents a hydrogen atom, or a functional derivative of this last, is submitted to the action of an alcohol with the formula $A_1OH$ or of a functional derivative of this alcohol, in which formula $A_1$ has the same significance as A in Claim 1, with the exception of hydrogen, so as to obtain the corresponding compound with the formula (I).

9. Variant of the process according to Claim 8, characterized in that the compound with the formula (II) as defined in Claim 8, in any one of its isomeric forms or in the form of a mixture of isomers, is submitted in accordance with the Wittig reaction to the action of a compound with the formula:

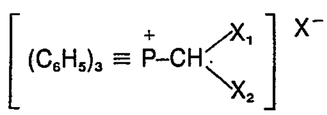

in which $X_1$ and $X_2$ are defined as in Claim 1 and $X^-$ represents a halide anion, so as to obtain the compound with the formula ($I_a$), then, if desired, the synthesis as in Claim 8 is continued.

10. Use of the compounds with the formula (I) as defined in any one of the Claims 1 to 7, for combatting parasites of vegetation.

11. Compositions intended for combatting the parasites of vegetation and of warm-blooded animals, characterized in that they contain, as active

principle, at least one of the products defined in any one of the Claims 1 to 7.

12. Insecticidal compositions according to Claim 11, containing as active principle at least one of the products defined in any one of the Claims 1 to 7.

13. Perfuming compositions containing as active principle at least one product with the formula (I) as defined in Claim 1.

14. Perfuming compositions containing as active principle at least one product with the formula (I) as defined in Claim 5.

15. As medicaments, the compounds defined in any one of the Claims 1 to 7.

16. Pharmaceutical compositions containing as active principle at least one of the medicaments defined in Claim 15.

17. Compositions intended for animal feeding containing as active principle at least one of the products defined in any one of the Claims 1 to 7, combined with a foodstuff intended for animals.

**Claims for the contracting State AT**

1. Process for the preparation in all their possible stereo-isomeric forms or in the form of mixtures of stereo-isomers, of the compounds with the formula (I):

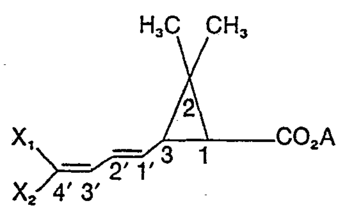

(I)

in which each of $X_1$ and $X_2$, identical or different, represents a halogen atom, and A represents,

– either a hydrogen atom,

– or an alkyl radical containing from 1 to 18 carbon atoms,

– or a benzyl radical possibly substituted by one or more radicals chosen from the group constituted by the alkyl radicals containing from 1 to 4 carbon atoms, the alkenyl radicals containing from 2 to 6 carbon atoms, the alkenyloxy radicals containing from 2 to 6 carbon atoms, the alkadienyl radicals containing from 4 to 8 carbon atoms, the methylenedioxyl radical, the benzyl radical and the halogen atoms,

– or a group

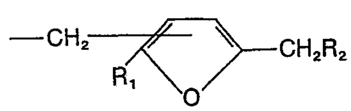

in which the substituent $R_1$ represents a hydrogen atom or a methyl radical and the substituent $R_2$ a monocyclic aryl or a $-CH_2-C\equiv CH$ group, and in particular, a 5-benzyl-3-furyl-methyl group,

– or a group

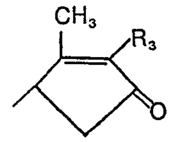

in which $R_3$ represents an aliphatic organic radical containing from 2 to 6 carbon atoms and one or more carbon-carbon unsaturations, and in particular the vinyl, propen-1-yl, buta-1,3-dienyl or buten-1-yl radical,

– or a group

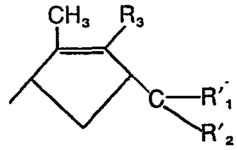

in which $R_3$ retains the same significance as previously, $R'_1$ and $R'_2$, identical or different, each represents a hydrogen atom, a halogen atom, an alkyl radical containing from 1 to 6 carbon atoms, an aryl radical containing from 6 to 10 carbon atoms, an alkyloxycarbonyl group containing from 2 to 5 carbon atoms, or a cyano group,

– or a group

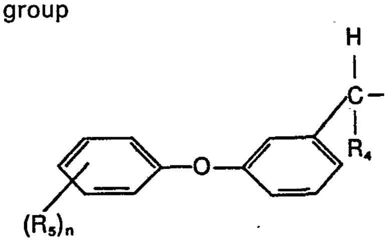

in which $R_4$ represents a hydrogen atom, a $-C\equiv N$ group, a methyl radical, a $-CONH_2$ radical, a $-CSNH_2$ radical, or a $-C\equiv CH$ group, $R_5$ represents a chlorine atom or a methyl radical and n represents a numeral, 0, 1 or 2, and in particular, the 3-phenoxybenzyl group, the α-cyano-3-phenoxybenzyl group or the α-ethynyl-3-phenoxybenzyl group,

– or a group

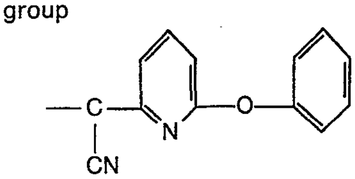

– or a group

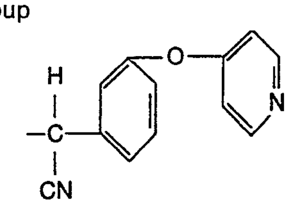

– or a group

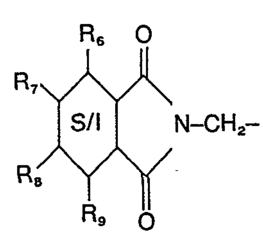

in which the substituents $R_6$, $R_7$, $R_8$ and $R_9$ each represent a hydrogen atom, a chlorine atom or a methyl radical and in which S/I symbolizes an aromatic ring or an analogous dihydro or tetrahydro ring, characterized in that a compound with the formula (II):

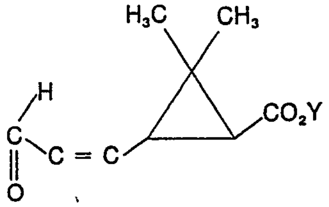

(II)

in which Y represents a hydrogen atom or an alkyl radical containing from 1 to 18 carbon atoms, in any one of its isomeric forms or in the form of a mixture of isomers, is submitted to the action of a compound with the formula (III):

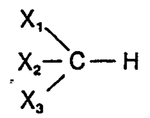

(III)

in which, either the substituents $X_1$, $X_2$ and $X_3$ are identical and each represents a chlorine or a bromine atom, or at least two of the substituents $X_1$, $X_2$ and $X_3$ are different, $X_1$, $X_2$ and $X_3$ then representing a fluorine, a chlorine or a bromine atom, $X_1$, $X_2$ and $X_3$ being of equal atomic weight or of atomic weight increasing from $X_1$ to $X_3$, so as to obtain the compound with the formula (IV):

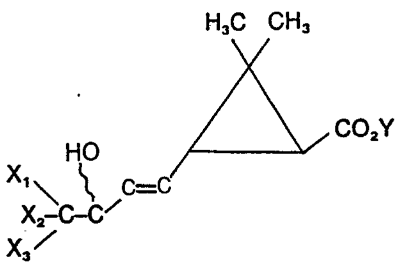

(IV)

of which the hydroxyl is blocked by the action of an acid or of a functional derivative of an acid $RCO_2H$, then the product obtained with the formula:

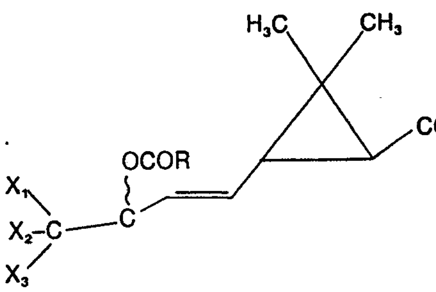

(V)

is submitted to the action of a reducing agent, so as to obtain after elimination of the substituent $X_3$, the substituent or one of the substituents with the highest atomic weight, the compound with the formula ($I_A$):

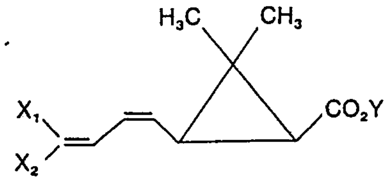

($I_A$)

in which $X_1$ and $X_2$ either are identical and each represents an atom of chlorine or of bromine, or are different and represent a fluorine, chlorine or a bromine atom, then, if desired, a compound with the formula ($I_A$), in which Y represents an alkyl radical containing from 1 to 18 carbon atoms is submitted to the action of a saponifying agent, so as to obtain a compound with the formula (I) in which A represents a hydrogen atom, or a salt of this compound, or, if desired, either a compound with the formula ($I_A$) in which Y is an alkyl radical containing from 1 to 18 carbon atoms, or a compound with the formula ($I_A$) in which Y represents a hydrogen atom, or a functional derivative of this latter, is submitted to the action of an alcohol with the formula $A_1OH$ or a functional derivative of this alcohol, in which formula $A_1$ has the same significance as A with the exception of hydrogen, so as to obtain the corresponding compound with the formula (I).

2. Variant of the process according to Claim 1, characterized in that the compound with the formula (II) as defined in Claim 1, in any of its isomeric forms or in the form of a mixture of isomers, is submitted according to the Wittig reaction to the action of a compound with the formula:

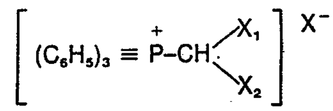

in which $X_1$, $X_2$ are defined as in Claim 1 and $X^-$ represents a halide anion, so as to obtain the compound with the formula ($I_A$), then, if desired, the synthesis as in Claim 1 is continued.

3. Process according to Claim 1 or 2, characterized in that a compound with the formula (II), of 1R cis or 1R trans structure is used at the start.

4. Process according to any of the Claims 1 to 3, characterized in that a compound with the formula (III) in which $X_1$ and $X_2$ are identical and both represent a bromine or a chlorine atom is used at the start.

5. Process according to any of the Claims 1 to 4, characterized in that a compound with the formula $A_1OH$ in which $A_1$ represents an alkyl radical containing from 1 to 4 carbon atoms and in particular a methyl radical is used at the start.

6. Process according to any of the Claims 1 to 4, characterized in that a compound with the formula $A_1OH$ in which $A_1$ represents an α-cyano-3-phenoxybenzyl group is used at the start.

7. Process according to any of the Claims 1 to 4, characterized in that a compound with the formula $A_1OH$ in which $A_1$ represents a 3-allyl-2-methyl-4-oxo-cyclopent-2-en-1-yl group is used at the start.

8. Use as pesticides of the compounds with the formula (I) as defined in any of the Claims 1 and 3 to 7.

9. Pesticidal compositions intended for combatting parasites, characterized in that they contain as active principle at least one of the products defined in any of the Claims 1 and 3 to 7.

10. Insecticidal compositions according to Claim 9, containing as active principle at least one of the products defined in any of the Claims 1 and 3 to 7.

11. Use as perfuming agents of the compounds with the formula (I), as defined in Claim 1.

12. Perfuming compositions containing as active principle at least one product with the formula (I) as defined in Claim 1.

13. Perfuming compositions containing as active principle at least one product with the formula (I) as defined in Claim 5.

**Claims for the contracting States: BE, CH, DE, FR, GB, LI, NL**

1. In all their possible stereo-isomeric forms or in the form of mixtures of stereo-isomers, the compounds with the formula (I):

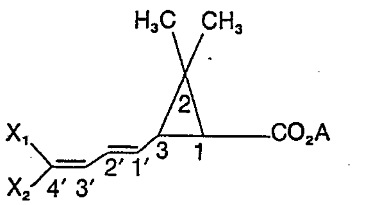

(I)

in which $X_1$ and $X_2$, identical or different, each represents a halogen atom, and A represents,
- either a benzyl radical possibly substituted by one or more radicals chosen from the group constituted by the alkyl radicals containing from 1 to 4 carbon atoms, the alkenyl radicals containing from 2 to 6 carbon atoms, the alkenyloxy radicals containing from 2 to 6 carbon atoms, the alkadienyl radicals containing from 4 to 8 carbon atoms, the methylenedioxyl radical, the benzyl radical and the halogen atoms,
- or a group

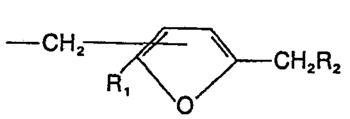

in which the substituent $R_1$ represents a hydrogen atom or a methyl radical and the substituent $R_2$ represents a monocyclic aryl radical or a group $-CH_2-C\equiv CH$ and particularly a 5-benzyl-3-furylmethyl group,
- or a group

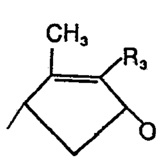

in which $R_3$ represents an aliphatic organic radical containing from 2 to 8 carbon atoms and one or more carbon-carbon unsaturations and in particular the vinyl, propen-1-yl, buta-1,3-dienyl or buten-1-yl radical,
- or a group

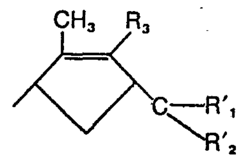

in which $R_3$ retains the same significance as previously, each of $R'_1$ and $R'_2$, identical or different, represents a hydrogen atom, a halogen atom, an alkyl radical containing from 1 to 6 carbon atoms, an aryl radical containing from 6 to 10 carbon atoms, an alkyloxycarbonyl group containing from 2 to 5 carbon atoms, or a cyano group,
- or a group

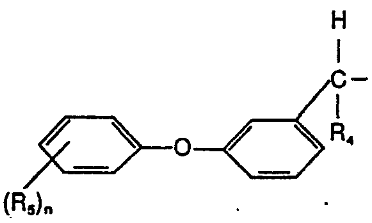

in which $R_4$ represents a hydrogen atom, a $-C\equiv N$ group, a methyl radical, a $-CONH_2$ radical, a $-CSNH_2$ radical, or a $-C\equiv CH$ group, $R_5$ represents a chlorine atom or a methyl radical, and n represents a numeral 0, 1 or 2, on condition that when n = 0, $R_4$ represents neither a hydrogen atom nor a $C\equiv N$ group, nor a $C\equiv CH$ group,
- or a group

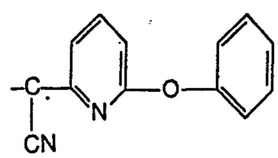

- or a group

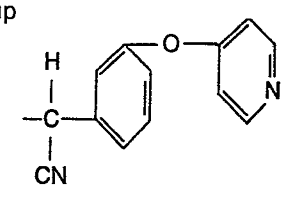

- or a group

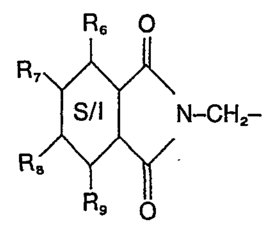

in which each of the substituents $R_6$, $R_7$, $R_8$ and $R_9$ represents a hydrogen atom, a chlorine atom or a methyl radical and in which S/I symbolizes an

aromatic ring or a similar dihydro or tetrahydro ring.

2. In all their possible stereo-isomeric forms or in the form of mixtures of stereo-isomers, the compounds with the formula ($I_a$):

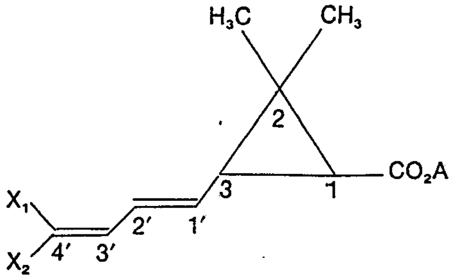

($I_a$)

in which the cyclopropanic acid copula is of 1R cis or 1R trans structure and in which each of $X_1$ and $X_2$, identical or different, represents a halogen atom, and A represents,

- either a hydrogen atom,
- or an alkyl radical containing from 1 to 18 carbon atoms,
- or a benzyl radical possibly substituted by one or more radicals chosen from the group constituted by the alkyl radicals containing from 1 to 4 carbon atoms, the alkenyl radicals containing from 2 to 6 carbon atoms, the alkenyloxy radicals containing from 2 to 6 carbon atoms, the alkadienyl radicals containing from 4 to 8 carbon atoms, the methylenedioxyl radical, the benzyl radical and the halogen atoms,
- or a group

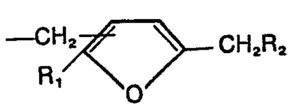

in which the substituent $R_1$ represents a hydrogen atom or a methyl radical and the substituent $R_2$ a monocyclic aryl or a group $-CH_2-CH{\equiv}CH$ and in particular a 5-benzyl-3-furyl-methyl group,

- or a group

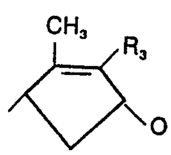

in which $R_3$ represents an aliphatic organic radical containing from 2 to 6 carbon atoms and one or more carbon-carbon unsaturations, and in particular the vinyl, propen-1-yl, buta-1,3-dienyl or buten-1-yl radical,

- or a group

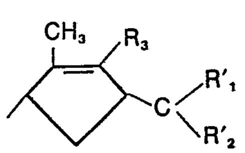

in which $R_3$ retains the same significance as previously, $R'_1$ and $R'_2$, identical or different, each represents a hydrogen atom, a halogen atom, an alkyl radical containing from 1 to 6 carbon atoms, an aryl radical containing from 6 to 10 carbon atoms, an alkyloxycarbonyl group containing from 2 to 5 carbon atoms, or a cyano group,

- or a group

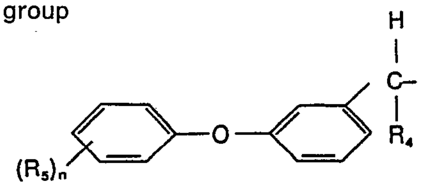

in which $R_4$ represents a hydrogen atom, a $-C{\equiv}N$ group, a methyl radical, a $-CONH_2$ radical, a $-CSNH_2$ radical, or a $-C{\equiv}CH$ group, $R_5$ represents a chlorine atom or a methyl radical and n represents a numeral 0, 1 or 2, and particularly the 3-phenoxybenzyl group, the α-cyano-3-phenoxybenzyl group or the α-ethynyl-3-phenoxybenzyl group,

- or a group

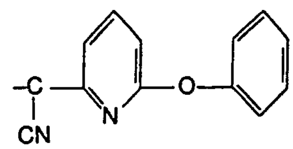

- or a group

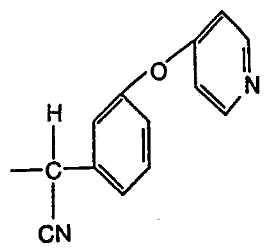

- or a group

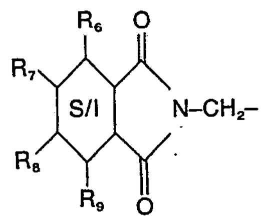

in which each substituent $R_6$, $R_7$, $R_8$ and $R_9$ represents a hydrogen atom, a chlorine atom or a methyl radical, and in which S/I symbolizes an aromatic ring or a similar dihydro or tetrahydro ring.

3. The compounds with the formula (I) and ($I_a$) as defined in Claim 1 or 2, for which $X_1$ and $X_2$ are identical and both represent a bromine or a chlorine atom.

4. The compounds with the formula ($I_a$) as defined in Claim 2 or 3, for which A represents a hydrogen atom, as well as their salts.

5. The compounds with the formula ($I_a$) as defined in Claim 2 or 3, for which A represents an alkyl radical containing from 1 to 4 carbon atoms and in particular the methyl radical.

6. The compounds with the formula ($I_a$) as defined in either of the Claims 2 or 3, for which A represents an α-cyano-3-phenoxybenzyl group.

7. The compounds with the formula (I) as defined in any one of the Claims 1, 2 or 3, for which A represents a 3-allyl-2-methyl-4-oxo-cyclopent-2-en-1-yl group.

8. Preparation process for the compounds with the formula (I) as defined in Claim 1, characterized in that a compound with the formula (II):

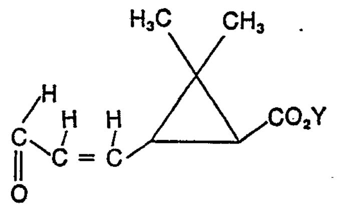

(II)

in which Y represents a hydrogen atom or an alkyl radical containing from 1 to 18 carbon atoms, in any one of its isomeric forms or in the form of a mixture of isomers, is submitted to the action of a compound with the formula (III):

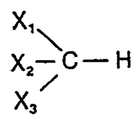

(III)

in which, either the substituents $X_1$, $X_2$ and $X_3$ are indentical and each represents a chlorine or a bromine atom, or at least two of the substituents $X_1$, $X_2$ and $X_3$ are different, $X_1$, $X_2$ and $X_3$ then each representing a fluorine, chlorine or bromine atom, $X_1$, $X_2$ and $X_3$ being of equal atomic weight or of atomic weight increasing from $X_1$ to $X_3$, so as to obtain the compound with the formula (IV):

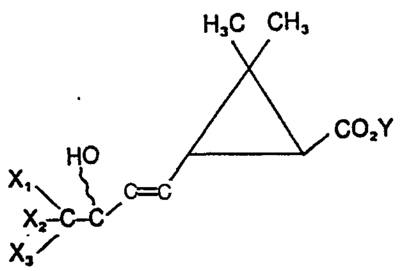

(IV)

of which the hydroxyl is blocked by the action of an acid or of a functional derivative of an acid $RCO_2H$, then the product obtained with the formula:

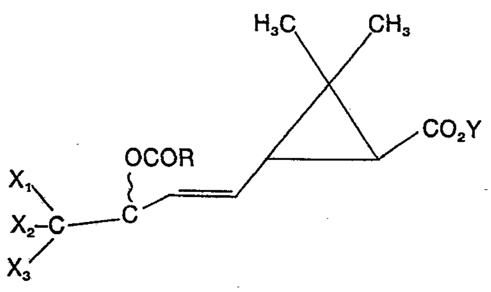

(V)

is submitted to the action of a reducing agent, so as to obtain, after elimination of the substituent $X_3$, which is the substituent or one of the substituentes of highest atomic weight, the compound with the formula ($I_A$):

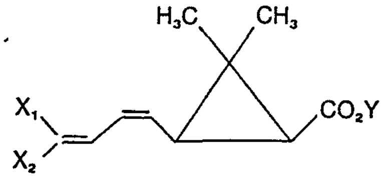

($I_A$)

in which $X_1$ and $X_2$ either are identical and each represents a chlorine or a bromine atom, or are different and each represents a fluorine, chlorine or bromine atoms, then a compound with the formula ($I_A$), in which Y represents an alkyl radical containing from 1 to 18 carbon atoms, is submitted to the action of a saponifying agent, so as to obtain a compound with the formula (I) in which A represents a hydrogen atom, or a salt of this compound, or else, either a compound with the formula ($I_A$) in which Y is an alkyl radical containing from 1 to 18 carbon atoms, or a compound with the formula ($I_A$) in which Y represents a hydrogen atom, or a functional derivative of this latter, is submitted to the action of an alcohol with the formula AOH, or of a functional derivative of this alcohol, in which formula A has the same significance as in Claim 1, so as to obtain the corresponding compound with the formula (I).

9. Preparation process for the compounds with the formula ($I_a$) as defined in Claim 2, characterized in that a compound with the formula ($II_A$):

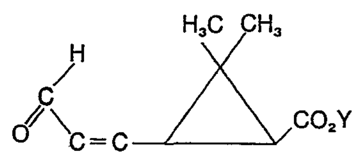

($II_A$)

in which Y represents a hydrogen atom or an alkyl radical containing from 1 to 18 carbon atoms, in any of its isomeric forms or in the form of a mixture of isomers, is submitted to the action of a compound with the formula (III):

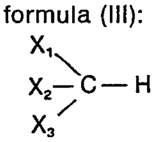

(III)

in which, either the substituents $X_1$, $X_2$ and $X_3$ are identical, each representing a chlorine or a bromine atom, or at least two of the substituents $X_1$, $X_2$ and $X_3$ are different, each of $X_1$, $X_2$ and $X_3$ then representing a fluorine, chlorine or bromine atom, $X_1$, $X_2$ and $X_3$ being of equal atomic weight or of atomic weight increasing from $X_1$ to $X_3$, so as to obtain the compound with the formula ($IV_A$):

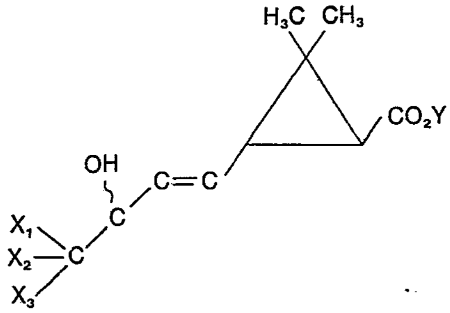

($IV_A$)

of which the hydroxyl is blocked by the action of an acid or of a functional derivative of an acid $RCO_2H$, then the product obtained with the formula:

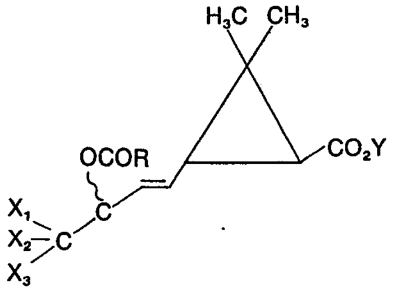

$(V_A)$

is submitted to the action of a reducing agent, so as to obtain, after elimination of the substituent $X_3$, which is the substituent or one of the substituents with the highest atomic weight, the compound with the formula $(I_A)$:

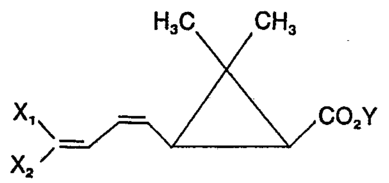

$(I_A)$

in which $X_1$ and $X_2$ either are identical and each represents a chlorine or a bromine atom, or are different and each represents a fluorine, chlorine or a bromine atom, then, if desired, a compound with the formula $I_A$, in which Y represents an alkyl radical containing from 1 to 18 carbon atoms, is submitted to the action of a saponifying agent, so as to obtain a compound with the formula $(I_a)$ in which A represents a hydrogen atom, or a salt of this compound, then if desired, either a compound with the formula $(I_A)$ in which Y is an alkyl radical containing from 1 to 18 carbon atoms, or a compound with the formula $(I_A)$ in which Y represents a hydrogen atom, or a functional derivative of this latter, is submitted to the action of an alcohol with the formula $A_1OH$ or of a functional derivative of this alcohol, in which formula $A_1$ has the same significance as A in Claim 1, with the exception of hydrogen, so as to obtain the corresponding compound with the formula (I).

10. Variant of the process according to Claim 8 or 9, characterized in that a compound with the formula (II) or $(II_A)$, as defined in Claim 8 or 9, in any one of its isomeric forms or in the form of a mixture of isomers, is submitted according to the Wittig reaction to the action of a compound with the formula:

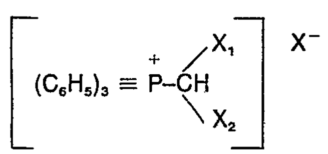

in which $X_1$ and $X_2$ are defined as in Claim 1, and $X^-$ represents a halide anion, so as to obtain the compound with the formula $(I_A)$, and then, if desired, the synthesis is continued as in Claim 8 or 9.

11. Utilization of the compounds with the formula (I) as defined in any one of the Claims 1 to 7 for combatting parasites of vegetation.

12. Compositions intended for combatting parasites of vegetation and warm-blooded animals, characterized in that they contain as active principle, at least one of the products defined in any one of the Claims 1 to 7.

13. Inseticidal compositions according to Claim 11, containing as active principle at least one of the products defined in any one of Claims 1 to 7.

14. Perfuming compositions containing as active principle at least one product with the formula (I), in any of its possible stereo-isomeric forms as well as in the form of mixtures of stereo-isomers:

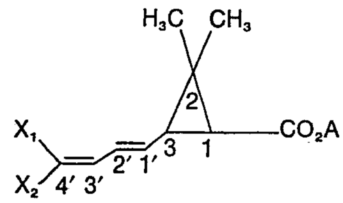

(I)

in which $X_1$ and $X_2$, identical or different, represent halogen atoms, and A represents,

- either a hydrogen atom,
- or an alkyl radical containing from 1 to 18 carbon atoms,
- or a benzyl radical possibly substituted by one or more radicals chosen from the group constituted by the alkyl radicals containing from 1 to 4 carbon atoms, the alkenyl radicals containing from 2 to 6 carbon atoms, the alkenyloxy radicals containing from 2 to 6 carbon atoms, the alkadienyl radicals containing from 4 to 8 carbon atoms, the methylene dioxyl radical, the benzyl radical and the halogen atoms,
- or a group

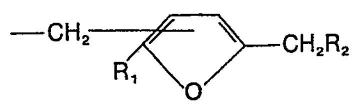

in which the substituent $R_1$ represents a hydrogen atom or a methyl radical and the substituent $R_2$ a monocyclic aryl or a $-CH_2-CH \equiv CH$ group, and in particular a 5-benzyl-3-furyl-methyl group,

- or a group

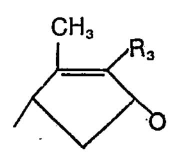

in which $R_3$ represents an aliphatic organic radical containing from 2 to 6 carbon atoms and one or more carbon-carbon unsaturations, and in particular, the vinyl, propen-1-yl, buta-1,3-dienyl or the buten-1-yl radical,

- or a group

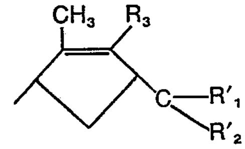

in which $R_3$ retains the same significance as previously, $R'_1$ and $R'_2$, identical or different, each represents a hydrogen atom, a halogen atom, an alkyl radical containing from 1 to 6 carbon atoms, an aryl radical containing from 6 to 10 carbon atoms, an alkyloxycarbonyl group containing from 2 to 5 carbon atoms, or a cyano group,

– or a group

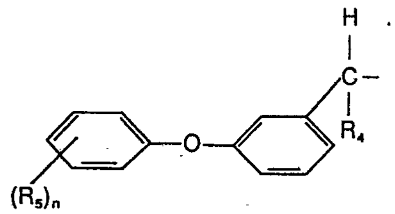

in which $R_4$ represents a hydrogen atom, a $-C\equiv N$ group, a methyl radical, a $-CONH_2$ radical, a $-CSNH_2$ radical, or a $-C\equiv CH$ troup, $R_5$ represents a chlorine atom or a methyl radical and n represents a numeral 0, 1 or 2, and in particular, the 3-phenoxybenzyl group, the α-cyano-3-phenoxybenzyl group or the α-ethynyl-3-phenoxybenzyl group,

– or a group

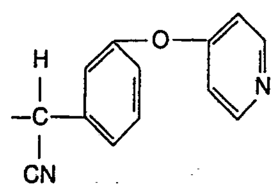

– or a group

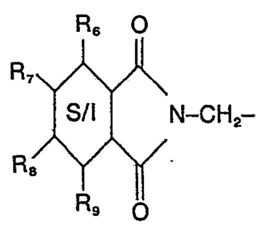

in which each substituent $R_6$, $R_7$, $R_8$ and $R_9$ represents a hydrogen atom, a chlorine atom or a methyl radical, and in which S/I symbolizes an aromatic ring or an analogous dihydro or tetrahydro ring.

15. The perfuming compositions containing as active principle at least one product with the formula (I) as defined in Claim 5.

16. As medicaments, the compounds with the formula (I) as defined in Claim 14.

17. The pharmaceutical compositions containing as active principle at least one of the medicaments defined in Claim 16.

18. Compositions intended for animal feeding containing as active principle at least one of the products defined by formula (I) as defined in Claim 14, combined with a food-stuff intended for animals.

**Patentansprüche für die Vertragsstaaten: IT, LU, SE**

1. In sämtlichen ihrer möglichen stereoisomeren Formen oder in Form von Gemischen der Stereoisomeren die Verbindungen der Formel (I):

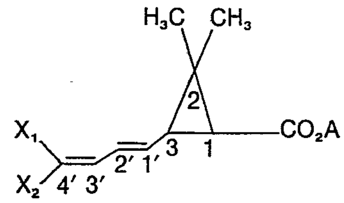

(I)

worin $X_1$ und $X_2$ gleich oder verschieden sind und ein Halogenatom bedeuten und A bedeutet

– entweder ein Wasserstoffatom

– oder einen Alkylrest mit 1 bis 18 Kohlenstoffatomen

– oder einen Benzylrest, gegebenenfalls substituiert durch einen oder mehrere Reste, ausgewählt unter den Alkylresten mit 1 bis 4 Kohlenstoffatomen, den Alkenylresten mit 2 bis 6 Kohlenstoffatomen, den Alkenyloxyresten mit 2 bis 6 Kohlenstoffatomen, den Alkadienylresten mit 4 bis 8 Kohlenstoffatomen, dem Methylendioxylrest, dem Benzylrest und den Halogenatomen,

– oder eine Gruppe

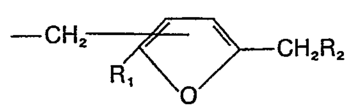

worin der Substituent $R_1$ ein Wasserstoffatom oder einen Methylrest bedeutet und der Substituent $R_2$ einen monocyclischen Arylrest oder eine Gruppe $-CH_2-C\equiv CH$ bedeutet, und insbesondere eine 5-Benzyl-3-furylmethyl-Gruppe,

– oder eine Gruppe

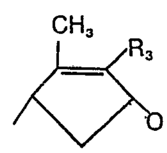

worin $R_3$ einen aliphatischen, organischen Rest mit 2 bis 6 Kohlenstoffatomen und einer oder mehreren Kohlenstoff-Kohlenstoff-Unsättigungen und insbesondere den Vinyl-, Propen-1-yl, Buta-1,3-dienyl- oder Buten-1-yl-Rest bedeutet,

– oder eine Gruppe

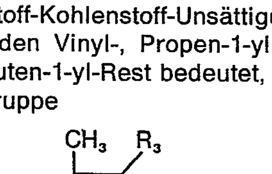

worin $R_3$ die vorstehend angegebene Bedeutung besitzt, $R'_1$ und $R'_2$, die gleich oder verschieden sind, ein Wasserstoffatom, ein Halogenatom, einen Alkylrest mit 1 bis 6 Kohlenstoffatomen, einen Arylrest mit 6 bis 10 Kohlenstoffatomen, eine Alkoxycarbonylgruppe mit 2 bis 5 Kohlenstoffatomen oder eine Cyanogruppe bedeutet,

– oder eine Gruppe

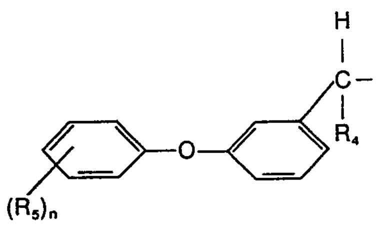

worin $R_4$ ein Wasserstoffatom, eine $-C\equiv N$-Gruppe, einen Methylrest, eine Gruppe $-CONH_2$, eine Gruppe $-CSNH_2$ oder eine Gruppe $-C\equiv CH$ bedeutet, $R_5$ ein Chloratom oder einen Methylrest bedeutet und n eine ganze Zahl entsprechend 0, 1 oder 2 bedeutet, und insbesondere die 3-Phenoxybenzyl-, α-Cyano-3-phenoxybenzyl- oder α-Ethinyl-3-phenoxybenzylgruppe,

– oder eine Gruppe

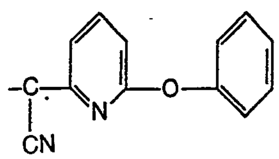

– oder eine Gruppe

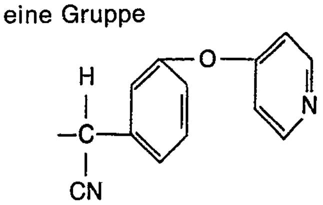

– oder eine Gruppe

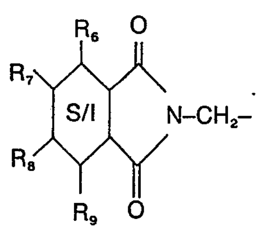

worin die Substituenten $R_5$, $R_7$, $R_8$ und $R_9$ ein Wasserstoffatom, ein Chloratom oder einen Methylrest bedeuten, und worin S/I einen aromatischen Ring oder einen analogen Dihydro- oder Tetrahydro-Ring symbolisiert.

2. Verbindungen der Formel (I) gemäss Anspruch 1, worin die Säureverknüpfungskomponente 1R,cis- oder 1R,trans-Struktur besitzt.

3. Verbindungen der Formel (I) gemäss Anspruch 1 oder 2, worin $X_1$ und $X_2$ identisch sind und alle beide ein Brom- oder Chloratom bedeuten.

4. Verbindungen der Formel (I) gemäss einem der Ansprüche 1 bis 3, worin A ein Wasserstoffatom bedeutet, sowie deren Salze.

5. Verbindungen der Formel (I) gemäss einem der Ansprüche 1 bis 3, worin A einen Alkylrest mit 1 bis 4 Kohlenstoffatomen und insbesondere den Methylrest bedeutet.

6. Verbindungen der Firmel (I) gemäss einem der Ansprüche 1 bis 3, worin A eine α-Cyano-3-phenoxybenzylgruppe bedeutet.

7. Verbindungen der Formel (I) gemäss einem der Ansprüche 1 bis 3, worin A eine 3-Allyl-2-methyl-4-oxo-cyclopent-2-en-1-yl-Gruppe bedeutet.

8. Verfahren zur Herstellung der Verbindungen der Formel (I) gemäss Anspruch 1, dadurch gekennzeichnet, dass man die Verbindung der Formel (II)

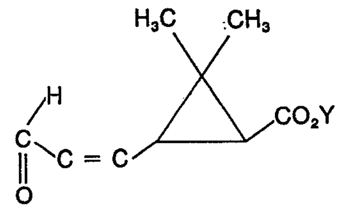

(II)

worin Y ein Wasserstoffatom oder einen Alkylrest mit 1 bis 18 Kohlenstoffatomen bedeutet, in irgendeiner ihrer isomeren Formen oder in Form eines Gemisches der Isomeren der Einwirkung einer Verbindung der Formel (III)

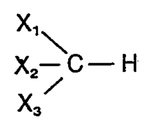

(III)

unterzieht, worin entweder die Substituenten $X_1$, $X_2$ und $X_3$ identisch sind und ein Chlor- oder Bromatom bedeuten oder zumindest zwei der Substituenten $X_1$, $X_2$ und $X_3$ verschieden sind, wobei $X_1$, $X_2$ und $X_3$ dann ein Fluor-, Chlor- oder Bromatom bedeuten, wobei $X_1$, $X_2$ und $X_3$ Atomgewichte aufweisen, die gleich sind oder von $X_1$ nach $X_3$ zunehmen, um die Verbindung der Formel (IV)

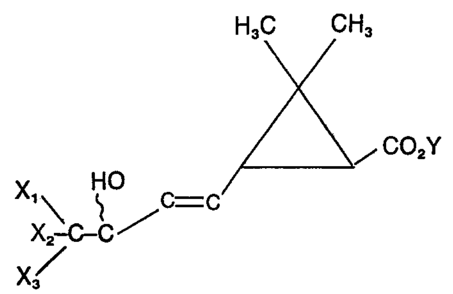

(IV)

zu erhalten, deren Hydroxylgruppe man durch Einwirken einer Säure oder eines funktionellen Derivats einer Säure $RCO_2H$ blockiert, anschliessend das erhaltene Produkt der Formel

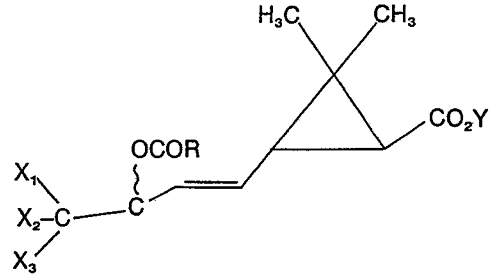

(V)

der Einwirkung eines Reduktionsmittels unterzieht, um nach Eliminierung des Substituenten $X_3$, der der Substituent oder einer der Substituenten mit höchstem Atomgewicht ist, die Verbindung der Formel ($I_A$)

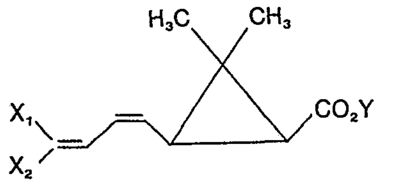

($I_A$)

zu erhalten, worin $X_1$ und $X_2$ entweder identisch sind und ein Chlor- oder Bromatom bedeuten oder verschieden sind und ein Fluor-, Chlor- oder Bromatom bedeuten, anschliessend gewünschtenfalls eine Verbindung der Formel $I_A$, worin Y einen Alkylrest mit 1 bis 18 Kohlenstoffatomen bedeutet, der Einwirkung eines Verseifungsmittels unterzieht, um eine Verbindung der Formel (I), worin A ein Wasserstoffatom bedeutet, oder ein Salz dieser Verbindung zu erhalten, oder gewünschtenfalls entweder eine Verbindung der Formel ($I_A$), worin Y einen Alkylrest mit 1 bis 18 Kohlenstoffatomen darstellt, oder eine Verbindung der Formel ($I_A$), worin Y ein Wasserstoffatom bedeutet, oder ein funktionelles Derivat dieser letzteren der Einwirkung eines Alkohols der Formel $A_1OH$ oder eines funktionellen Derivats dieses Alkohols unterzieht, wobei in der Formel $A_1$ die gleiche Bedeutung wie A in Patentanspruch 1 mit Ausnahme von Wasserstoff besitzt, um die entsprechende Verbindung der Formel (I) zu erhalten.

9. Variante des Verfahrens gemäss Anspruch 8, dadurch gekennzeichnet, dass man nach der Wittig-Reaktion die Verbindung der Formel (II) gemäss Anspruch 8 in irgendeiner ihrer isomeren Formen oder in Form eines Gemisches der Isomeren der Einwirkung einer Verbindung der Formel

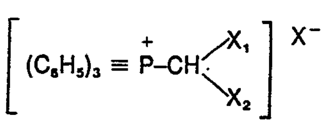

unterzieht, worin $X_1$ und $X_2$ die in Anspruch 1 angegebene Bedeutung besitzen und $X^-$ ein Halogenidanion bedeutet, um die Verbindung der Formel ($I_A$) zu erhalten, und danach gewünschtenfalls die Synthese wie in Anspruch 8 fortsetzt.

10. Verwendung der Verbindungen der Formel (I) gemäss einem der Ansprüche 1 bis 7 zur Bekämpfung von Parasiten der Pflanzen.

11. Zusammensetzungen für die Bekämpfung von Parasiten der Pflanzen und warmblütigen Tiere, dadurch gekennzeichnet, dass sie als Wirkstoff zumindest eines der Produkte gemäss einem der Ansprüche 1 bis 7 enthalten.

12. Insektizide Zusammensetzungen gemäss Anspruch 11, enthaltend als Wirkstoff zumindest eines der Produkte gemäss einem der Ansprüche 1 bis 7.

13. Duftverleihende Zusammensetzungen, enthaltend als Wirkstoff zumindest ein Produkt der Formel (I) gemäss Anspruch 1.

14. Duftverleihende Zusammensetzungen, enthaltend als Wirkstoff zumindest ein Produkt der Formel (I) gemäss Anspruch 5.

15. Als Arzneimittel die Verbindungen gemäss einem der Ansprüche 1 bis 7.

16. Pharmazeutische Zusammensetzungen, enthaltend als Wirkstoff zumindest eines der Arzneimittel gemäss Anspruch 15.

17. Zusammensetzungen für die tierische Ernährung, enthaltend als Wirkstoff zumindest eines der Produkte gemäss einem der Ansprüche 1 bis 7 gemeinsam mit einem für die tierische Ernährung bestimmten Nahrungsmittel.

**Patentansprüche für den Vertragsstaat: AT**

1. Verfahren zur Herstellung in sämtlichen ihrer möglichen stereoisomeren Formen oder in Form von Gemischen der Stereoisomeren der Verbindungen der Formel (I)

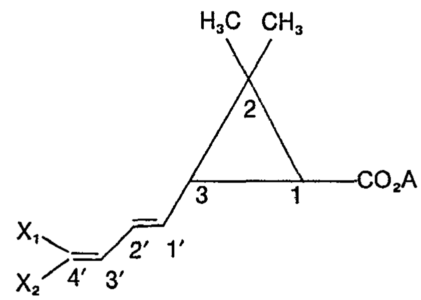

(I)

worin $X_1$ und $X_2$ gleich oder verschieden sind und ein Halogenatom bedeuten und A bedeutet
– entweder ein Wasserstoffatom
– oder einen Alkylrest mit 1 bis 18 Kohlenstoffatomen
– oder einen Benzylrest, gegebenenfalls substituiert durch einen oder mehrere Reste, ausgewählt unter den Alkylresten mit 1 bis 4 Kohlenstoffatomen, den Alkenylresten mit 2 bis 6 Kohlenstoffatomen, den Alkenyloxyresten mit 2 bis 6 Kohlenstoffatomen, den Alkadienylresten mit 4 bis 8 Kohlenstoffatomen, dem Methylendioxylrest, dem Benzylrest und den Halogenatomen,
– oder eine Gruppe

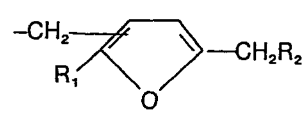

worin der Substituent $R_1$ ein Wasserstoffatom oder einen Methylrest bedeutet und der Substituent $R_2$ einen monocyclischen Arylrest oder eine Gruppe $-CH_2C{\equiv}CH$ bedeutet und insbesondere eine 5-Benzyl-3-furylmethylgruppe,
– oder eine Gruppe

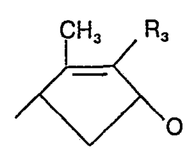

worin $R_3$ einen aliphatischen, organischen Rest mit 2 bis 6 Kohlenstoffatomen und einer oder mehreren Kohlenstoff-Kohlenstoff-Unsättigungen und insbesondere den Vinyl-, Propen-1-yl-, Buta-1,3-dienyl- oder Buten-1-yl-Rest bedeutet,
– oder eine Gruppe

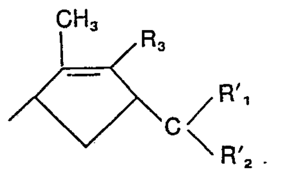

worin $R_3$ die vorstehend angegebene Bedeutung besitzt, $R'_1$ und $R'_2$ gleich oder verschieden sind und ein Wasserstoffatom, ein Halogenatom, einen Alkylrest mit 1 bis 6 Kohlenstoffatomen, einen Arylrest mit 6 bis 10 Kohlenstoffatomen, eine Alkoxycarbonylgruppe mit 2 bis 5 Kohlenstoffatomen oder eine Cyanogruppe bedeuten,

– oder eine Gruppe

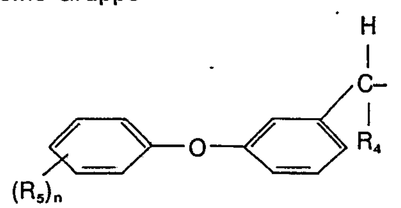

worin $R_4$ ein Wasserstoffatom, eine $-C\equiv N$-Gruppe, einen Methylrest, eine Gruppe $-CONH_2$, eine Gruppe $-CSNH_2$ oder eine Gruppe $-C\equiv CH$ bedeutet, $R_5$ ein Chloratom oder einen Methylrest bedeutet und n eine Zahl entsprechend 0, 1 oder 2 darstellt, und insbesondere die 3-Phenoxy-benzyl-, α-Cyano-3-phenoxybenzyl- oder α-Ethinyl-3-phenoxybenzyl-Gruppe,

– oder eine Gruppe

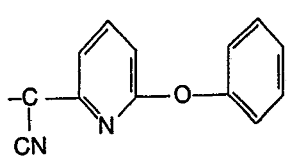

– oder eine Gruppe

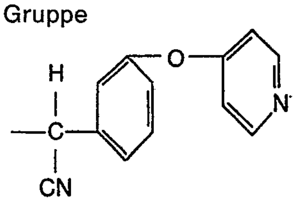

– oder eine Gruppe

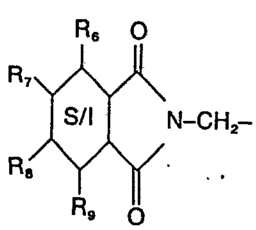

worin die Substituenten $R_6$, $R_7$, $R_8$ und $R_9$ ein Wasserstoffatom, ein Chloratom oder einen Methylrest bedeuten und worin S/I einen aromatischen Ring oder einen analogen Dihydro- oder Tetrahydro-Ring symbolisiert, dadurch gekennzeichnet, dass man die Verbindung der Formel (II)

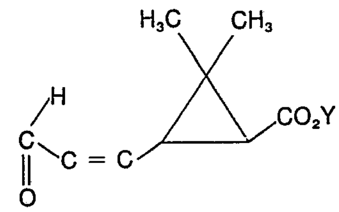

(II)

worin Y ein Wasserstoffatom oder einen Alkylrest mit 1 bis 18 Kohlenstoffatomen bedeutet, in irgendeiner ihrer isomeren Formen oder in Form eines Gemisches der Isomeren der Einwirkung einer Verbindung der Formel (III)

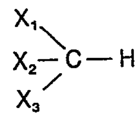

(III)

unterzieht, worin entweder die Substituenten $X_1$, $X_2$ und $X_3$ identisch sind und ein Chlor- oder Bromatom bedeuten oder zumindest zwei der Substituenten $X_1$, $X_2$ und $X_3$ verschieden sind, wobei $X_1$, $X_2$ und $X_3$ dann ein Fluor-, Chlor- oder Bromatom bedeuten, wobei $X_1$, $X_2$ und $X_3$ gleiche Atomgewichte oder von $X_1$ bis $X_3$ zunehmende Atomgewichte aufweisen, um die Verbindung der Formel (IV)

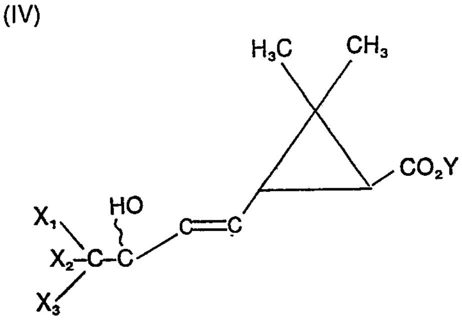

(IV)

zu erhalten, deren Hydroxylgruppe man durch Einwirken einer Säure oder eines funktionellen Derivats einer Säure $RCO_2H$ blockiert, anschliessend die erhaltene Verbindung der Formel

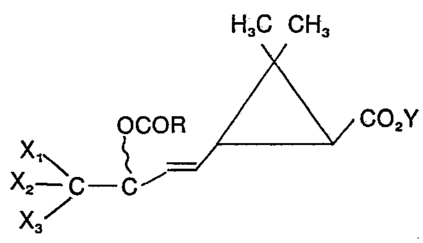

(V)

der Einwirkung eines Reduktionsmittels unterzieht, um nach Eliminierung des Substituenten $X_3$, der der Substituent oder einer der Substituenten mit dem höchsten Atomgewicht ist, die Verbindung der Formel ($I_A$)

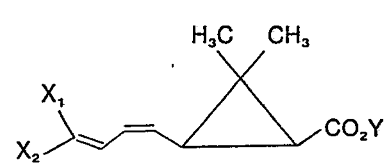

($I_A$)

zu erhalten, worin $X_1$ und $X_2$ entweder identisch sind und ein Chlor- oder Bromatom bedeuten oder verschieden sind und ein Fluor-, Chlor- oder Bromatom bedeuten, danach gewünschtenfalls eine Verbindung der Formel $I_A$, worin Y einen Alkylrest mit 1 bis 18 Kohlenstoffatomen bedeutet, der Einwirkung eines Verseifungsmittels unterzieht, um eine Verbindung der Formel (I), worin A ein Wasserstoffatom bedeutet, oder ein Salz dieser Verbindung zu erhalten, oder gewünschtenfalls entweder eine Verbindung der Formel ($I_A$), worin Y einen Alkylrest mit 1 bis 18 Kohlenstoffatomen bedeutet, oder eine Verbindung der Formel ($I_A$), worin Y ein Wasserstoffatom bedeutet, oder ein funktionelles Derivat dieser letzteren der Einwirkung eines Alkohols der Formel $A_1OH$ oder eines funktionellen Derivats dieses Alkohols unterzieht, wobei in der Formel $A_1$ die gleiche Bedeutung wie A mit Ausnahme von Wasserstoff besitzt, um die entsprechende Verbindung der Formel (I) zu erhalten.

2. Variante des Verfahrens gemäss Anspruch 1, dadurch gekennzeichnet, dass gemäss der Wittig-Reaktion die Verbindung der Formel (II) gemäss Anspruch 1 in irgendeiner ihrer isomeren Formen oder in Form eines Gemisches der Isomeren der Einwirkung einer Verbindung der Formel

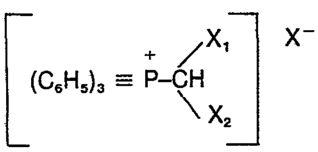

unterzieht, worin $X_1$ und $X_2$ wie in Anspruch 1 definiert sind und $X^-$ ein Halogenidanion bedeutet, um die Verbindung der Formel ($I_A$) zu erhalten, und danach gewünschtenfalls die Synthese wie in Anspruch 1 fortsetzt.

3. Verfahren gemäss Anspruch 1 oder 2, dadurch gekennzeichnet, dass man von einer Verbindung der Formel (II) mit 1R,cis- oder 1R,trans-Struktur ausgeht.

4. Verfahren gemäss einem der Ansprüche 1 bis 3, dadurch gekennzeichnet, dass man von einer Verbindung der Formel (III) ausgeht, worin $X_1$ und $X_2$ identisch sind und alle beide ein Chlor- oder Bromatom darstellen.

5. Verfahren gemäss einem der Ansprüche 1 bis 4, dadurch gekennzeichnet, dass man von einer Verbindung der Formel $A_1OH$ ausgeht, worin $A_1$ einen Alkylrest mit 1 bis 4 Kohlenstoffatomen und insbesondere den Methylrest bedeutet.

6. Verfahren gemäss einem der Ansprüche 1 bis 4, dadurch gekennzeichnet, dass man von einer Verbindung der Formel $A_1OH$ ausgeht, worin $A_1$ eine α-Cyano-3-phenoxybenzylgruppe bedeutet.

7. Verfahren gemäss einem der Ansprüche 1 bis 4, dadurch gekennzeichnet, dass man von einer Verbindung der Formel $A_1OH$ ausgeht, worin $A_1$ eine 3-Allyl-2-methyl-4-oxo-cyclopent-2-en-1-yl-Gruppe bedeutet.

8. Verwendung der Verbindungen der Formel (I) gemäss einem der Ansprüche 1 und 3 bis 7 als Pestizide.

9. Pestizide Zusammensetzung für die Bekämpfung von Parasiten, dadurch gekennzeichnet, dass sie als Wirkstoff zumindest eines der Produkte gemäss einem der Ansprüche 1 und 3 bis 7 enthalten.

10. Insektizide Zusammensetzungen gemäss Anspruch 9, enthaltend als Wirkstoff zumindest eines der Produkte gemäss einem der Ansprüche 1 und 3 bis 7.

11. Verwendung der Verbindungen der Formel (I) gemäss Anspruch 1 als duftverleihende Mittel.

12. Duftverleihende Zusammensetzungen, enthaltend als Wirkstoff zumindest ein Produkt der Formel (I) gemäss Anspruch 1.

13. Duftverleihende Zusammensetzungen, enthaltend als Wirkstoff zumindest ein Produkt der Formel (I) gemäss Anspruch 5.

**Patentansprüche für die Vertragsstaaten: BE, CH, DE, FR, GB, LI, NL**

1. In sämtlichen ihrer möglichen stereoisomeren Formen oder in Form von Gemischen der Stereoisomeren die Verbindungen der Formel (I)

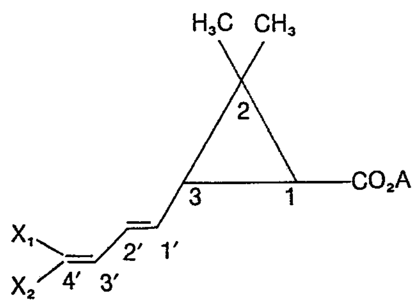

(I)

worin $X_1$ und $X_2$ identisch oder verschieden sind und ein Halogenatom bedeuten und A bedeutet

– entweder einen Benzylrest, gegebenenfalls substituiert durch einen oder mehrere Reste, ausgewählt unter den Alkylresten mit 1 bis 4 Kohlenstoffatomen, den Alkenylresten mit 2 bis 6 Kohlenstoffatomen, den Alkenyloxyresten mit 2 bis 6 Kohlenstoffatomen, den Alkadienylresten mit 4 bis 8 Kohlenstoffatomen, dem Methylendioxylrest, dem Benzylrest und den Halogenatomen,

– oder eine Gruppe

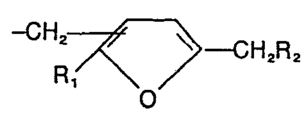

worin der Substituent $R_1$ ein Wasserstoffatom oder einen Methylrest bedeutet und der Substituent $R_2$ einen monocyclischen Arylrest oder eine Gruppe $-CH_2-C\equiv CH$ bedeutet, und insbesondere eine 5-Benzyl-3-furyl-methylgruppe,

– oder eine Gruppe

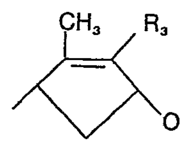

worin $R_3$ einen aliphatischen, organischen Rest mit 2 bis 6 Kohlenstoffatomen und einer oder mehreren Kohlenstoff-Kohlenstoff-Unsättigungen und insbesondere den Vinyl-, Propen-1-yl-, Buta-1,3-dienyl- oder Buten-1-yl-Rest bedeutet,

– oder eine Gruppe

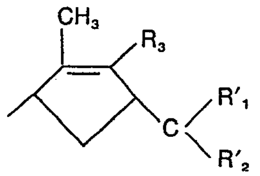

worin $R_3$ die vorstehend angegebene Bedeutung besitzt, $R'_1$ und $R'_2$ gleich oder verschieden sind und ein Wasserstoffatom, ein Halogenatom, einen Alkylrest mit 1 bis 6 Kohlenstoffatomen, einen Arylrest mit 6 bis 10 Kohlenstoffatomen, eine Alkoxycarbonylgruppe mit 2 bis 5 Kohlenstoffatomen oder eine Cyanogruppe bedeuten,

– oder eine Gruppe

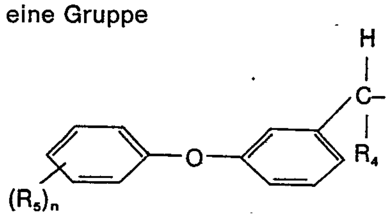

worin $R_4$ ein Wasserstoffatom, eine $-C \equiv N$-Gruppe, einen Methylrest, eine Gruppe $-CONH_2$, eine Gruppe $-CSNH_2$ oder eine Gruppe $-C \equiv CH$ bedeutet, $R_5$ ein Chloratom oder einen Methylrest bedeutet und n eine Zahl entsprechend 0, 1 oder 2 darstellt, unter der Voraussetzung, dass, wenn n = O, $R_4$ weder ein Wasserstoffatom noch eine Gruppe $C \equiv N$ noch eine Gruppe $C \equiv CH$ bedeutet,

– oder eine Gruppe

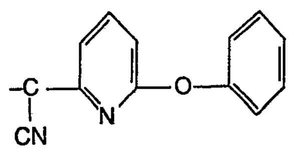

– oder eine Gruppe

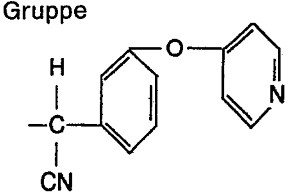

– oder eine Gruppe

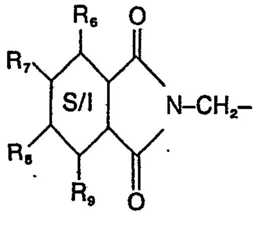

worin die Substituenten $R_6$, $R_7$, $R_8$ und $R_9$ ein Wasserstoffatom, ein Chloratom oder einen Methylrest bedeuten, und worin S/I einen aromatischen Ring oder einen analogen Dihydro- oder Tetrahydro-Ring symbolisiert.

2. In sämtlichen ihrer möglichen stereoisomeren Formen oder in Form von Gemischen der Stereoisomeren die Verbindungen der Formel ($I_a$)

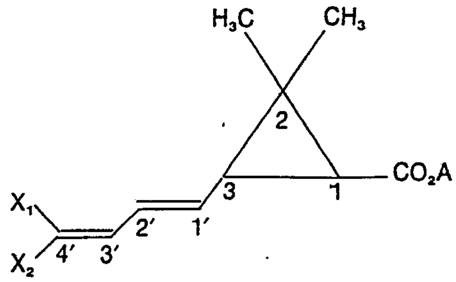

($I_a$)

worin die Cyclopropansäure-Verknüpfungskomponente 1R,cis- oder 1R,trans-Struktur besitzt und worin $X_1$ und $X_2$, die gleich oder verschieden sind, ein Halogenatom bedeuten und A bedeutet

– entweder ein Wasserstoffatom

– oder einen Alkylrest mit 1 bis 18 Kohlenstoffatomen

– oder einen Benzylrest, gegebenenfalls substituiert durch einen oder mehrere Reste, ausgewählt unter den Alkylresten mit 1 bis 4 Kohlenstoffatomen, den Alkenylresten mit 2 bis 6 Kohlenstoffatomen, den Alkenyloxyresten mit 2 bis 6 Kohlenstoffatomen, den Alkadienylresten mit 4 bis 8 Kohlenstoffatomen, dem Methylendioxylrest, dem Benzylrest und den Halogenatomen,

– oder eine Gruppe

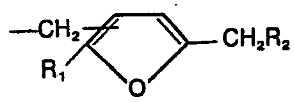

worin der Substituent $R_1$ ein Wasserstoffatom oder einen Methylrest bedeutet und der Substituent $R_2$ einen monocyclischen Arylrest oder eine Gruppe $-CH_2-C \equiv CH$ bedeutet und insbesondere eine 5-Benzyl-3-furylmethylgruppe,

– oder eine Gruppe

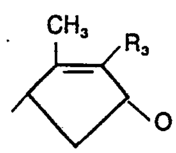

worin $R_3$ einen aliphatischen, organischen Rest mit 2 bis 6 Kohlenstoffatomen und einer oder mehreren Kohlenstoff-Kohlenstoff-Unsättigungen und insbesondere den Vinyl-, Propen-1-yl-, Buta-1,3-dienyl- oder Buten-1-yl-Rest bedeutet,

– oder eine Gruppe

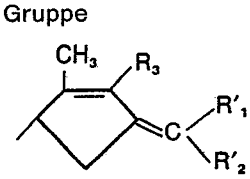

worin $R_3$ die vorstehend angegebene Bedeutung besitzt, $R'_1$ und $R'_2$, die gleich oder verschieden sind, ein Wasserstoffatom, ein Halogenatom, einen Alkylrest mit 1 bis 6 Kohlenstoffatomen, einen

Arylrest mit 6 bis 10 Kohlenstoffatomen, eine Alkoxycarbonylgruppe mit 2 bis 5 Kohlenstoffatomen oder eine Cyanogruppe bedeuten,

– oder eine Gruppe

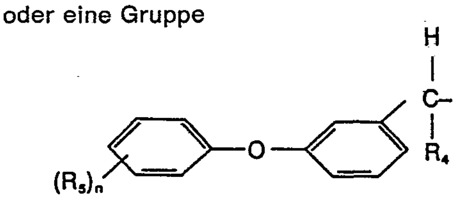

worin $R_4$ ein Wasserstoffatom, eine $-C\equiv N$-Gruppe, einen Methylrest, einen Rest $-CONH_2$, einen Rest $-CSNH_2$ oder eine Gruppe $-C\equiv CH$ bedeutet, $R_5$ ein Chloratom oder einen Methylrest bedeutet und n eine Zahl entsprechend 0, 1 oder 2 bedeutet, und insbesondere die 3-Phenoxybenzyl-, α-Cyano-3-phenoxybenzyl- oder α-Ethinyl-3-phenoxybenzyl-Gruppe,

– oder eine Gruppe

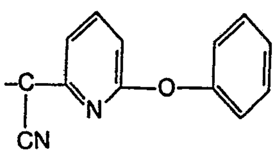

– oder eine Gruppe

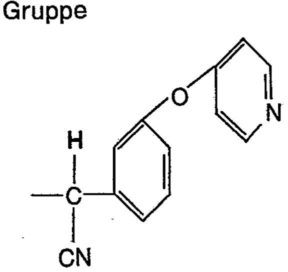

– oder eine Gruppe

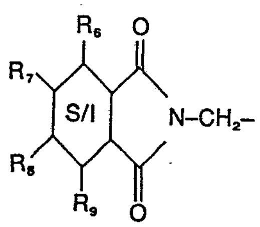

worin die Substituenten $R_6$, $R_7$, $R_8$ und $R_9$ ein Wasserstoffatom, ein Chloratom oder einen Methylrest bedeuten und worin S/I einen aromatischen Ring oder einen analogen Dihydro- oder Tetrahydro-Ring symbolisiert.

3. Verbindungen der Formel (I) und ($I_a$) gemäss Anspruch 1 oder 2, worin $X_1$ und $X_2$ identisch sind und alle beide ein Brom- oder Chloratom bedeuten.

4. Verbindungen der Formel ($I_a$) gemäss Anspruch 2 oder 3, worin A ein Wasserstoffatom bedeutet, sowie deren Salze.

5. Verbindungen der Formel ($I_a$) gemäss Anspruch 2 oder 3, worin A einen Alkylrest mit 1 bis 4 Kohlenstoffatomen und insbesondere den Methylrest bedeutet.

6. Verbindungen der Formel ($I_a$) gemäss einem der Ansprüche 2 oder 3, worin A eine α-Cyano-3-phenoxybenzylgruppe bedeutet.

7. Verbindungen der Formel (I) gemäss einem der Ansprüche 1, 2 oder 3, worin A eine 3-Allyl-2-methyl-4-oxo-cyclopent-2-en-1-yl-Gruppe bedeutet.

8. Verfahren zur Herstellung der Verbindungen der Formel (I) gemäss Anspruch 1, dadurch gekennzeichnet, dass man die Verbindung der Formel (II)

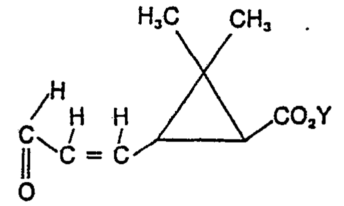
(II)

worin Y ein Wasserstoffatom oder einen Alkylrest mit 1 bis 18 Kohlenstoffatomen bedeutet, in irgendeiner ihrer isomeren Formen oder in Form eines Gemisches der Isomeren der Einwirkung einer Verbindung der Formel (III)

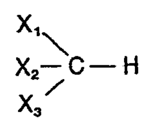
(III)

unterzieht, worin entweder die Substituenten $X_1$, $X_2$ und $X_3$ identisch sind und ein Chlor- oder Bromatom bedeuten oder zumindest zwei der Substituenten $X_1$, $X_2$ und $X_3$ verschieden sind, wobei $X_1$, $X_2$ und $X_3$ dann ein Fluor-, Chlor- oder Bromatom bedeuten, wobei $X_1$, $X_2$ und $X_3$ gleiche Atomgewichte aufweisen oder von $X_1$ bis $X_3$ zunehmende Atomgewichte, um die Verbindung der Formel (IV)

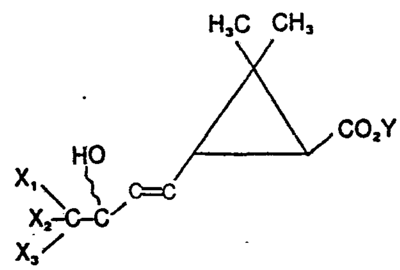
(IV)

zu erhalten, deren Hydroxylgruppe man durch Einwirken einer Säure oder eines funktionellen Derivats einer Säure $RCO_2H$ blockiert, anschliessend das erhaltene Produkt der Formel

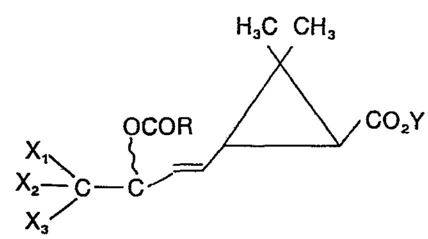
(V)

der Einwirkung eines Reduktionsmittels unterzieht, um nach Eliminierung des Substituenten $X_3$, der der Substituent oder einer der Substituenten mit dem höchsten Atomgewicht ist, die Verbindung der Formel ($I_A$)

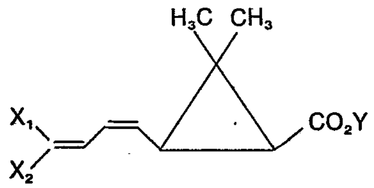

$(I_A)$

zu erhalten, worin $X_1$ und $X_2$ entweder identisch sind und ein Chlor- oder Bromatom bedeuten oder verschieden sind und ein Fluor-, Chlor- oder Bromatom bedeuten, danach eine Verbindung der Formel $I_A$, worin Y einen Alkylrest mit 1 bis 18 Kohlenstoffatomen bedeutet, der Einwirkung eines Verseifungsmittels unterzieht, um eine Verbindung der Formel (I), worin A ein Wasserstoffatom bedeutet, oder ein Salz dieser Verbindung zu erhalten, oder entweder eine Verbindung der Formel $(I_A)$, worin Y einen Alkylrest mit 1 bis 18 Kohlenstoffatomen bedeutet, oder eine Verbindung der Formel $(I_A)$, worin Y ein Wasserstoffatom bedeutet, oder ein funktionelles Derivat dieser letzteren der Einwirkung eines Alkohols der Formel A OH oder eines funktionellen Derivats dieses Alkohols unterzieht, wobei in dieser Formel A die gleiche Bedeutung wie in Anspruch 1 besitzt, um die entsprechende Verbindung der Formel (I) zu erhalten.

9. Verfahren zur Herstellung der Verbindungen der Formel $(I_a)$ gemäss Anspruch 2, dadurch gekennzeichnet, dass man die Verbindung der Formel $(II_A)$

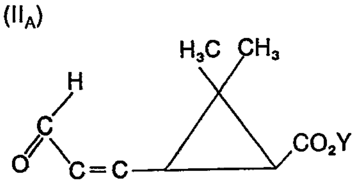

$(II_A)$

worin Y ein Wasserstoffatom oder einen Alkylrest mit 1 bis 18 Kohlenstoffatomen bedeutet, in irgendeiner ihrer isomeren Formen oder in Form eines Gemisches der Isomeren der Einwirkung einer Verbindung der Formel (III)

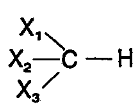

(III)

unterzieht, worin entweder die Substituenten $X_1$, $X_2$ und $X_3$ identisch sind und ein Chlor- oder Bromatom bedeuten oder zumindest zwei der Substituenten $X_1$, $X_2$ und $X_3$ verschieden sind, wobei $X_1$, $X_2$ und $X_3$ dann ein Fluor-, Chlor- oder Bromatom bedeuten, wobei $X_1$, $X_2$ und $X_3$ gleiche Atomgewichte oder von $X_1$ bis $X_3$ zunehmende Atomgewichte aufweisen, um die Verbindung der Formel $(IV_A)$

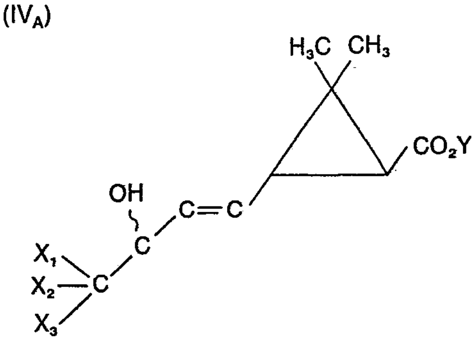

$(IV_A)$

zu erhalten, deren Hydroxylgruppe man durch Einwirken einer Säure oder eines funktionellen Derivats einer Säure $RCO_2H$ blockiert, anschliessend das erhaltene Produkt der Formel

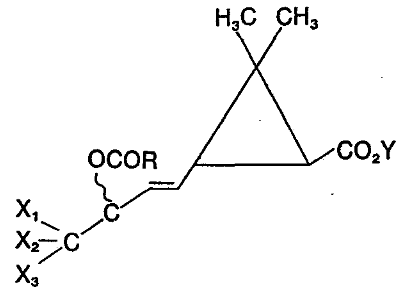

$(V_A)$

der Einwirkung eines Reduktionsmittels unterzieht, um nach Eliminierung des Substituenten $X_3$, der der Substituent oder einer der Substituenten mit dem höchsten Atomgewicht ist, die Verbindung der Formel $(I_A)$

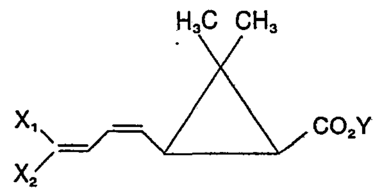

$(I_A)$

zu erhalten, worin $X_1$ und $X_2$ entweder identisch sind und ein Chlor- oder Bromatom bedeuten oder verschieden sind und ein Fluor-, Chlor- oder Bromatom bedeuten, anschliessend gewünschtenfalls eine Verbindung der Formel $(I_A)$, worin Y einen Alkylrest mit 1 bis 18 Kohlenstoffatomen bedeutet, der Einwirkung eines Verseifungsmittels unterzieht, um eine Verbindung der Formel $(I_a)$, worin A ein Wasserstoffatom bedeutet, oder ein Salz dieser Verbindung zu erhalten, danach gewünschtenfalls entweder eine Verbindung der Formel $(I_A)$, worin Y einen Alkylrest mit 1 bis 18 Kohlenstoffatomen bedeutet, oder eine Verbindung der Formel $(I_A)$, worin Y ein Wasserstoffatom bedeutet, oder ein funktionelles Derivat dieser letzteren der Einwirkung eines Alkohols der Formel $A_1OH$ oder eines funktionellen Derivats dieses Alkohols unterzieht, wobei in dieser Formel $A_1$ die gleiche Bedeutung besitzt wie A in Anspruch 1 mit Ausnahme von Wasserstoff, um die entsprechende Verbindung der Formel $(I_a)$ zu erhalten.

10. Variante des Verfahrens gemäss Anspruch 8 oder 9, dadurch gekennzeichnet, dass man nach der Wittig-Reaktion die Verbindung der Formel (II) oder $(II_A)$ gemäss Anspruch 8 oder 9 in irgendeiner ihrer isomeren Formen oder in Form eines Gemisches der Isomeren der Einwirkung einer Verbindung der Formel

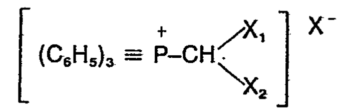

unterzieht, worin $X_1$ und $X_2$ wie in Anspruch 1 definiert sind und $X^-$ ein Halogenidanion bedeutet, um die Verbindung der Formel ($I_A$) zu erhalten, und danach gewünschtenfalls die Synthese wie in Anspruch 8 oder 9 fortsetzt.

11. Verwendung der Verbindung der Formel (I) gemäss einem der Ansprüche 1 bis 7 zur Bekämpfung von Parasiten der Pflanzen.

12. Zusammensetzungen für die Bekämpfung von Parasiten der Pflanzen und warmblütigen Tiere, dadurch gekennzeichnet, dass sie als Wirkstoff zumindest eines der Produkte gemäss einem der Ansprüche 1 bis 7 enthalten.

13. Insektizide Zusammensetzungen gemäss Anspruch 11, enthaltend als Wirkstoff zumindest eines der Produkte gemäss einem der Ansprüche 1 bis 7.

14. Duftverleihende Zusammensetzungen, enthaltend als Wirkstoff zumindest ein Produkt der Formel (I) in sämtlichen seiner möglichen stereoisomeren Formen sowie in Form von Gemischen der Stereoisomeren:

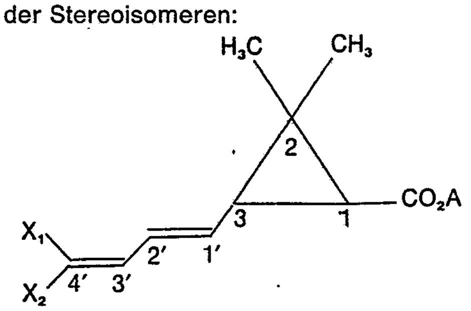
(I)

worin $X_1$ und $X_2$, die gleich oder verschieden sind, ein Halogenatom bedeuten und A bedeutet
– entweder ein Wasserstoffatom
– oder einen Alkylrest mit 1 bis 18 Kohlenstoffatomen
– oder einen Benzylrest, gegebenenfalls substituiert durch einen oder mehrere Reste, ausgewählt unter den Alkylresten mit 1 bis 4 Kohlenstoffatomen, den Alkenylresten mit 2 bis 6 Kohlenstoffatomen, den Alkenyloxyresten mit 2 bis 6 Kohlenstoffatomen, den Alkadienylresten mit 4 bis 8 Kohlenstoffatomen, dem Methylendioxylrest, dem Benzylrest und den Halogenatomen,
– oder eine Gruppe

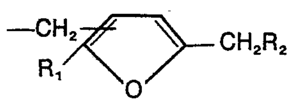

worin der Substituent $R_1$ ein Wasserstoffatom oder einen Methylrest bedeutet und der Substituent $R_2$ einen monocyclischen Arylrest oder eine Gruppe $-CH_2-C\equiv CH$ bedeutet, und insbesondere einen 5-Benzyl-3-furyl-methylrest,
– oder eine Gruppe

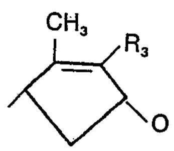

worin $R_3$ einen aliphatischen, organischen Rest mit 2 bis 6 Kohlenstoffatomen und einer oder mehreren Kohlenstoff-Kohlenstoff-Unsättigungen und insbesondere den Vinyl-, Propen-1-yl-, Buta-1,3-dienyl- oder Buten-1-yl-Rest bedeutet,
– oder eine Gruppe

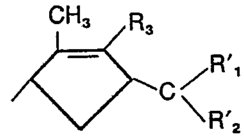

worin $R_3$ die vorstehend angegebene Bedeutung besitzt, $R'_1$ und $R'_2$, die identisch oder verschieden sind, ein Wasserstoffatom, ein Halogenatom, einen Alkylrest mit 1 bis 6 Kohlenstoffatomen, einen Arylrest mit 6 bis 10 Kohlenstoffatomen, einen Alkoxycarbonylrest mit 2 bis 5 Kohlenstoffatomen oder eine Cyanogruppe bedeuten,
– oder eine Gruppe

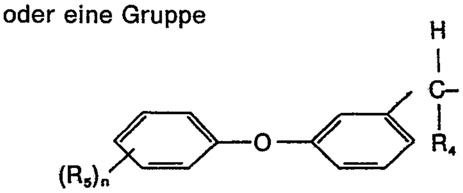

worin $R_4$ ein Wasserstoffatom, eine $-C\equiv N$-Gruppe, einen Methylrest, einen Rest $-CONH_2$, einen Rest $-CSNH_2$ oder eine Gruppe $-C\equiv CH$ bedeutet, $R_5$ ein Chloratom oder einen Methylrest bedeutet und n eine Zahl entsprechend 0, 1 oder 2 darstellt, und insbesondere die 3-Phenoxybenzyl-, α-Cyano-3-phenoxybenzyl- oder α-Ethinyl-3-phenoxybenzylgruppe,
– oder eine Gruppe

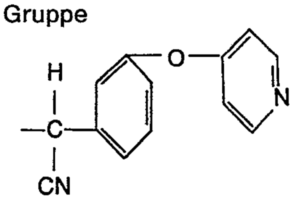

– oder eine Gruppe

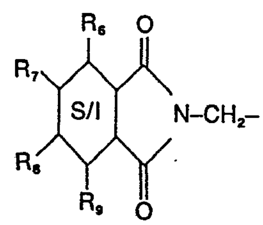

worin die Substituenten $R_6$, $R_7$, $R_8$ und $R_9$ ein Wasserstoffatom, ein Chloratom oder einen Methylrest bedeuten und worin S/I einen aromatischen Ring oder einen analogen Dihydro- oder Tetrahydro-Ring symbolisiert.

15. Duftverleihende Zusammensetzungen, enthaltend als Wirkstoff zumindest ein Produkt der Formel (I) gemäss Anspruch 5.

16. Als Arzneimittel die Verbindungen der Formel (I) gemäss Anspruch 14.

17. Pharmazeutische Zusammensetzungen, enthaltend als Wirkstoff zumindest eines der Arzneimittel gemäss Anspruch 16.

18. Zusammensetzungen für die tierische Ernährung, enthaltend als Wirkstoff zumindest eines der Produkte der Formel (I) gemäss Anspruch 14 zusammen mit einem Nahrungsmittel für Tiere.